# EUROPÄISCHE PATENTSCHRIFT

(11) **EP 3 227 269 B1**
(45) Veröffentlichungstag und Bekanntmachung des Hinweises auf die Patenterteilung: **26.02.2020**
(21) Anmeldenummer: 15790840.1
(22) Anmeldetag: 05.11.2015
(51) Int. Cl.: C07D 311/96, C07D 333/76, C07D 335/12, C07C 211/61, C07D 209/82, C07D 307/91, C09B 57/00, H01L 51/00, C07D 335/20, C07D 209/86, H01L 51/50, C09B 19/00, C09B 21/00, C09B 57/10, C09K 11/02

(54) **MATERIALIEN FÜR ORGANISCHE ELEKTROLUMINESZENZVORRICHTUNGEN**
MATERIALS FOR ORGANIC ELECTROLUMINESCENT DEVICES
MATIÈRES POUR DISPOSITIFS ÉLECTROLUMINESCENTS ORGANIQUES

(30) Priorität: 01.12.2014 EP 14004045
(43) Veröffentlichungstag der Anmeldung: 11.10.2017
(73) Patentinhaber: Merck Patent GmbH, 64293 Darmstadt (DE)
(72) Erfinder: MUJICA-FERNAUD, Teresa, 64283 Darmstadt (DE); MONTENEGRO, Elvira, 69469 Weinheim (DE); PFISTER, Jochen, 64342 Seeheim-Jugenheim (DE); MARTYNOVA, Irina, 64347 Griesheim (DE); STIEBER, Frank, 64683 Einhausen (DE)
(86) Internationale Anmeldenummer: PCT/EP2015/002225
(87) Internationale Veröffentlichungsnummer: WO 2016/087017

(56) Entgegenhaltungen:
- WO-A1-2009/003455
- WO-A1-2013/083216
- YI WEI ET AL: "Emission mechanism of doubly ortho-linked quinoxaline / diphenylfluorene or cis-stilbene / fluorene hybrid compounds based on the transient absorption and emission measurements during pulse radiolysis", JOURNAL OF THE AMERICAN CHEMICAL SOCIETY, AMERICAN CHEMICAL SOCIETY, US, Bd. 131, Nr. 19, 20. Mai 2009 (2009-05-20) , Seiten 6698-6707, XP002739995, ISSN: 0002-7863, DOI: 10.1021/JA8090005 [gefunden am 2009-04-22]
- CHEN CHIEN-TIEN ET AL: "Doubly Ortho-Linked Quinoxaline/Diphenylfluorene Hybrids as Bipolar, Fluorescent Chameleons for Optoelectronic Applications", JOURNAL OF THE AMERICAN CHEMICAL SOCIETY, AMERICAN CHEMICAL SOCIETY, US, Bd. 128, Nr. 34, 1. Januar 2006 (2006-01-01), Seiten 10992-10993, XP008090033, ISSN: 0002-7863, DOI: 10.1021/JA062660V
- HONGJI JIANG: "Organic Ambipolar Conjugated Molecules for Electronics: Synthesis and Structure?Property Relationships", MACROMOLECULAR RAPID COMMUNICATIONS, WILEY - V C H VERLAG GMBH & CO. KGAA, DE, Bd. 31, Nr. 23, 1. Dezember 2010 (2010-12-01), Seiten 2007-2034, XP009146014, ISSN: 1022-1336, DOI: 10.1002/MARC.201000040 [gefunden am 2010-07-27]

## Beschreibung

Die vorliegende Erfindung beschreibt Tribenzotropon-Derivate, insbesondere zur Verwendung in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

In Wei et al (JACS, 2009, pp 6698-6707), Chen et al (JACS, 2006, pp 10992-10993) und Jiang et al (Macromolecular Rapid communications, 2010, pp 2007-2034) sind Spirobiflouren-Verbindungen, die eine eingeschobene Chinoxalin-Gruppe aufweisen, offenbart.
In WO 2013/083216A1 werden Verbindung offenbart, die eine divalente Brücke -NR²- eingeschoben in ein Spirofluoren aufweisen.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Ladungstransportmaterial, insbesondere Lochtransport- bzw. Elektronenblockiermaterial. Insbesondere ist es die Aufgabe der vorliegenden Erfindung, Matrixmaterialien bereitzustellen, welche sich auch für grün und blau phosphoreszierende OLEDs eignen, sowie neue Ladungstransportmaterialien bereitzustellen.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Lochtransport- und Lochinjektionsmaterialien. 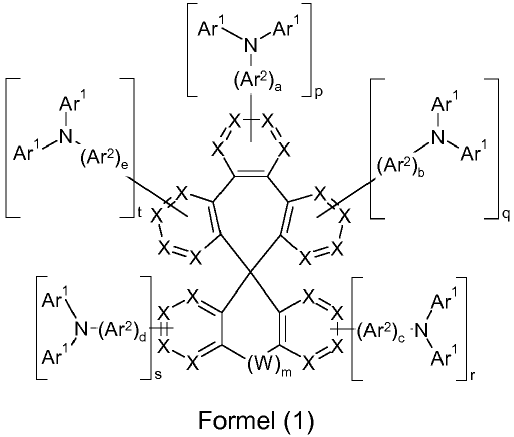 wobei für die verwendeten Symbole und Indizes gilt:
- X: ist bei jedem Auftreten gleich oder verschieden CR¹ oder N;
- Ar²: ist bei jedem Auftreten gleich oder verschieden ein bivalentes aromatisches oder heteroaromatisches Ringsystem mit 5 bis 60 aromatischen Ringatomen, welches mit einem oder mehreren Resten R² substituiert sein kann;
- Ar¹: ist bei jedem Auftreten gleich oder verschieden ein aromatisches oder heteroaromatisches Ringsystem mit 5 bis 60 aromatischen Ringatomen, das jeweils auch mit einem oder mehreren Resten R² substituiert sein kann; wobei an das gleiche N-Atom gebundene Reste Ar¹ und/oder Ar² über mindestens eine Gruppe K verbunden sein können;
- K: ist bei jedem Auftreten gleich oder verschieden eine Einfachbindung oder eine bivalente Brücke, ausgewählt aus N(R²), B(R²), O, C=O, C(R²)₂, Si(R²)₂, C=C(R²)₂, S=O, P(R²), P(=O)R² und S;
- W: ist bei jedem Auftreten gleich oder verschieden eine Einfachbindung oder eine bivalente Brücke, ausgewählt aus B(R²), O,C=O, C(R²)₂, Si(R²)₂, S, und R²C=CR²;
- Ar: ist bei jedem Auftreten gleich oder verschieden ein aromatisches oder heteroaromatisches Ringsystem, bevorzugt eine Aryl- oder Heteroarylgruppe mit 5 bis 40 aromatischen Ringatomen, die mit einem oder mehreren Resten R³ substituiert sein kann;
- R¹: ist bei jedem Auftreten gleich oder verschieden H, D, F, Cl, Br, I, C(=O)Ar, P(=O)Ar₂, S(=O)Ar, S(=O)₂Ar, CR²=CR²Ar, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R² substituiert sein kann, oder eine Aryloxy- oder Heteroaryloxygruppe mit 5 bis 40 aromatischen Ringatomen, die mit einem oder mehreren Resten R² substituiert sein kann, oder eine Aralkyl- oder Heteroaralkylgruppe mit 5 bis 40 aromatischen Ringatomen, die durch einen oder mehrere Reste R² substituiert sein kann;
- R²: ist bei jedem Auftreten gleich oder verschieden H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C=C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R³ substituiert sein kann, oder eine Aryloxy- oder Heteroaryloxygruppe mit 5 bis 40 aromatischen Ringatomen, die mit einem oder mehreren Resten R³ substituiert sein kann, oder eine Aralkyl- oder Heteroaralkylgruppe mit 5 bis 40 aromatischen Ringatomen, die durch einen oder mehrere Reste R³ substituiert sein kann;
- R³: ist bei jedem Auftreten gleich oder verschieden H, D, F, Cl, Br, I, C(=O)Ar, P(=O)Ar₂, S(=O)Ar, S(=O)₂Ar, CR⁴=CR⁴Ar, CN, NO₂, Si(R⁴)₃, B(OR⁴)₂, OSO₂R⁴, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁴ substituiert sein kann, wobei eine oder mehrere nicht benachbarte CH₂-Gruppen durch R⁴C=CR⁴, C≡C , Si(R⁴)₂, C=O, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 40 aromatischen Ringatomen, die durch einen oder mehrere Reste R⁴ substituiert sein kann, oder eine Aralkyl- oder Heteroaralkylgruppe mit 5 bis 40 aromatischen Ringatomen, die durch einen oder mehrere Reste R⁴ substituiert sein kann;
- R⁴: ist bei jedem Auftreten gleich oder verschieden H, D, F oder ein aliphatischer Kohlenwasserstoffrest mit 1 bis 20 C-Atomen oder eine Aryl- oder Heteroarylgruppe mit 5 bis 40 Ringatomen, die durch einen oder mehrere Reste R⁵ substituiert sein kann, oder eine Kombination dieser Gruppen;
- R⁵: ist bei jedem Auftreten gleich oder verschieden H, D, F oder ein aliphatischer Kohlenwasserstoffrest mit 1 bis 20 C-Atomen;
- m: ist 0 oder 1, wobei m gleich 0 bedeutet, dass an dieser Position keine Gruppe W gebunden ist, und die betreffenden an W bindenden Kohlenstoffatome jeweils durch eine Gruppe X ersetzt sind; und
- p, q, r, s, t: sind bei jedem Auftreten gleich oder verschieden 0, 1 oder 2;
- a, b, c, d, e: sind bei jedem Auftreten gleich oder verschieden 0, 1 oder 2;
wobei
- p+q+r+s+t: größer als 1 oder gleich 1 ist; und
- dass,: falls r größer oder gleich 1 ist und s größer oder gleich 1 ist und m gleich 0 ist, und für je mindestens eine Gruppe (Ar²)_{c}N(Ar¹)₂ und (Ar²)_{d}N(Ar¹)₂ c und d gleich 0 sind, diese beiden N(Ar¹)₂-Gruppen nicht jeweils in para-Position zum quartären Kohlenstoffatom des Grundgerüsts angeordnet sind.

Als Grundgerüst ist im Sinne der Erfindung eine Verbindung bezeichnet, welche der Verbindung der Formel (1) ohne Reste entspricht. Dies ist eine Verbindung gemäß der Formel (1) in der p+q+r+r+s+t gleich 0 und R¹ und R² gleich H sind. Das Grundgerüst in in der Formel (1b) dargestellt: 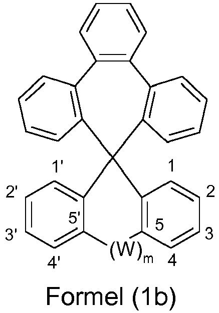 wobei die Symbole und Indizes der Formel (1) entsprechen.

Die (Ar²)N(Ar¹)₂-Gruppen sind jeweils an Stelle von R¹ in einer Einheit CR¹ mit dem Grundgerüst verbunden.

Bevorzugt ist die Verbindung keine Verbindung der Formel (1c): 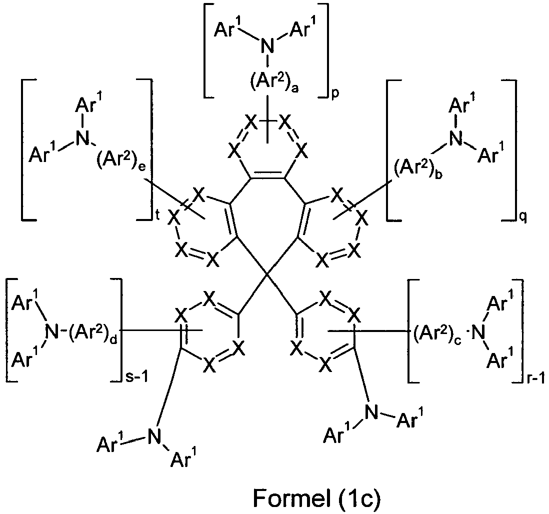 wobei die Symbole und Indizes der Formel (1) entsprechen und zusätzlich gilt, dass r größer oder gleich 1 und s größer oder gleich 1 ist.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Thiophen, etc., oder eine kondensierte (anellierte) Aryl- oder Heteroarylgruppe, beispielsweise Naphthalin, Anthracen, Phenanthren, Dibenzofuran, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

In einer bevorzugten Ausführungsform der Erfindung umfasst die Verbindung keine weiteren Diarylaminogruppen außer den in Formel (1) oder ihren bevozugten Ausführungsformen angegebenen Diarylaminogruppen. Dies bedeutet, dass bevorzugt heteroaromatische Ringsysteme keine Diarylaminogruppen umfassen. Dies umfasst auch Amine mit Heteroarylgruppen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie beispielsweise in Benzimidazol oder Chinolin.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthfoxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme. Dabei können diese Gruppen jeweils durch die oben genannten Reste substituiert sein.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc. Dabei können an diese Gruppen auch noch weitere 6-Ring-Aryl- oder 6-Ring-Heteroarylgruppen ankondensiert sein, wie beispielsweise in Benzimidazol oder Chinolin.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht: 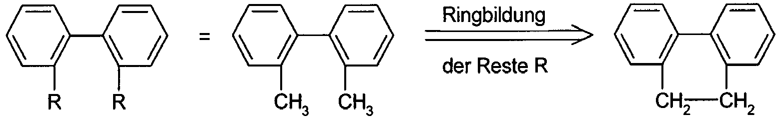 Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

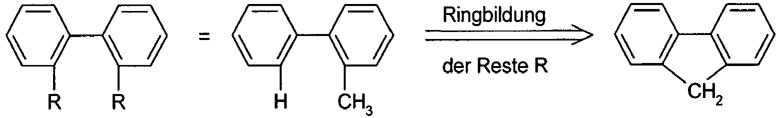

In einer weiteren Ausführungsform der Erfindung umfasst ein 6-Ring-Cyclus in Formel (1) maximal ein N als aromatisches Ringatom, d.h. nur ein X steht für N. Besonders bevorzugt steht keines der Symbole X in Formel (1) für N.

Bevorzugte Ausführungsform der Verbindung der Formel (1) ist eine Verbindung der folgenden Formel (2): 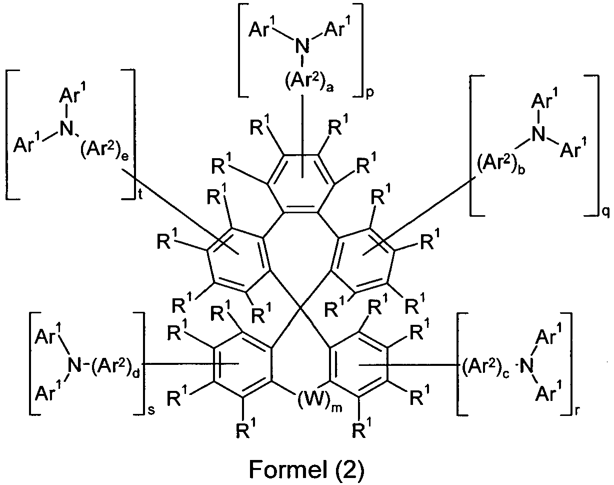 wobei die verwendeten Symbole und Indizes denen von Formel (1) entsprechen.

Erfindungsgemäß sind für m gleich 0, r größer oder gleich 1, s größer oder gleich 1 und mindestens ein c gleich 0 und mindestens ein d gleich 0, die mindestens zwei N(Ar¹)₂-Gruppen an den Positionen 1, 2, 4, 5, 1', 2', 4' oder 5' angeordnet.

In einer bevorzugten Ausführungsform der Erfindung umfasst die Verbindung keine Verbindung der folgenden Formel (3): 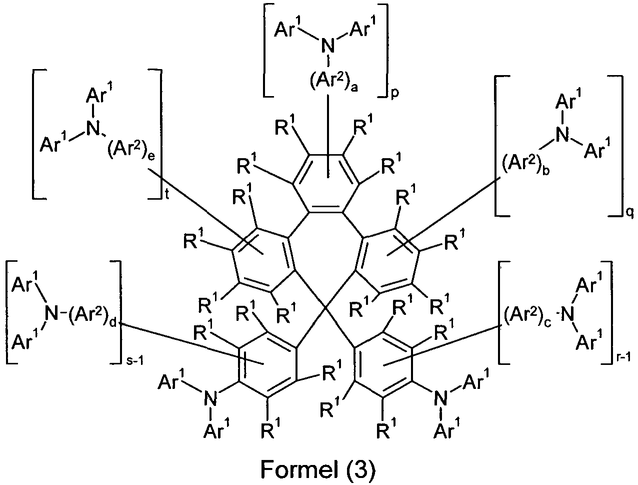 wobei die verwendeten Symbole und Indizes denen von Formel (1) entsprechen und zusätzlich gilt, dass r und s größer oder gleich 1 sind.

In einer bevorzugten Ausführungsform der Erfindung ist p+q+r+s+t ein Wert von 1 bis 10, bevorzugt von 1 bis 5, besonders bevorzugt 1 oder 2, ganz besonders bevorzugt 1.

In einer weiteren bevorzugten Ausführungsform der Erfindung sind p, q, r, s, und t bei jedem Auftreten gleich oder verschieden 0 oder 1.

In einer weiteren bevorzugten Ausführungsform der Erfindung ist p gleich 0.

In einer weiteren bevorzugten Ausführungsform ist r gleich 1, und p, q, s, und t sind gleich 0. In einer alternativen bevorzugten Ausführungsform ist s gleich 1, und p, q, r, und t sind gleich 0.

Bevorzugt sind p, q, r, s, und t bei jedem Auftreten gleich oder verschieden 0 oder 1. In diesem Fall ist pro Zyklus eine Diarylaminogruppe gebunden.

In einer bevorzugten Ausführungsform der Erfindung ist die Verbindung eine Verbindung gemäß einer der folgenden Formeln (4) und (5):

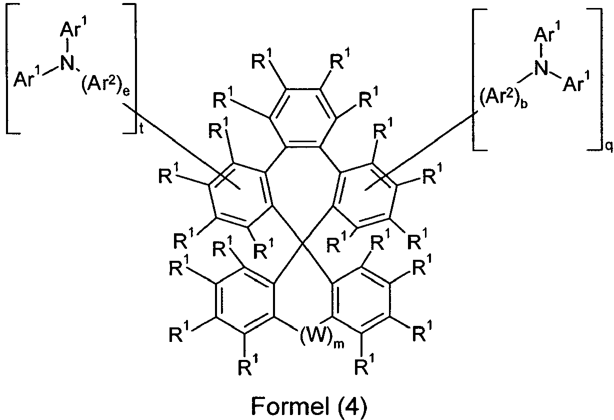

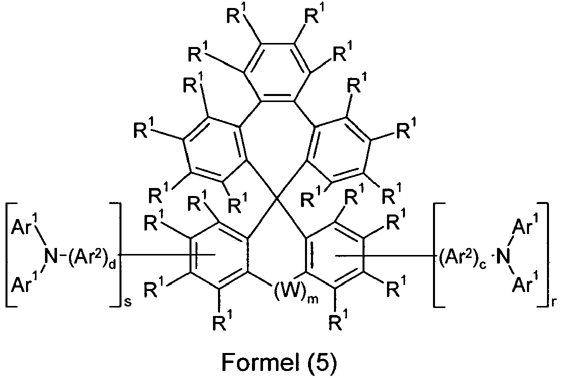

Dabei entsprechen die Symbole und Indizes den Symbolen und Indizes der Formel (1).

Bevorzugt weist die erfindungsgemäße Verbindung keine weiteren an das Grundgerüst ankondensierten oder anelierten Ringe auf.

In einer bevorzugten Ausführungsform der Erfindung ist W bei jedem Auftreten O, S oder eine Einfachbindung, besonders bevorzugt eine Einfachbindung.

In einer weiteren Ausführungsform der Erfindung ist die Verbindung eine Verbindung einer der folgenden Formeln (6) bis (11): 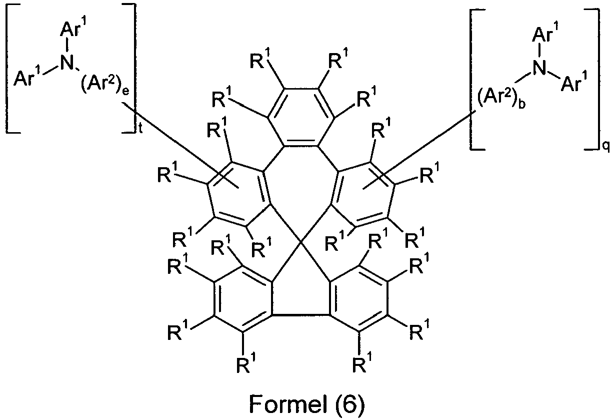 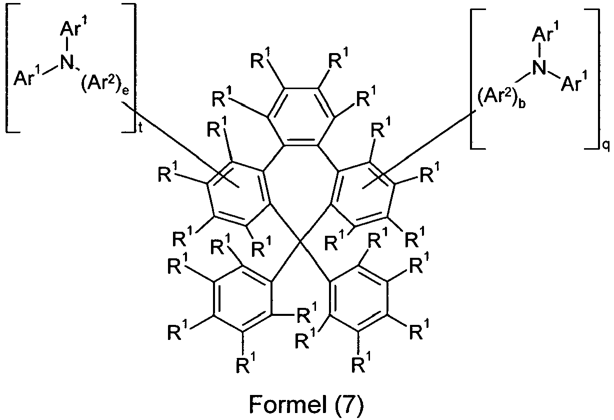 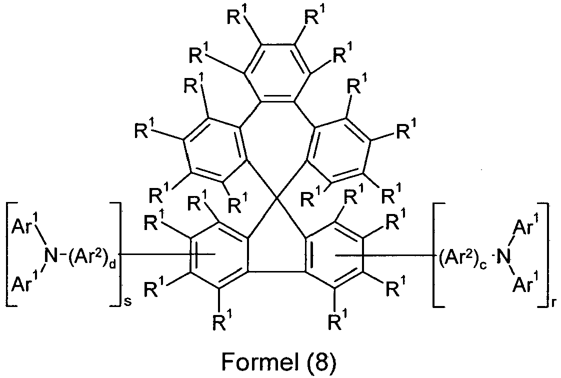 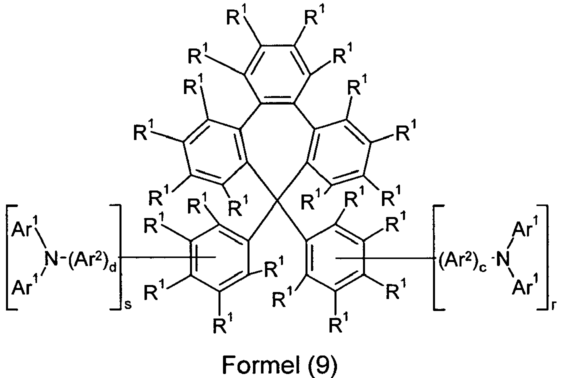 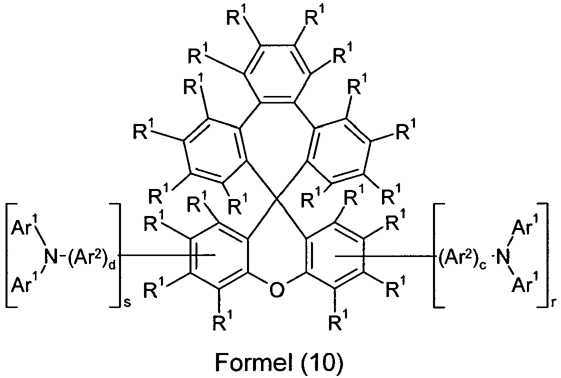 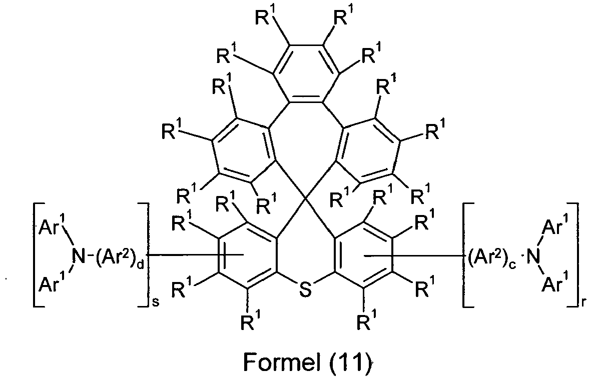 wobei die Symbole und Indizes den Symbolen und Indizes von Formel (1) entsprechen.

In einer bevorzugten Ausführungsform ist die Verbindung ausgewählt aus den Verbindungen der Formeln (8) bis (11).

Bevorzugt ist in Formeln (9) sind im Fall von r größer 0 und s größer 0 keine der Diarylaminogruppen para zum quartären Kohlenstoffatom des Grundgerüsts angeordnet.

In einer besonders bevorzugten Ausführungsform der Erfindung ist die Verbindung eine Verbindung einer der folgenden Formeln (8-1) bis (11-3): 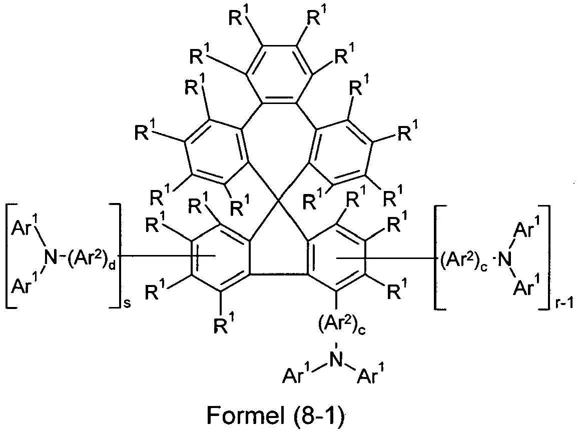 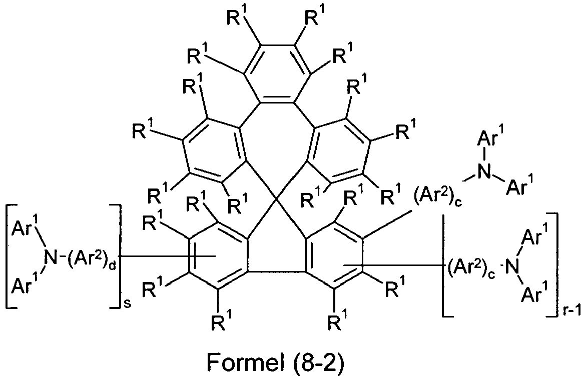 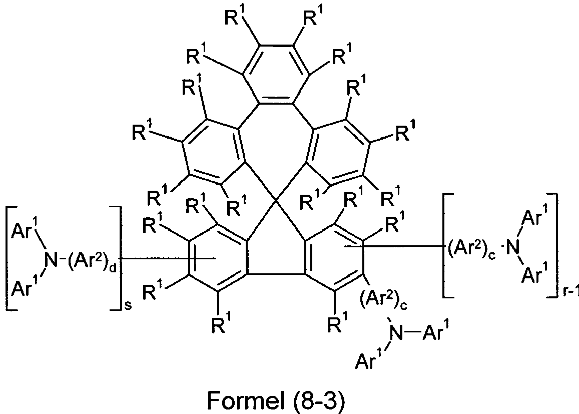 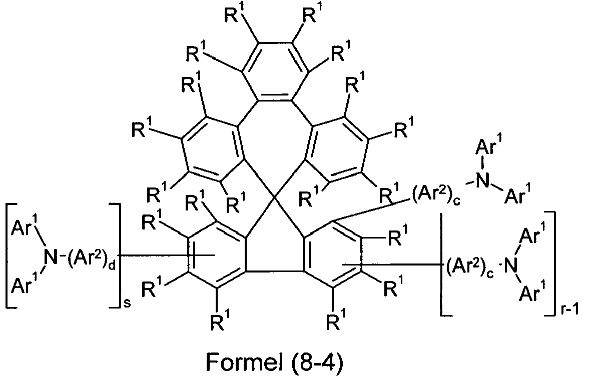 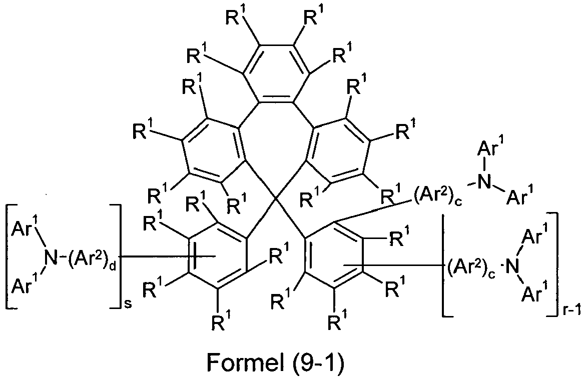 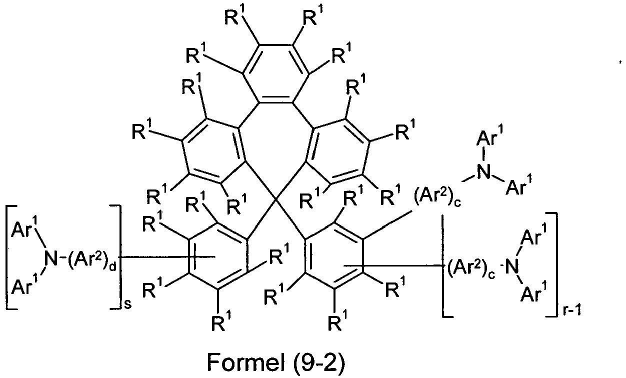 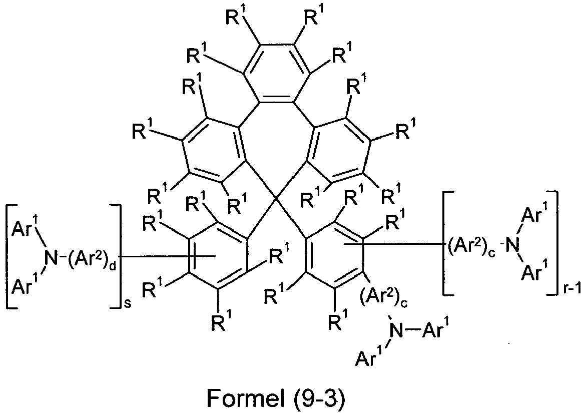 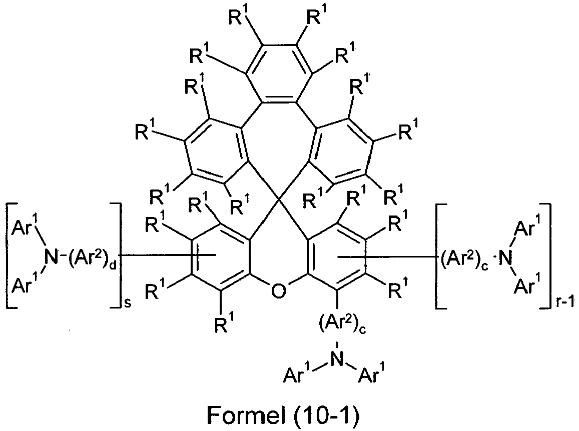 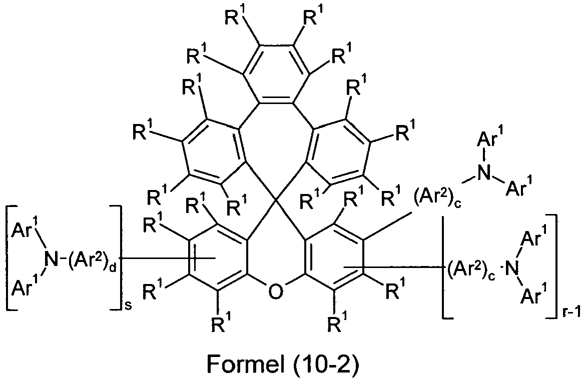 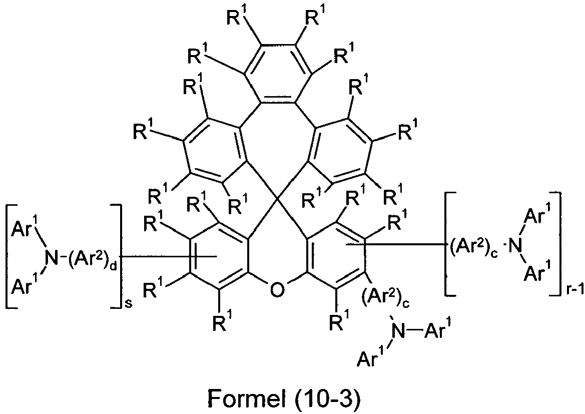 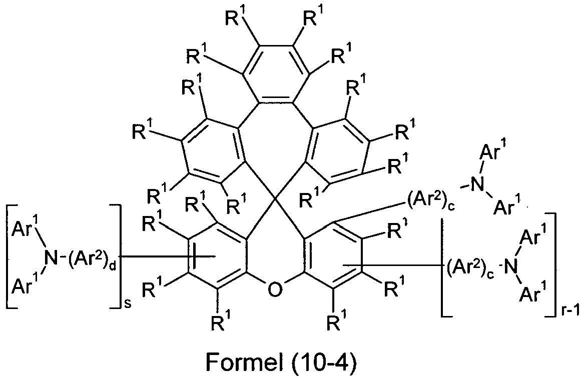 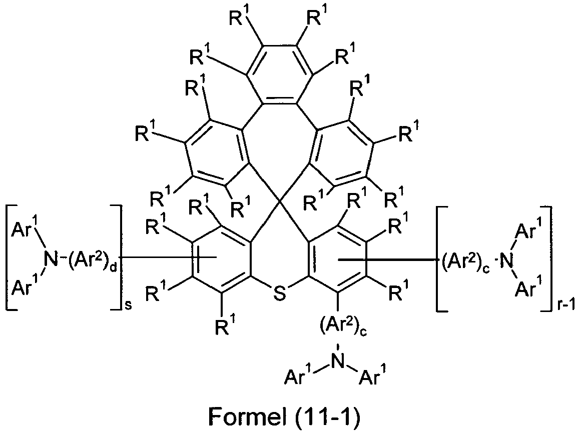 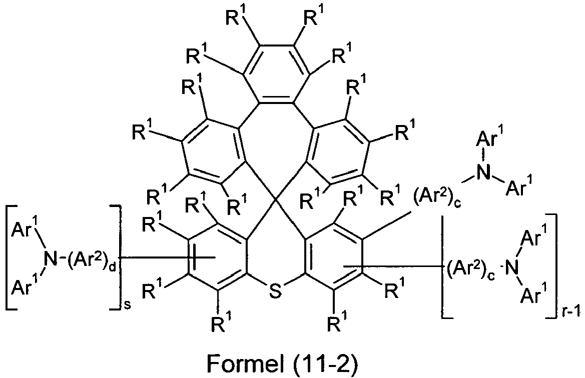 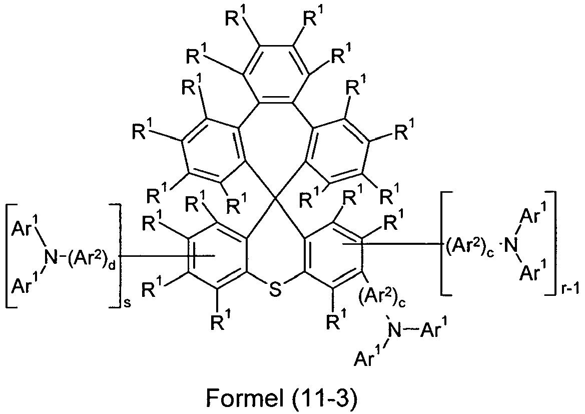 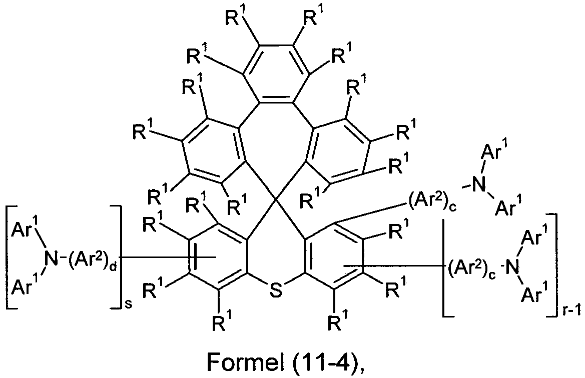 wobei die Symbole und Indizes den Symbolen und Indizes von Formel (1) entsprechen und zusätzlich gilt:
r ist mindestens 1; und
für die Formeln (9-1), (9-2) und (9-3) falls s größer oder gleich 1 ist keine zwei N(Ar¹)₂-Gruppen mit c und d gleich 0 in para-Position zum quartären Kohlenstoffatom des Grundgerüsts angeordnet sind.

Bevorzugt ist in den Formeln (8-1) bis (11-4) r gleich 1 und s gleich 0.

In einer weiteren besonders bevorzugten Ausführungsform der Erfindung ist die Verbindung eine Verbindung einer der folgenden Formeln (8-1-1) bis (11-4-1): 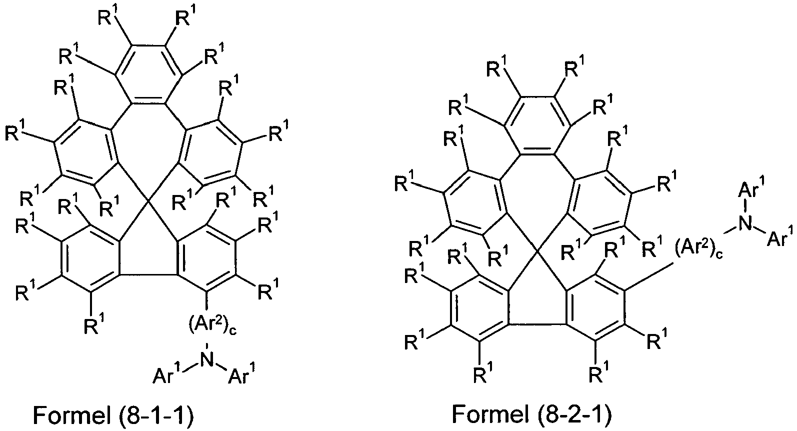 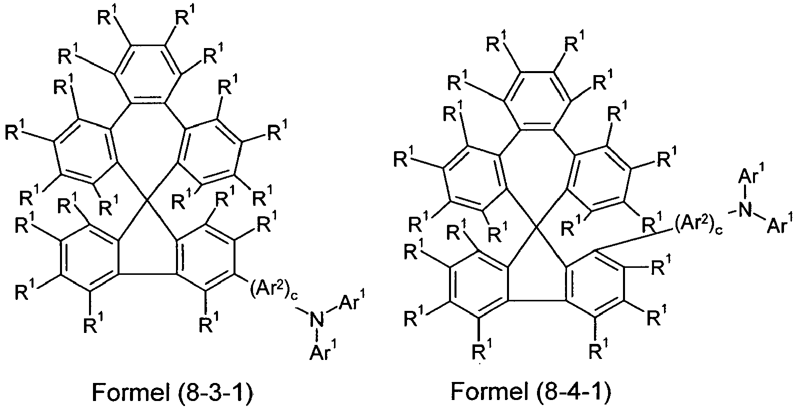 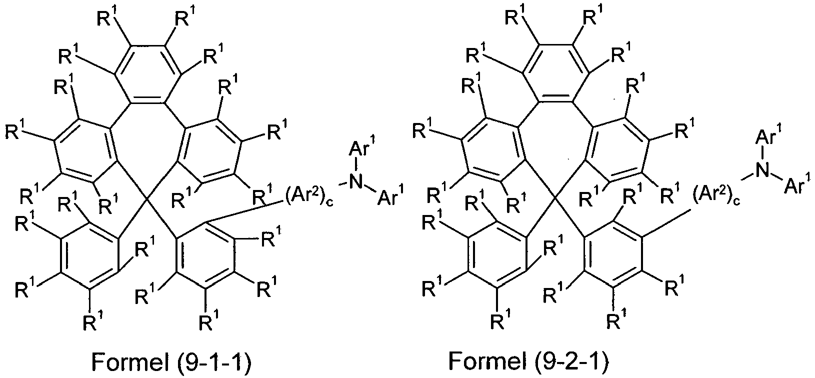 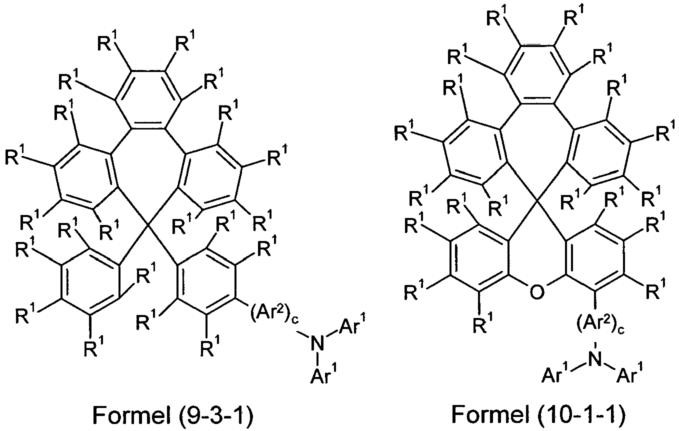 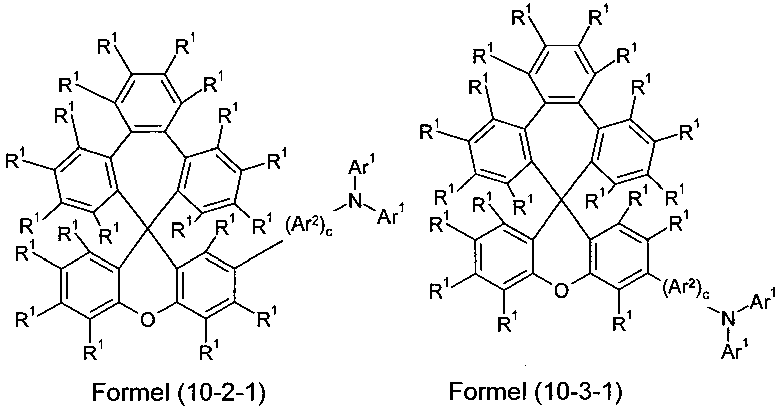 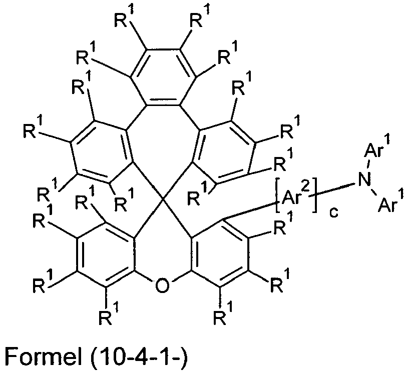 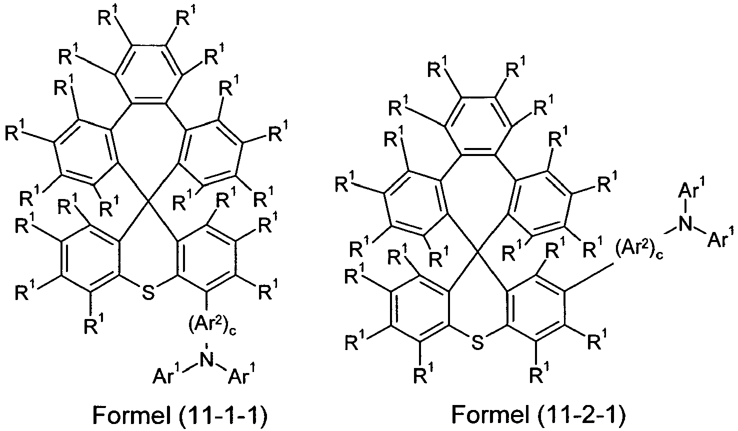 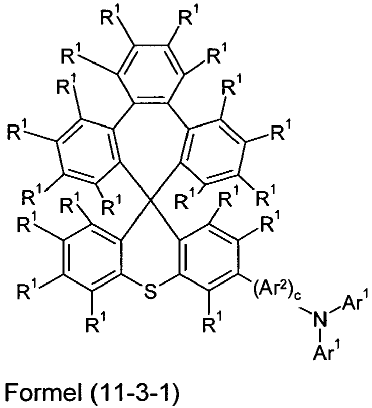 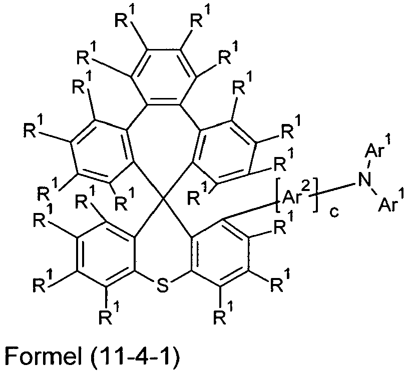 wobei die Symbole und Indizes den Symbolen und Indizes von Formel (1) entsprechen.

In einer bevorzugten Ausführungsform der Erfindung ist R¹ bei jedem Auftreten gleich oder verschieden H, D, F oder ein aliphatischer Kohlenwasserstoffrest mit 1 bis 20 C-Atomen, besonders bevorzugt H, D oder F, ganz besonders bevorzugt H oder D.

In einer bevorzugten Ausführungsform der Erfindung sind die Gruppen Ar¹ bei jedem Auftreten ausgewählt aus den Gruppen mit den folgenden Formeln (Ar-1) bis (Ar-15) 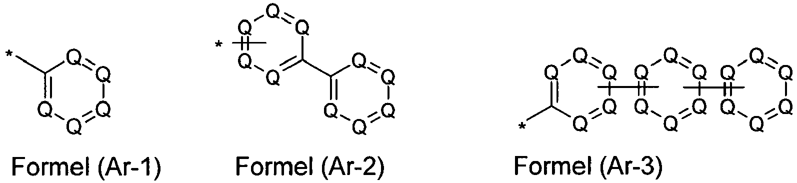 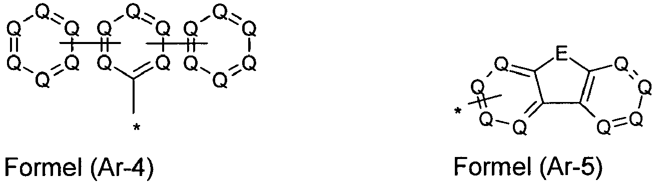 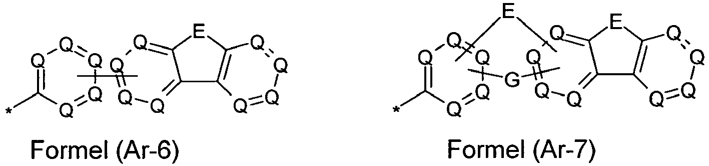 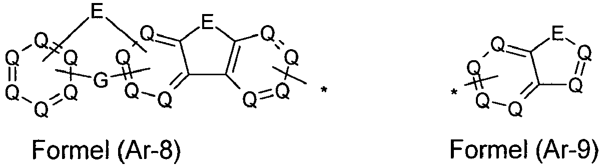 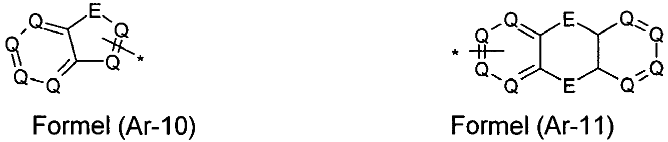 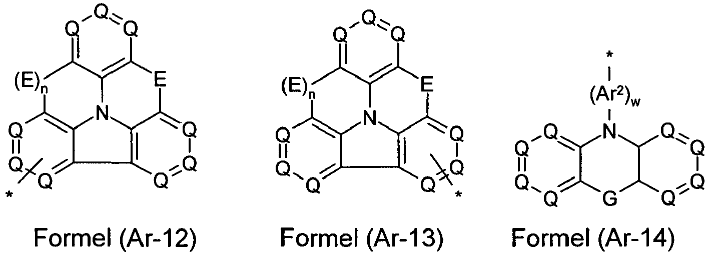 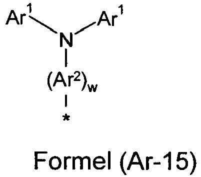 wobei die Symbole und Indizes den Symbolen und Indizes der Formel (1) entsprechen und zusätzlich gilt:
- Q: ist bei jedem Auftreten gleich oder verschieden CR² oder N, wobei maximal 3 Symbole Q pro Cyclus für N stehen;
- E: ist bei jedem Auftreten gleich oder verschieden (CR²)₂, NR², O, S, Si(R²)₂ oder C=O;
- G: ist bei jedem Auftreten eine Einfachbindung, (CR²)₂, NR², O, S, Si(R²)₂, oder C=O;
- w: ist 1, 2, 3 oder 4;
- n: ist 0 oder 1, wobei n gleich 0 bedeutet, dass an dieser Position keine Gruppe E gebunden ist und an den entsprechenden Kohlenstoffatomen statt dessen Reste R² gebunden sind; und
- *: die Bindung zum N-Atom darstellt.

Falls die Gruppen Ar¹ mit einer Gruppe K verbunden sind, ist die Gruppe N(Ar¹)₂ bevorzugt ausgewählt aus den folgenden Formeln (Ar-15) bis (Ar-18): 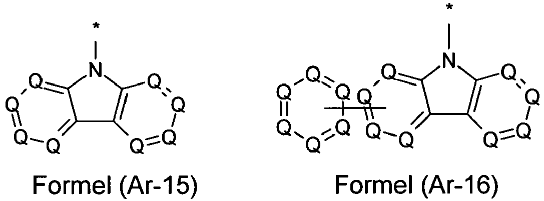 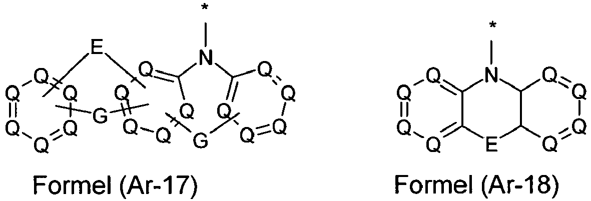 wobei die Symbole und Indizes den Symbolen und Indizes der Formel (1) entsprechen und zusätzlich für die Formeln (Ar-15) bis (Ar-18) gilt:
- Q: ist bei jedem Auftreten gleich oder verschieden CR² oder N, wobei maximal 2 Symbole Q pro Cyclus für N stehen;
- E: ist bei jedem Auftreten gleich oder verschieden (CR²)₂, NR², O, S, Si(R²)₂ oder C=O;
- G: ist bei jedem Auftreten eine Einfachbindung, (CR²)₂, Si(R²)₂, NR², O, S oder C=O;
- *: die Bindung zum Grundgerüst oder soweit vorhanden zu Ar² darstellt.

Falls an das gleiche N-Atom gebundene Reste Ar¹ und Ar² über mindestens eine Gruppe K verbunden sind, so sind die Gruppen Ar²N(Ar¹)₂ ausgewählt aus den Strukturen der Formeln (Ar-5) bis (Ar-14) wobei gilt:
- Q: ist bei jedem Auftreten gleich oder verschieden CR² oder N, wobei maximal 3 Symbole Q pro Cyclus für N stehen;
- E: ist bei jedem Auftreten gleich oder verschieden einmal NAr¹ und bei jedem weiteren Auftreten (CR²)₂, Si(R²)₂, NR², O, S oder C=O;
- G: ist bei jedem Auftreten eine Einfachbindung, (CR²)₂, Si(R²)₂, NR², O, S oder C=O;
- n: ist 0 oder 1, wobei n gleich 0 bedeutet, dass an dieser Position keine Gruppe E gebunden ist und an den entsprechenden Kohlenstoffatomen statt dessen Reste R² gebunden sind; und
- *: die Bindung zum Grundgerüst oder soweit vorhanden zu Ar² darstellt.

In diesem Fall ist die Gruppe Ar¹ bevorzugt ausgewählt aus den Formeln (Ar-1) bis (Ar-15).

In einer weiteren bevorzugten Ausführungsform stehen in der Formel (Ar-1) 0, 1, 2 oder 3 Symbole Q für N.

Bevorzugte Ausführungsformen der Formel (Ar-8) zeigen die folgenden Formeln (Ar-8-1) bis (Ar-8-7): 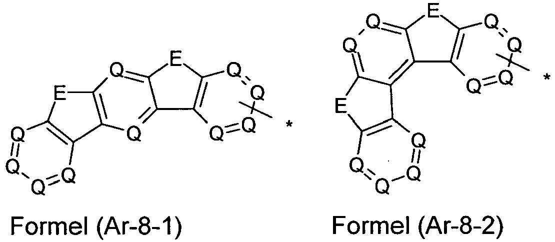 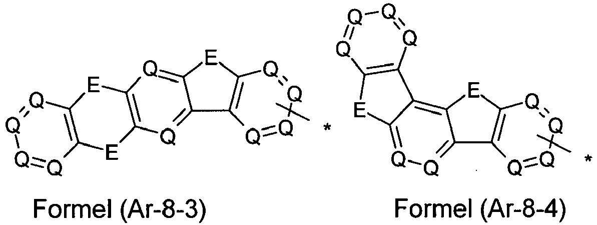 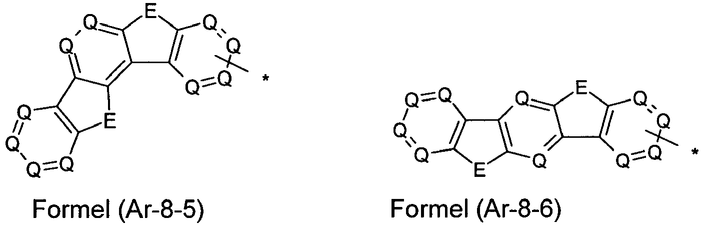 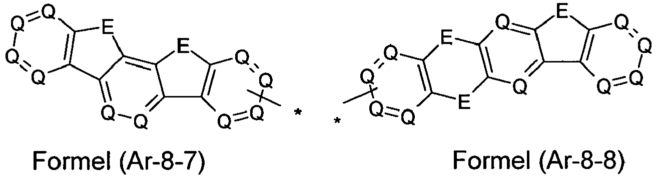 wobei die Symbole den Symbolen der Formel (Ar-8) entsprechen. Besonders bevorzugt ist Q immer CR².

In einer weiteren bevorzugten Ausführungsform ist die Gruppe Ar¹ bei jedem Auftreten gleich oder verschieden ausgewählt aus den Gruppen mit den Strukturen der Formeln (Ar-1) bis (Ar-14), wobei die allgemeinen Formeln durch die besonders bevorzugten Ausführungsformen jeweils gemäß der folgenden Formeln (Ar-1-1) bis (Ar-14-1) ersetzt werden (z. B. wird Formel (Ar-1) durch eine der Formeln (Ar-1-1) bis (Ar-1-12) ersetzt): 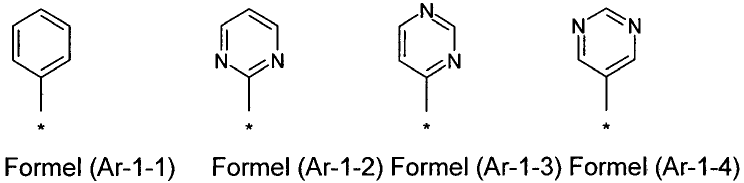 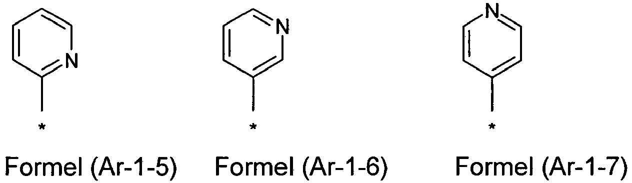 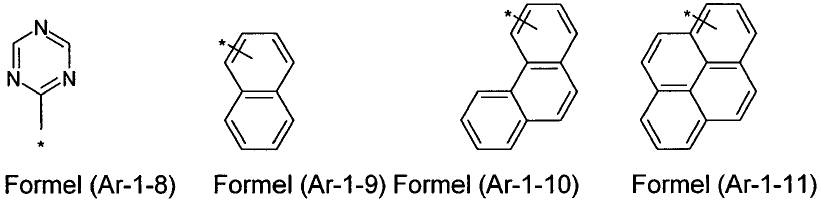 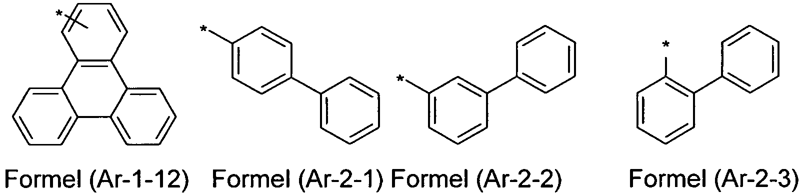 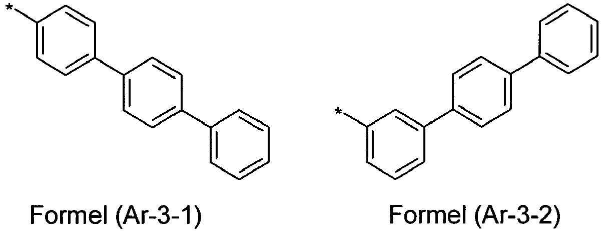 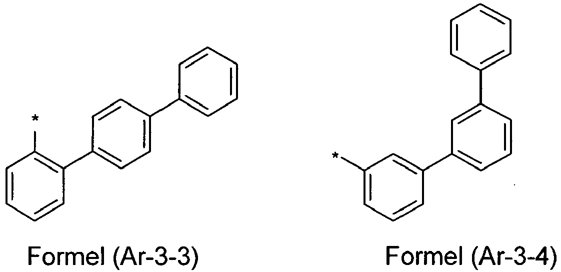 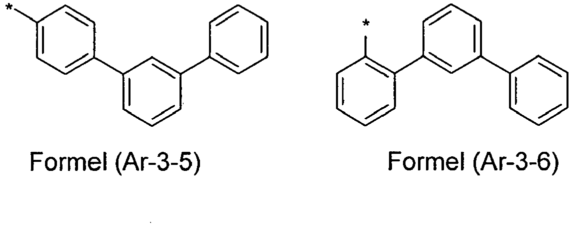 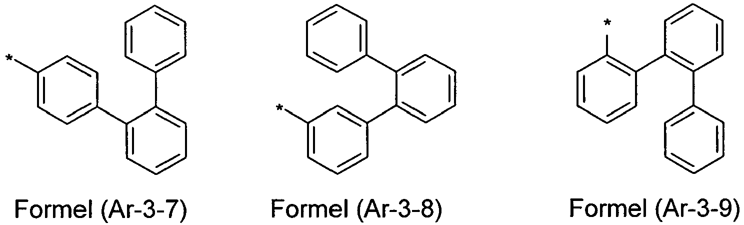 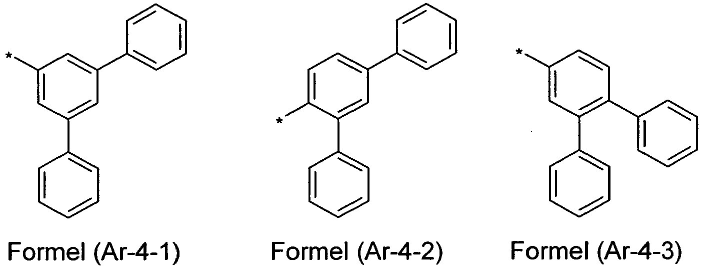 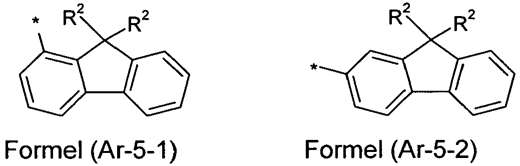 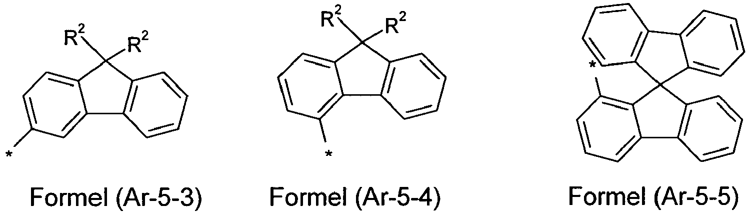 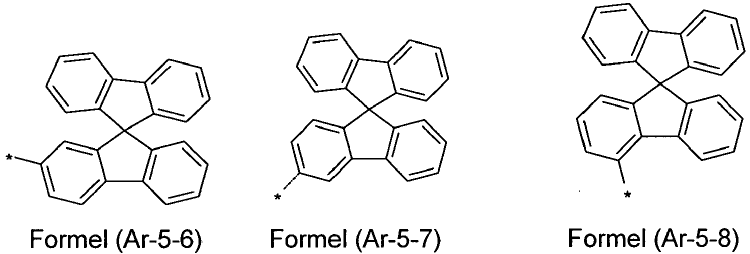 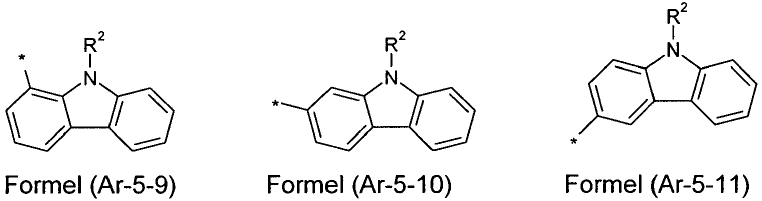 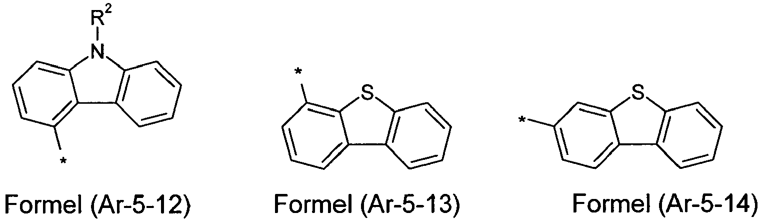 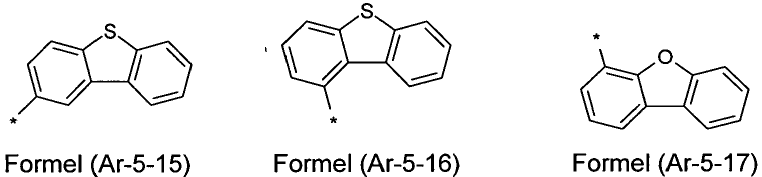 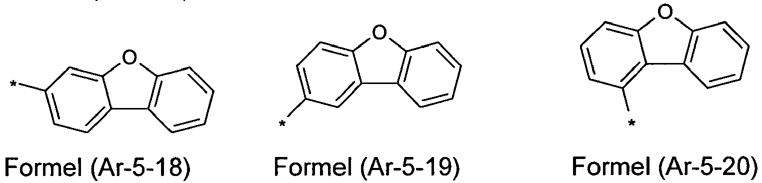 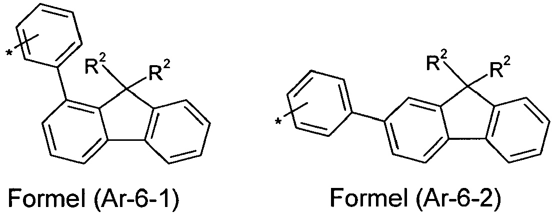 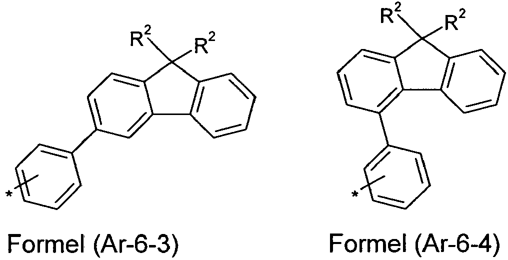 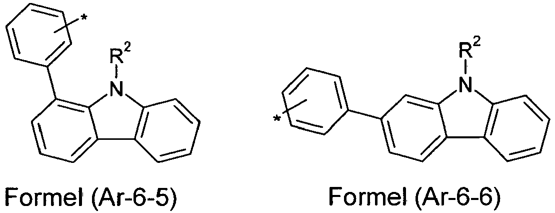 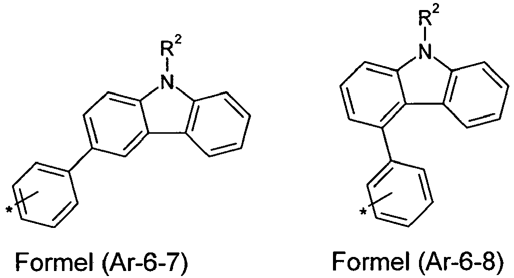 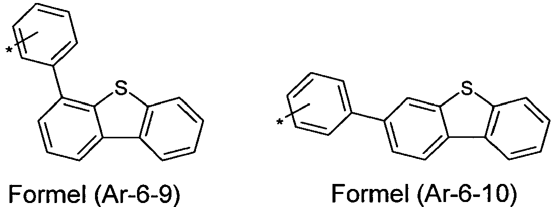 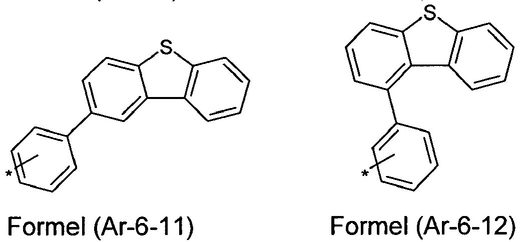 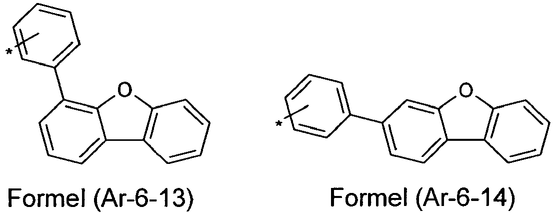 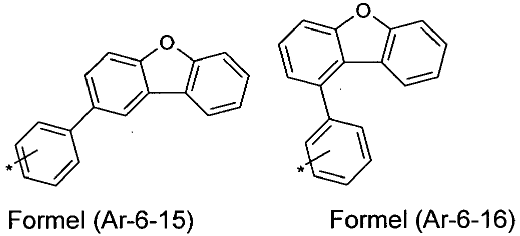 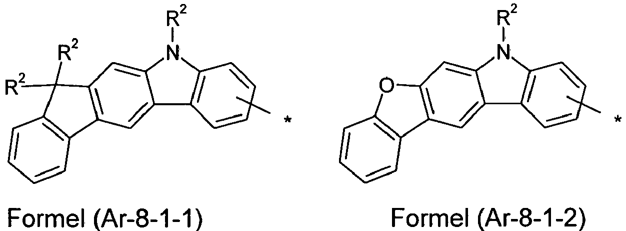 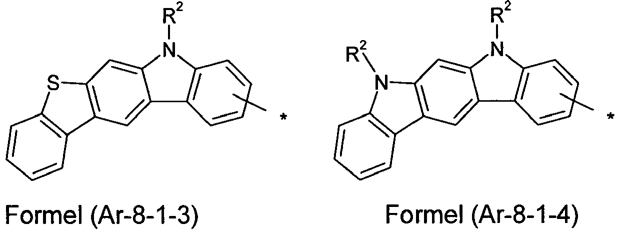 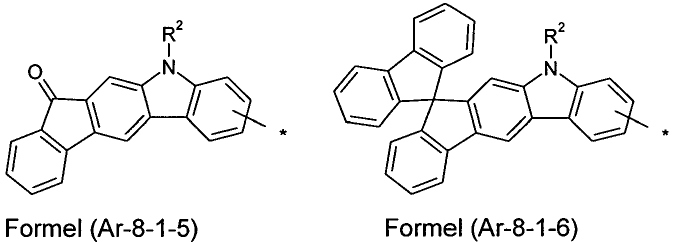 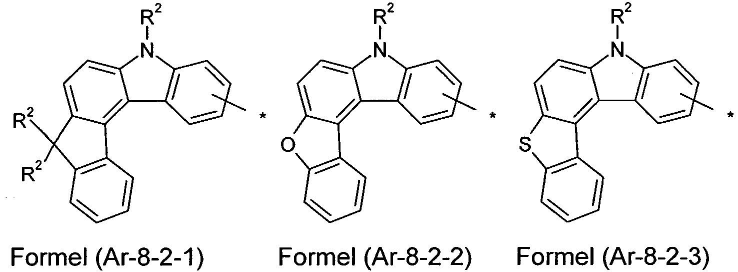 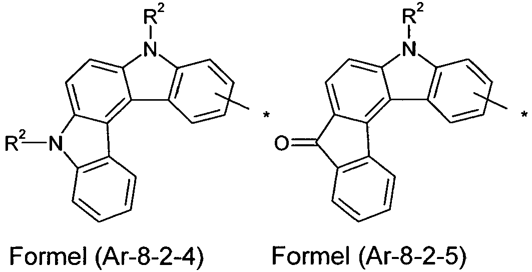 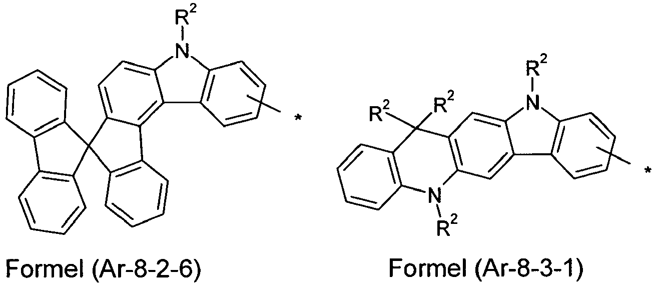 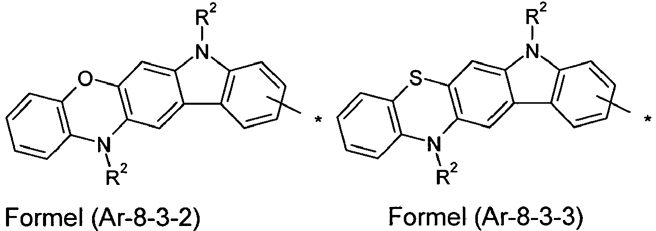 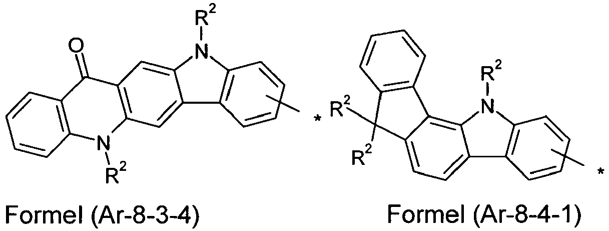 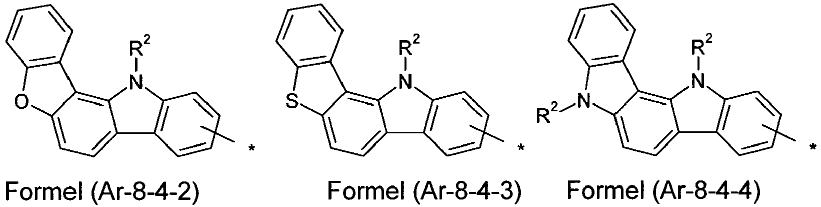 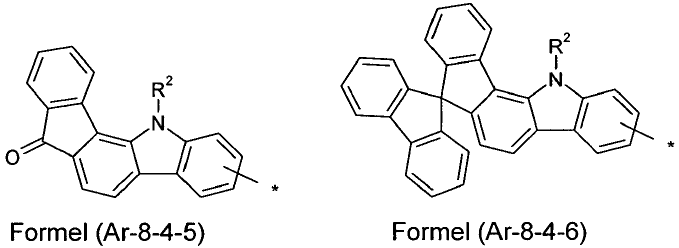 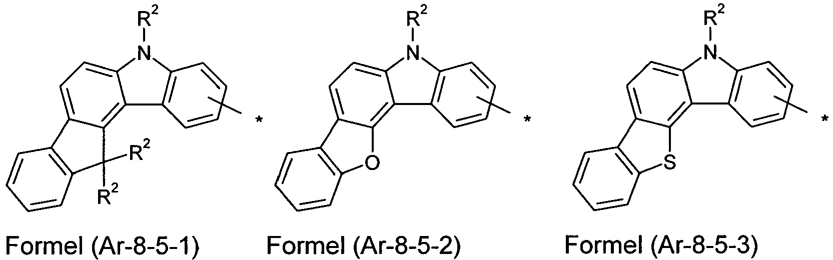 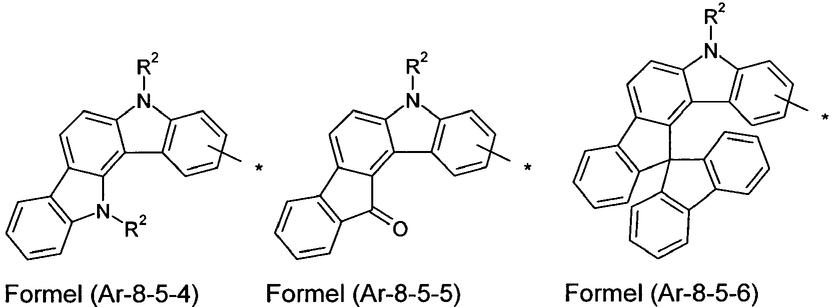 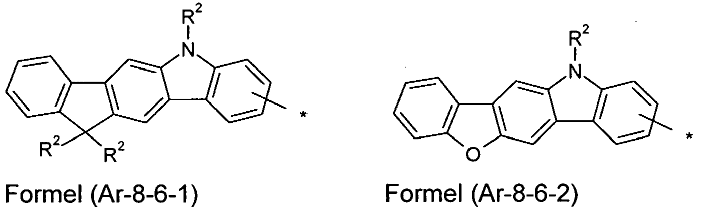 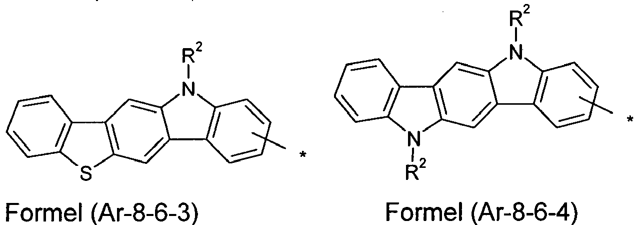 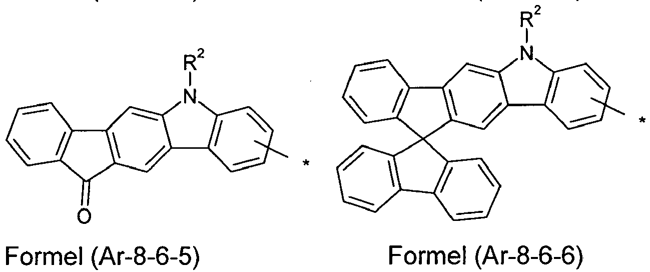 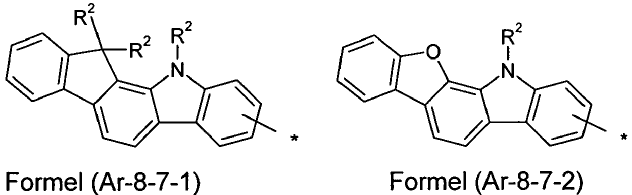 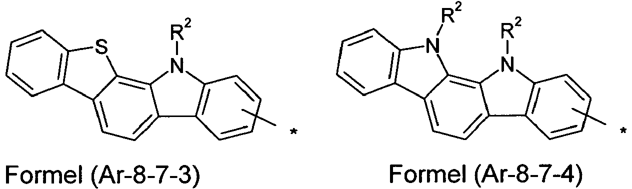 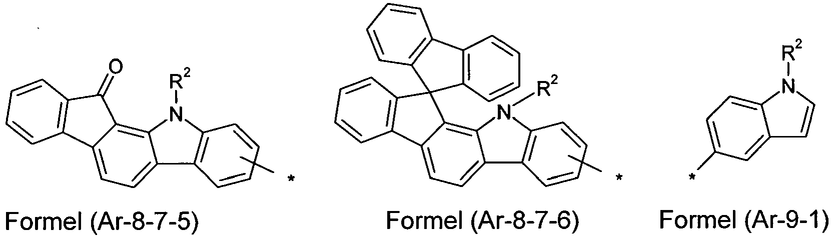 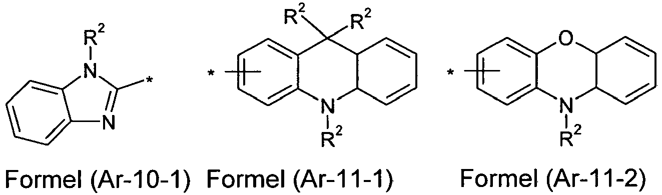 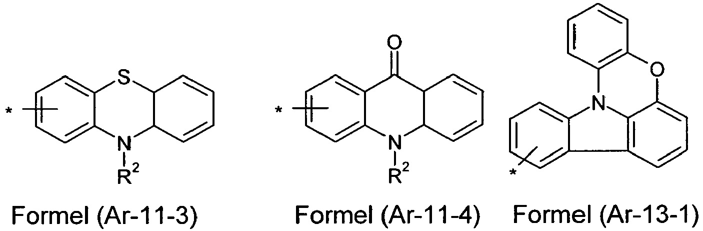 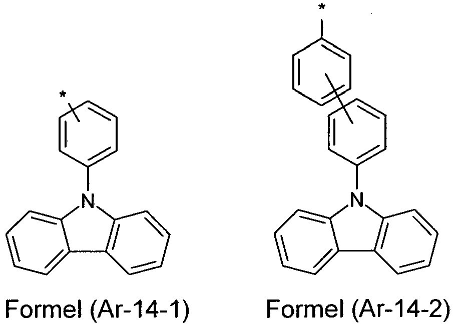 wobei die Symbole den Symbolen in den Formeln (Ar-1) bis (Ar-14) entsprechen. Die Formeln können an den freien Positionen mit R² substituiert sein.

In einer weiteren Ausführungsform der Erfindung ist, falls die Gruppen Ar¹ mit einer Gruppe K verbunden sind, die Gruppe N(Ar¹)₂ bevorzugt ausgewählt aus den Formeln (Ar-15) bis (Ar-18), wobei diese jeweils durch die bevorzugten Ausführungsformen gemäß den folgenden Formeln (Ar-15-1) bis (Ar-18-6) ersetzt werden: 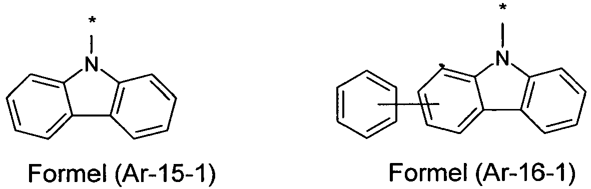 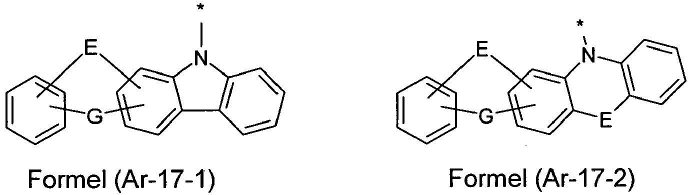 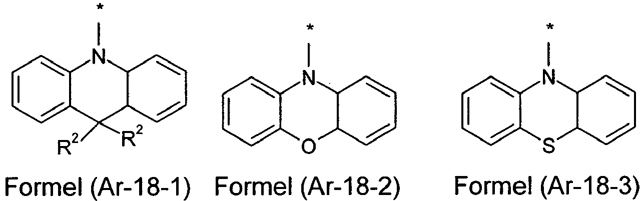 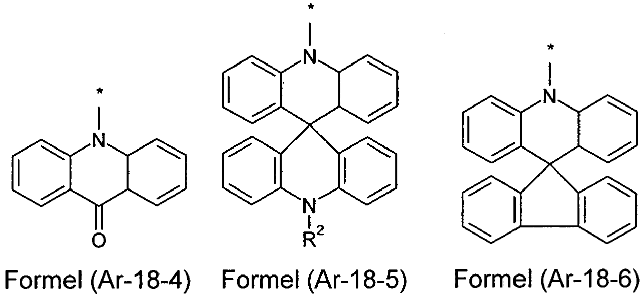 wobei die Symbole den Symbolen in den Formeln (Ar-15) bis (Ar-18) entsprechen. Die Formeln können an den freien Positionen mit R² substituiert sein.

In einer weiteren Ausführungsform der Erfindung sind die Gruppen gemäß Formel (Ar-8), bzw. deren bevorzugte Ausführungsformen ausgewählt aus den Gruppen gemäß einer der Formeln (Ar-8-1-1a) bis (Ar-8-7-6a) 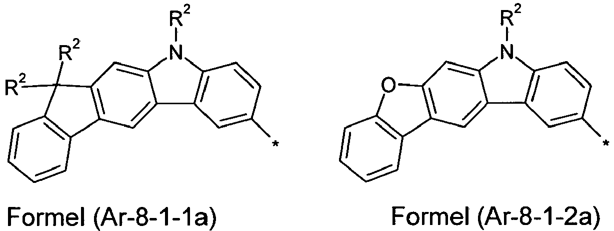 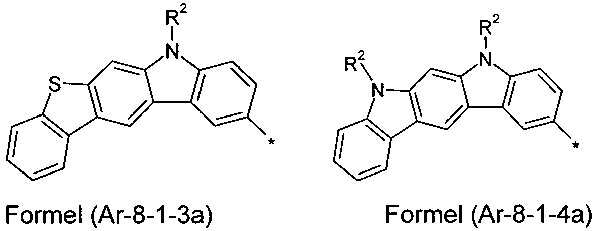 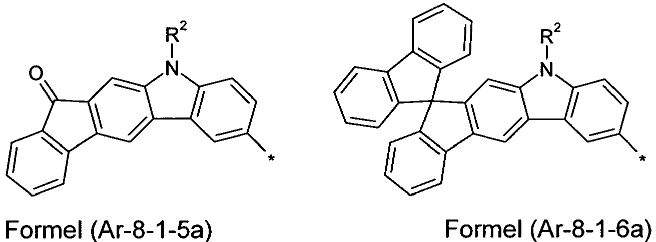 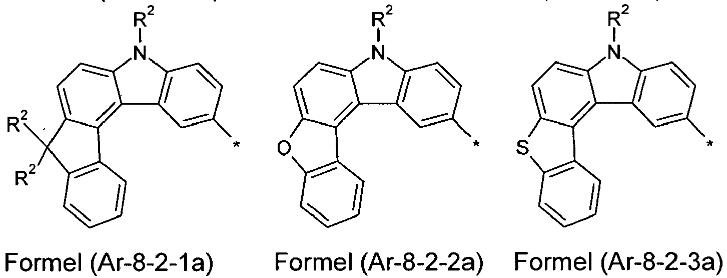 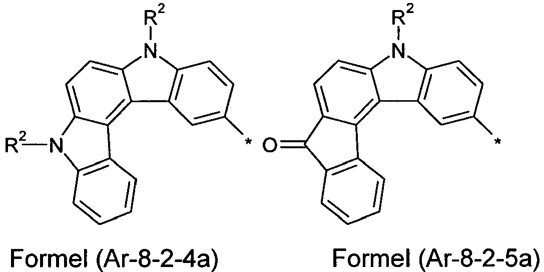 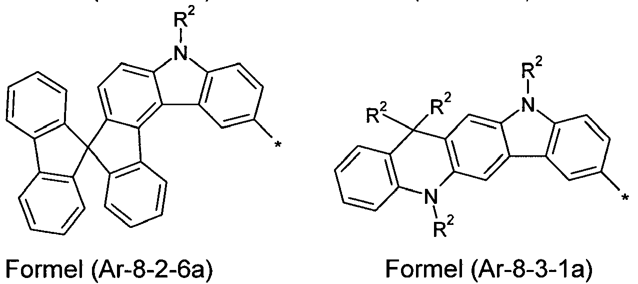 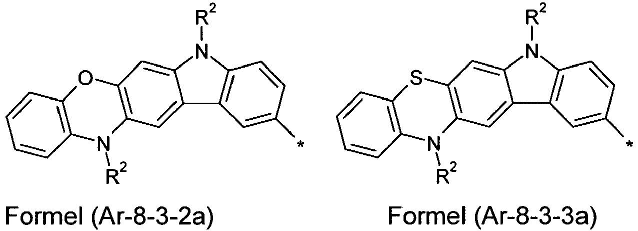 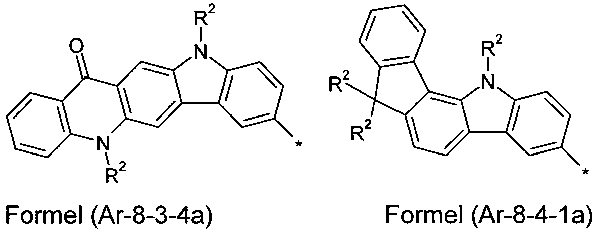 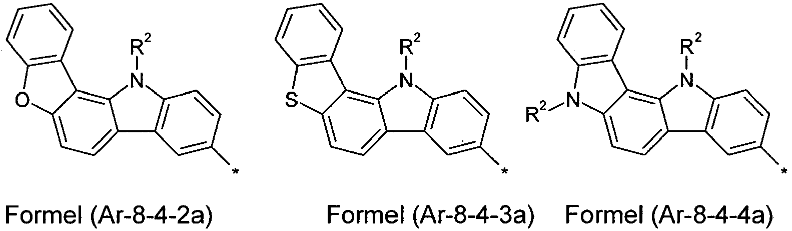 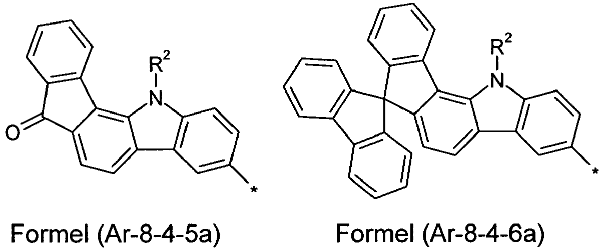 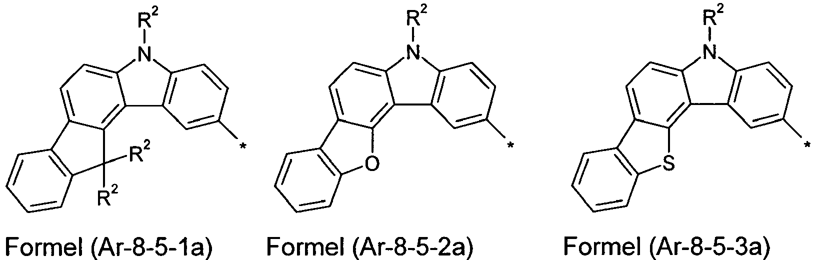 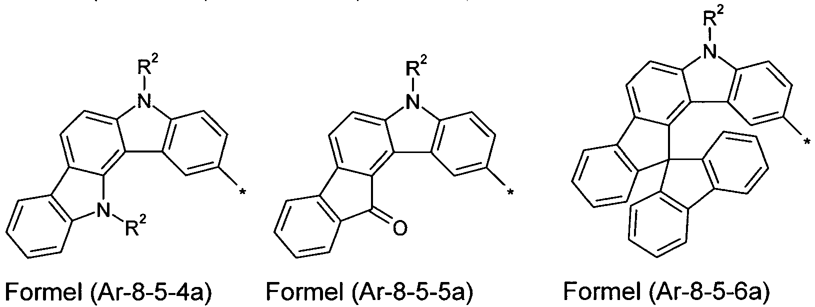 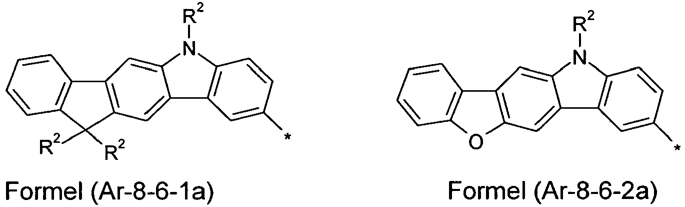 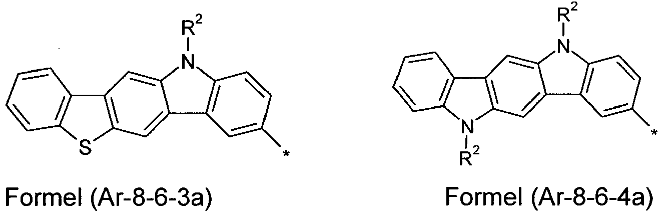 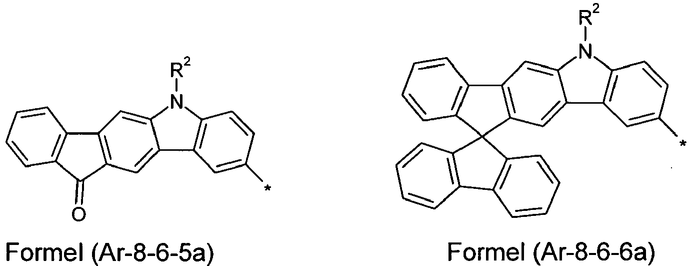 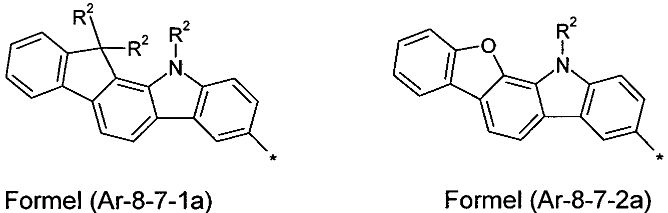 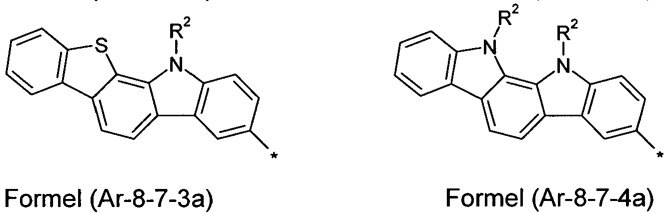 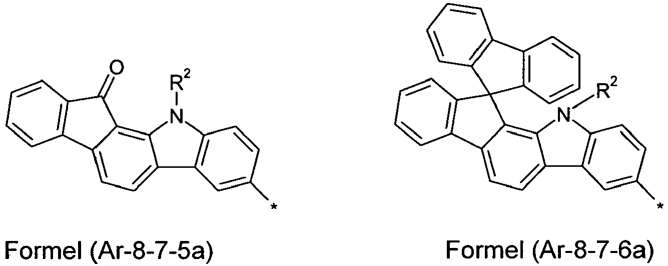 wobei die Symbole den Symbolen in Formel (Ar-8) entsprechen. Die Formeln können an den freien Positionen mit R² substituiert sein.

Wenn eine oder mehrere Gruppen Ar² vorhanden sind, stehen diese bevorzugt für ein bivalentes aromatisches oder heteroaromatisches Ringsystem mit 6 bis 24 aromatischen Ringatomen. Bevorzugte Gruppen Ar² sind gleich oder verschieden ausgewählt aus der Gruppe bestehend aus ortho-, meta- oder para-Benzol, ortho-, meta- oder para-Biphenyl, Terphenyl, insbesondere ortho-, meta- oder para-Terphenyl, Quaterphenyl, insbesondere ortho-, meta- oder para-Quaterphenyl, Fluoren, 9,9'-Spirobifluoren, Furan, Benzofuran, Dibenzofuran, Dibenzothiophen, Pyrrol, Indol oder Carbazol. Dabei können diese Gruppen mit einem oder mehreren Resten R² substituiert sein, sind aber bevorzugt unsubstituiert.

In einer weiteren Ausführungsform der Erfindung ist die Gruppe Ar² bei jedem Auftreten gleich oder verschieden ausgewählt aus den Formeln (Ar2-1) bis (Ar2-13): 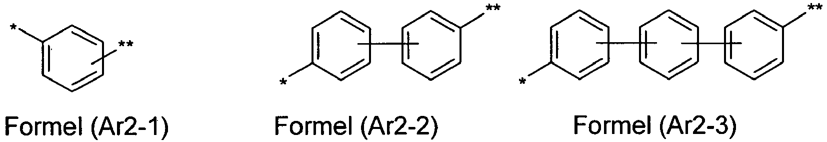 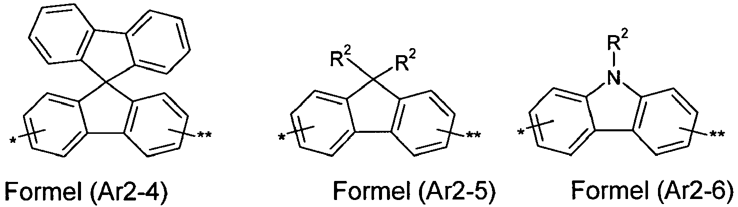 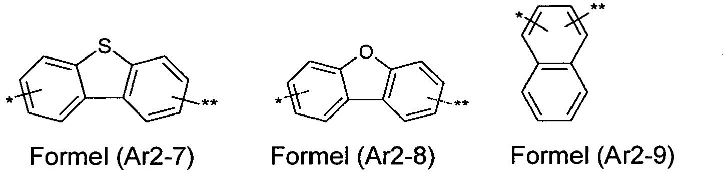 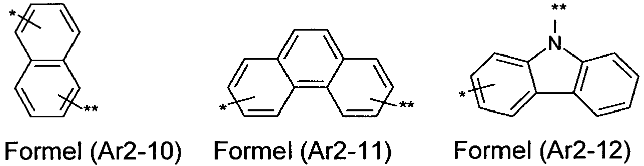 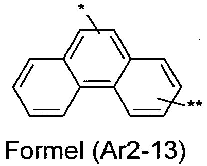 wobei die verwendeten Symbole für Formel (1) die genannten Bedeutungen aufweisen und die mit * und ** gekennzeichneten Bindungen, die beiden Bindungen des bivalenten aromatischen oder heteroaromatischen Ringsystems zu den benachbarten Gruppen darstellt. Es handelt sich dabei bevorzugt um eine C-C, C-N oder N-C-Bindung. Die Gruppen können dabei an den freien Positionen mit R² substituiert sein. Bevorzugt sind sie unsubstituiert.

In einer weiteren Ausführungsform der Erfindung sind die Gruppen Ar¹ bei jedem Auftreten gleich oder verschieden ausgewählt aus den Formeln (Ar-1) bis (Ar-15) oder ihren bevorzugten Ausführungsformen, und sind die Gruppen Ar² soweit vorhanden gleich oder verschieden ausgewählt aus den Formeln (Ar2-1) bis (Ar2-13).

In einer bevorzugten Ausführungsform der Erfindung ist R² gleich oder verschieden bei jedem Auftreten ausgewählt aus der Gruppe bestehend aus H, D, F, Si(R³)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R³ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 6 bis 60 aromatischen Ringatomen, das jeweils mit einem oder mehreren Resten R³ substituiert sein kann.

In einer besonders bevorzugten Ausführungsform der Erfindung ist R² gleich oder verschieden bei jedem Auftreten ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, einem aromatischen oder heteroaromatischen Ringsystem mit 6 bis 60 C-Atomen, das jeweils mit einem oder mehreren Resten R³ substituiert sein kann.

In einer weiteren bevorzugten Ausführungsform ist R², welcher an eine Kohlenstoffbrücke in einem aromatischen oder heteroaromatischen Ringsystem bindet, wie beispielsweise in den Formeln (Ar-5-1), (Ar-5-2), (Ar-5-3), (Ar-5-4), (Ar-6-1), (Ar-6-2), (Ar-6-3), (Ar-6-4), (Ar-8-3-1), (Ar-11-1), (Ar-18-1) oder (Ar2-2), gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das wie oben definiert ist und das mit einem oder mehreren Resten R³ substituiert sein kann.

In einer weiteren bevorzugten Ausführungsform ist R², welcher an ein Stickstoffatom bindet, ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, insbesondere ein aromatisches Ringsystem mit 6 bis 24 C-Atomen, das wie oben definiert ist, und das mit einem oder mehreren Resten R³ substituiert sein kann.

In einer weiteren bevorzugten Ausführungsform ist R², wenn es ein aromatisches oder heteroaromatisches Ringsystem steht, ausgewählt aus den Formeln (Ar-1) bis (Ar-15) oder N(Ar)₂.

Die oben genannten Ausführungsformen können beliebig miteinander kombiniert werden. Insbesondere ist es bevorzugt, die oben aufgeführten bevorzugten Ausführungsformen miteinander zu kombinieren.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die folgenden Verbindungen:

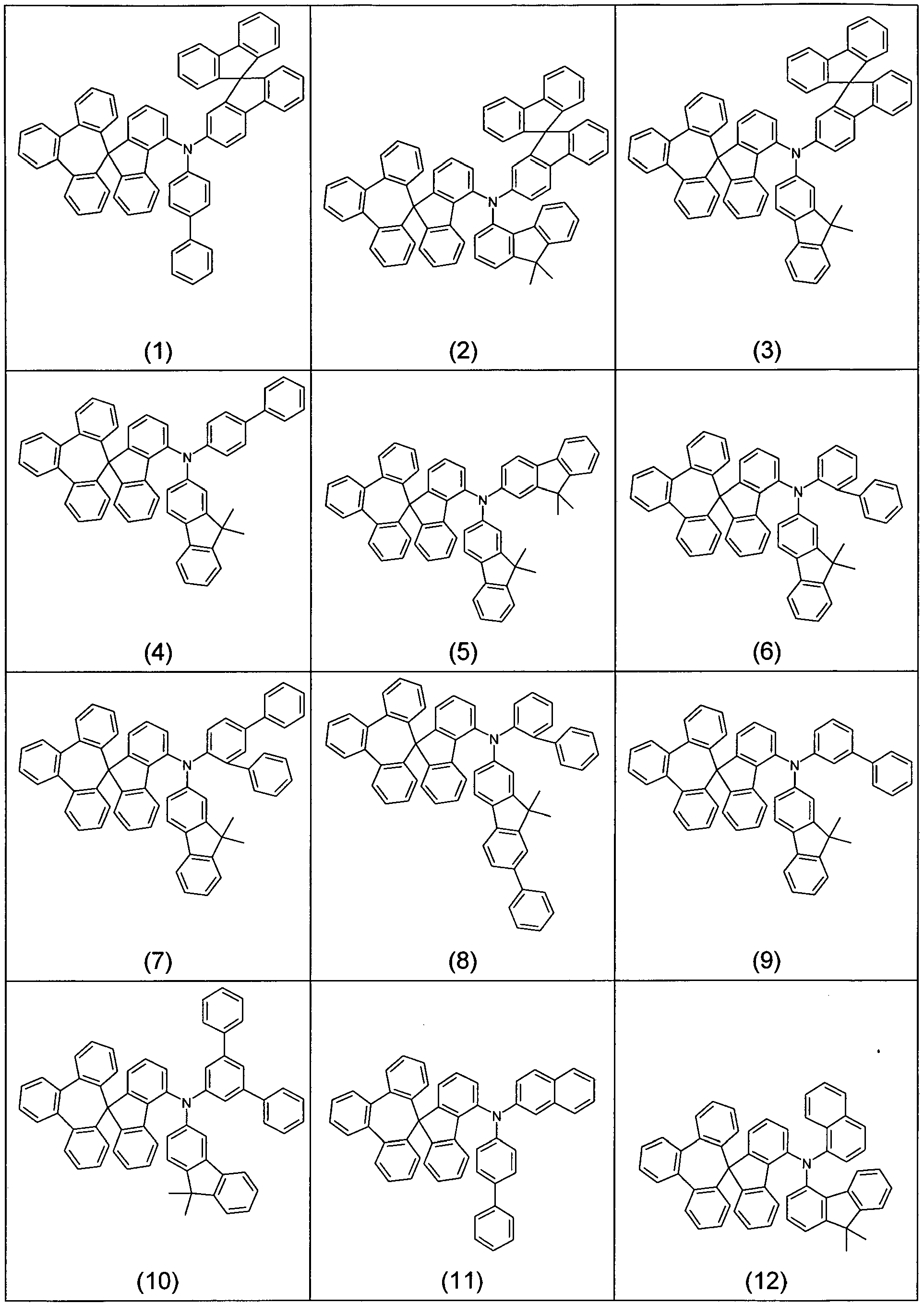

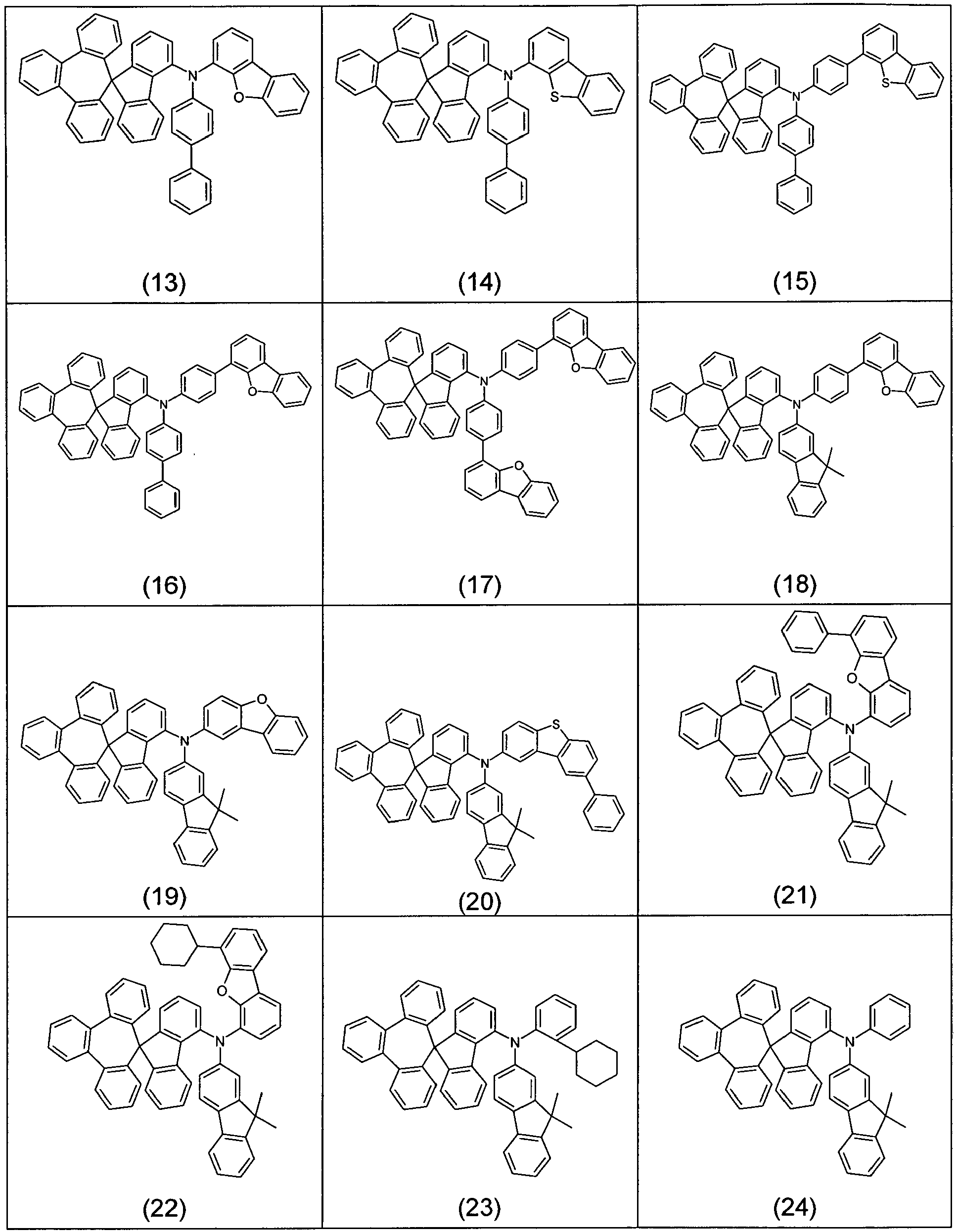

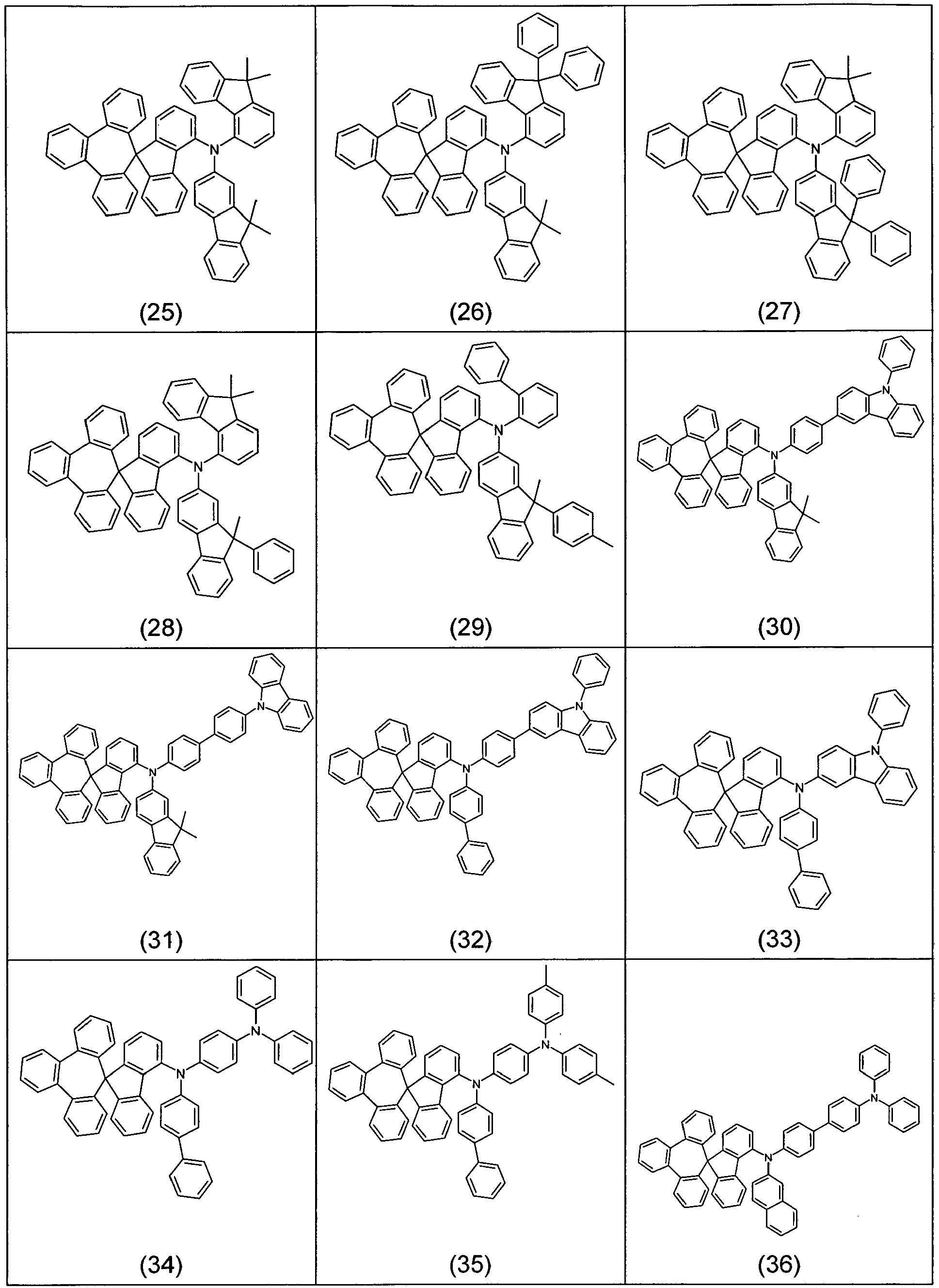

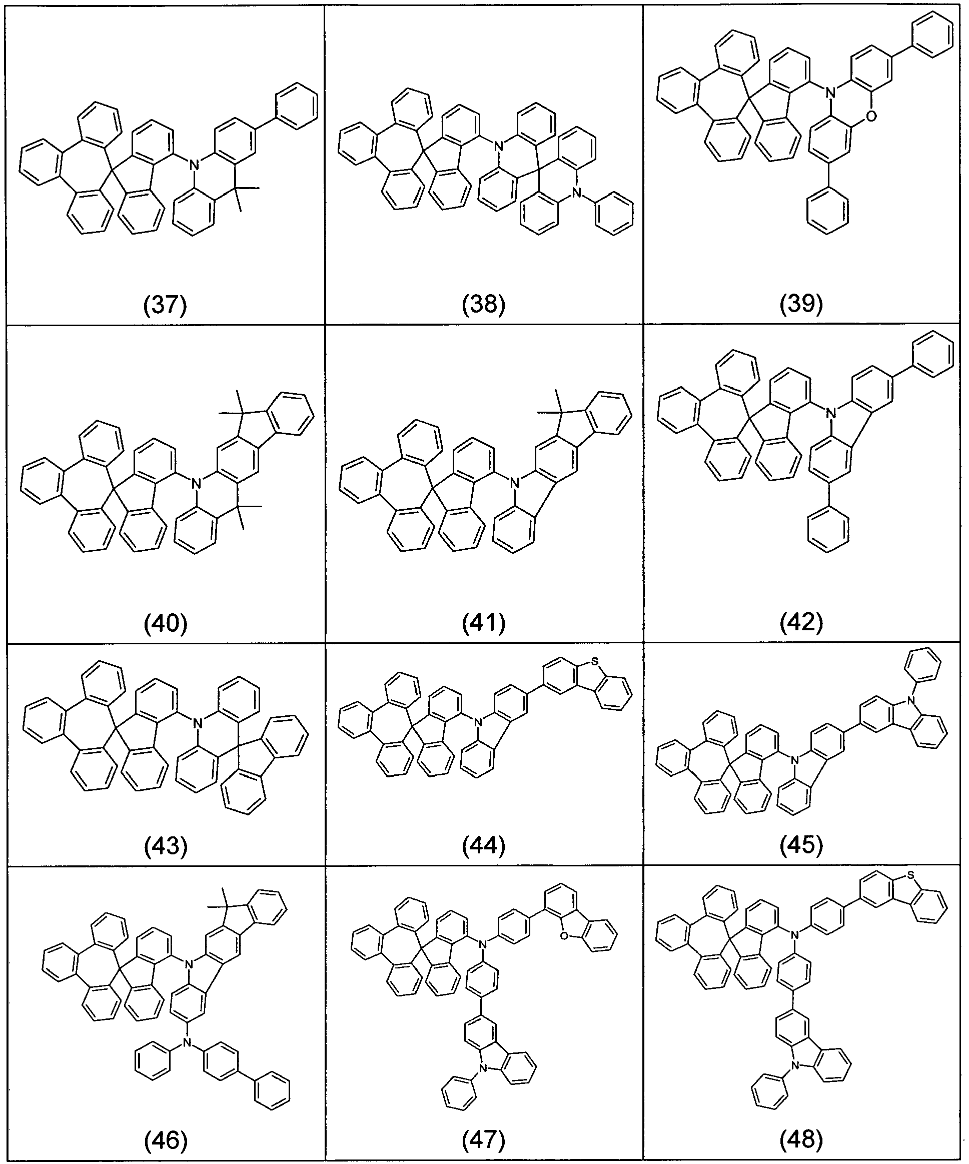

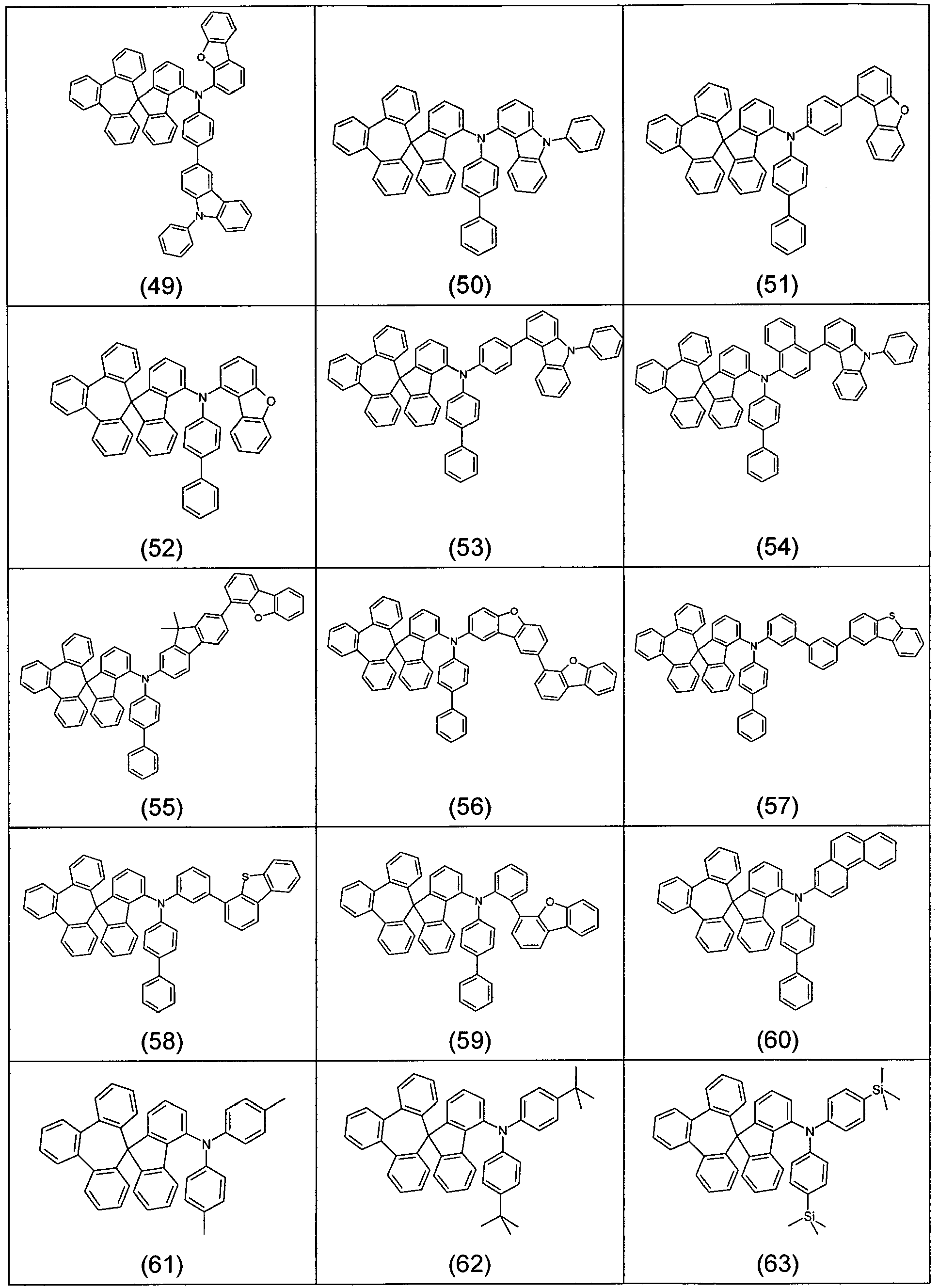

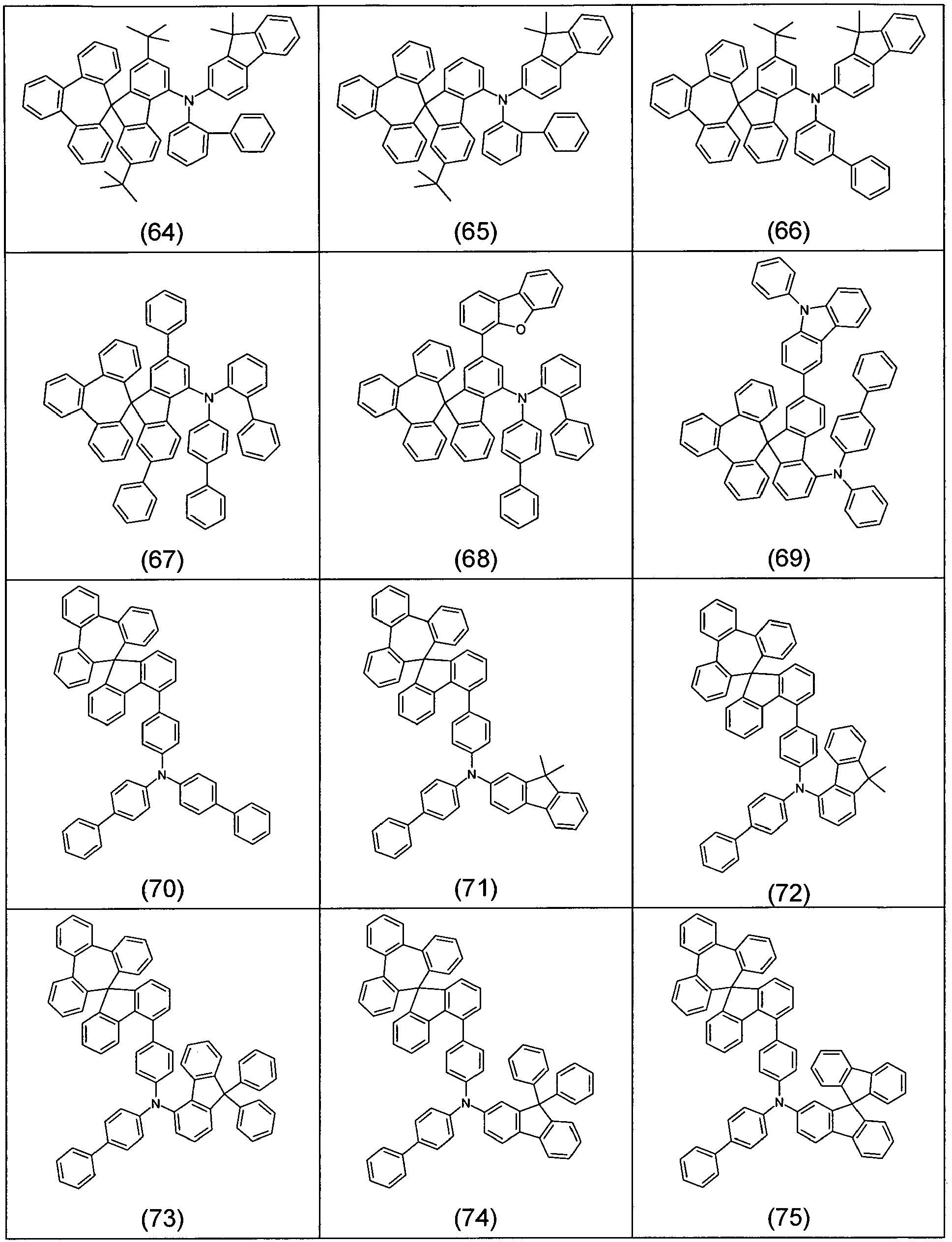

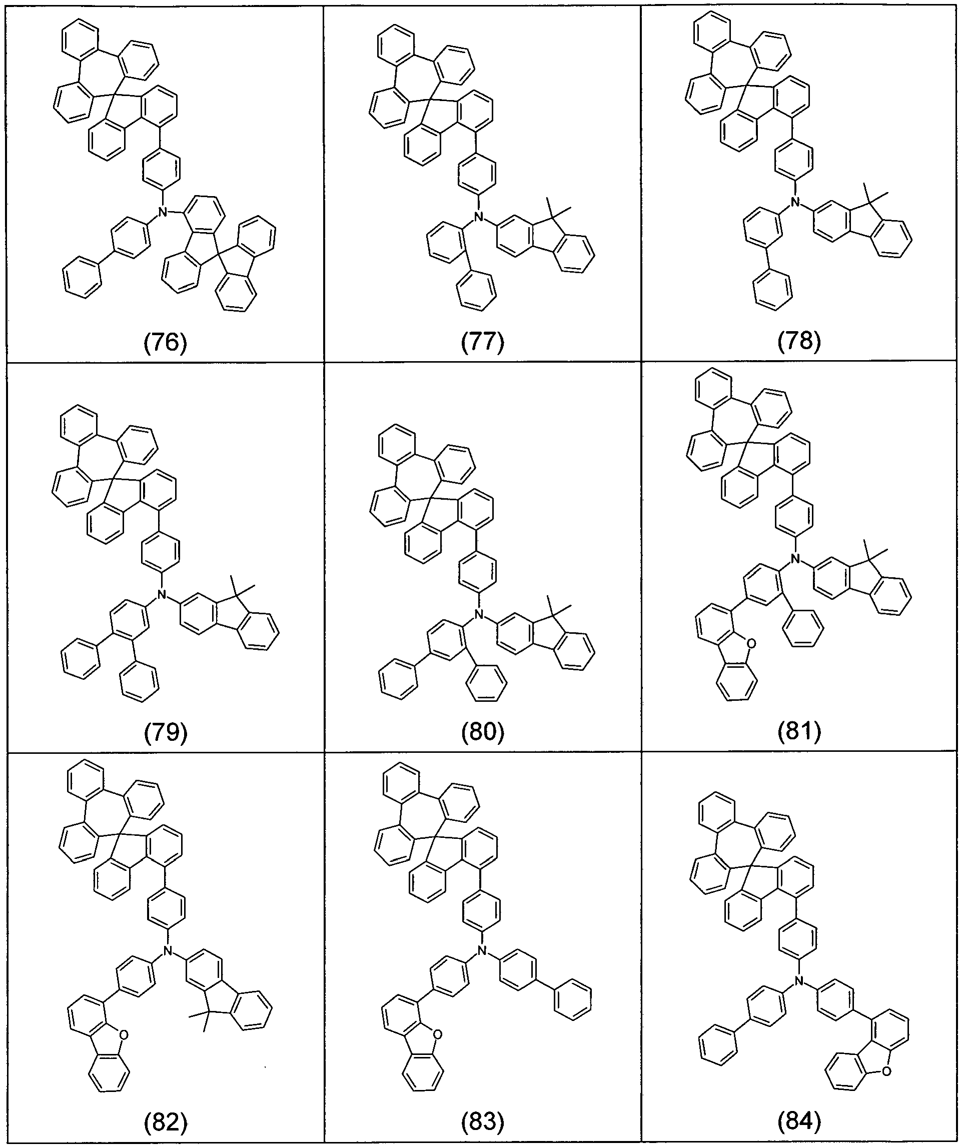

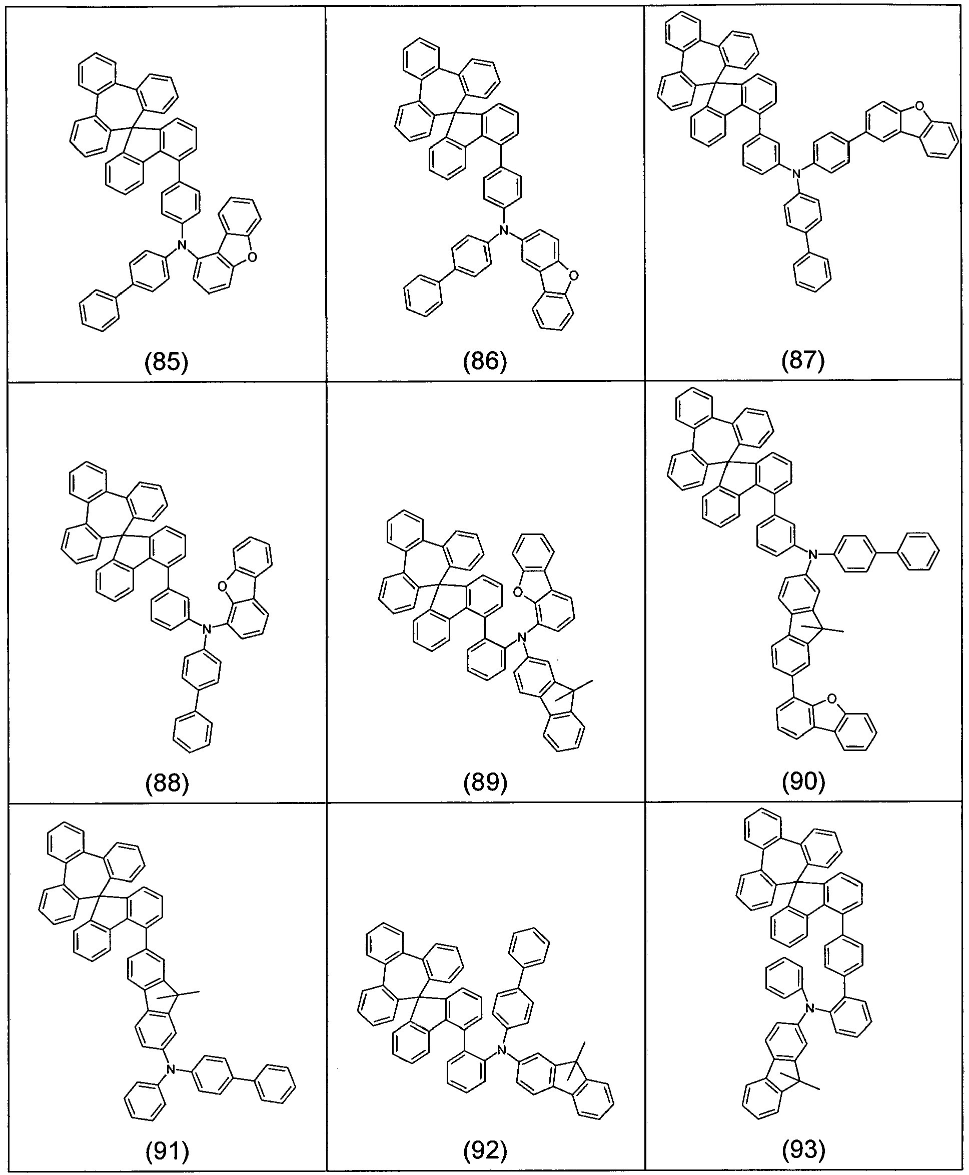

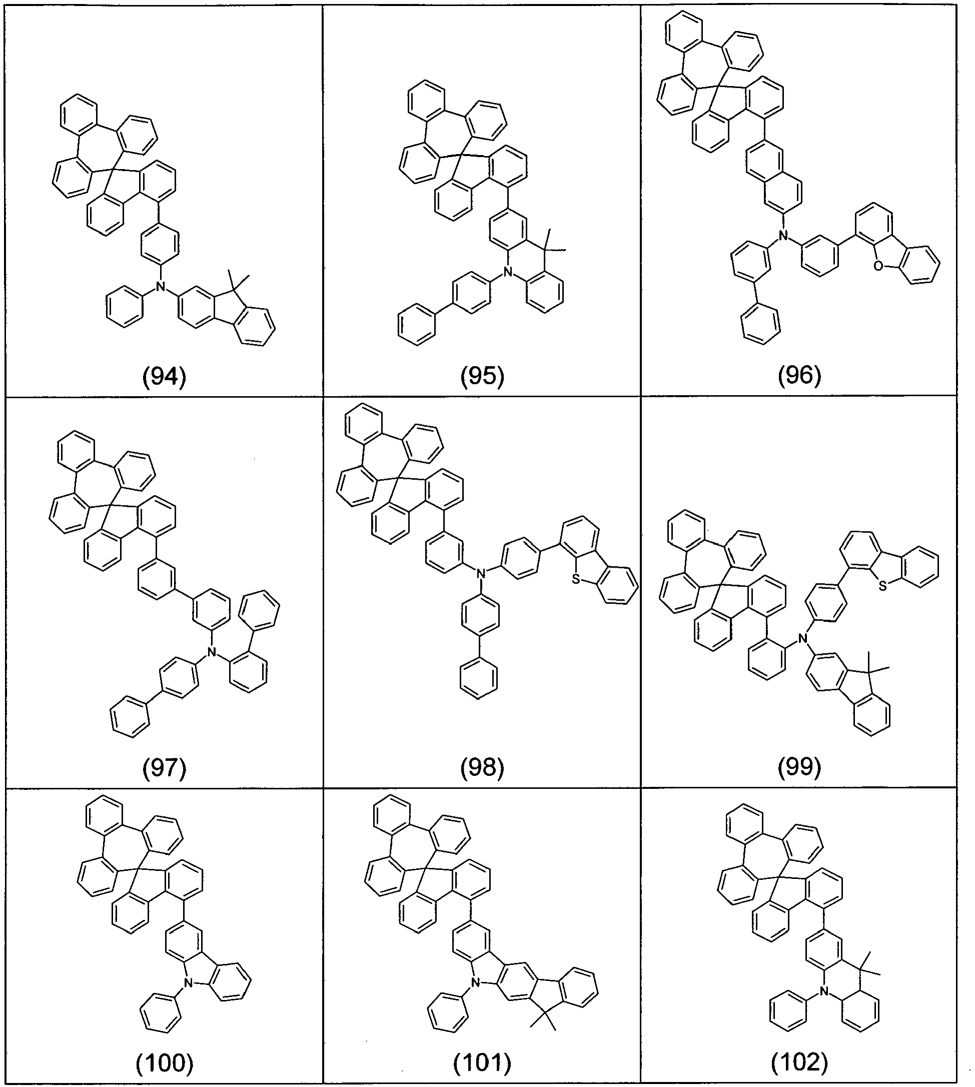

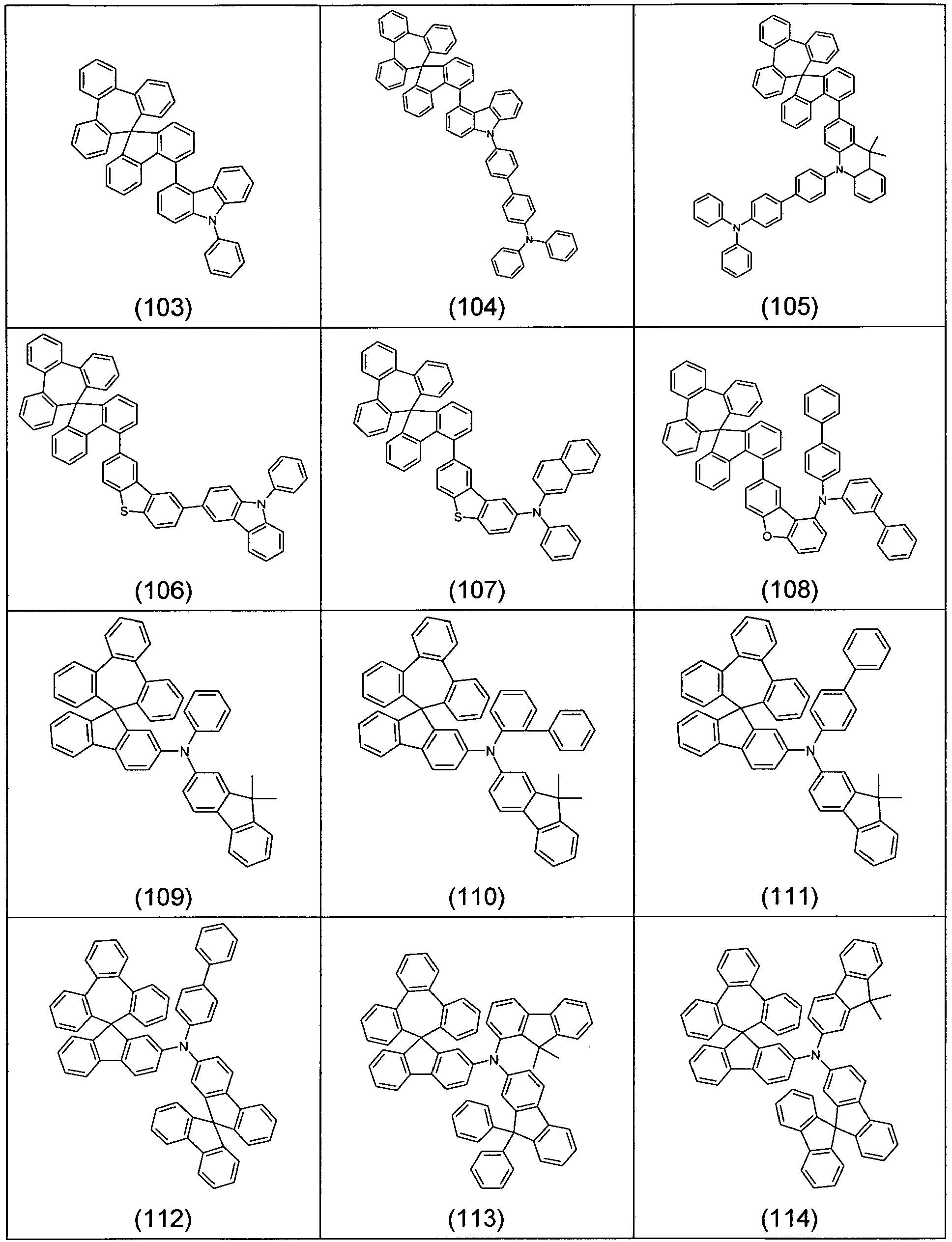

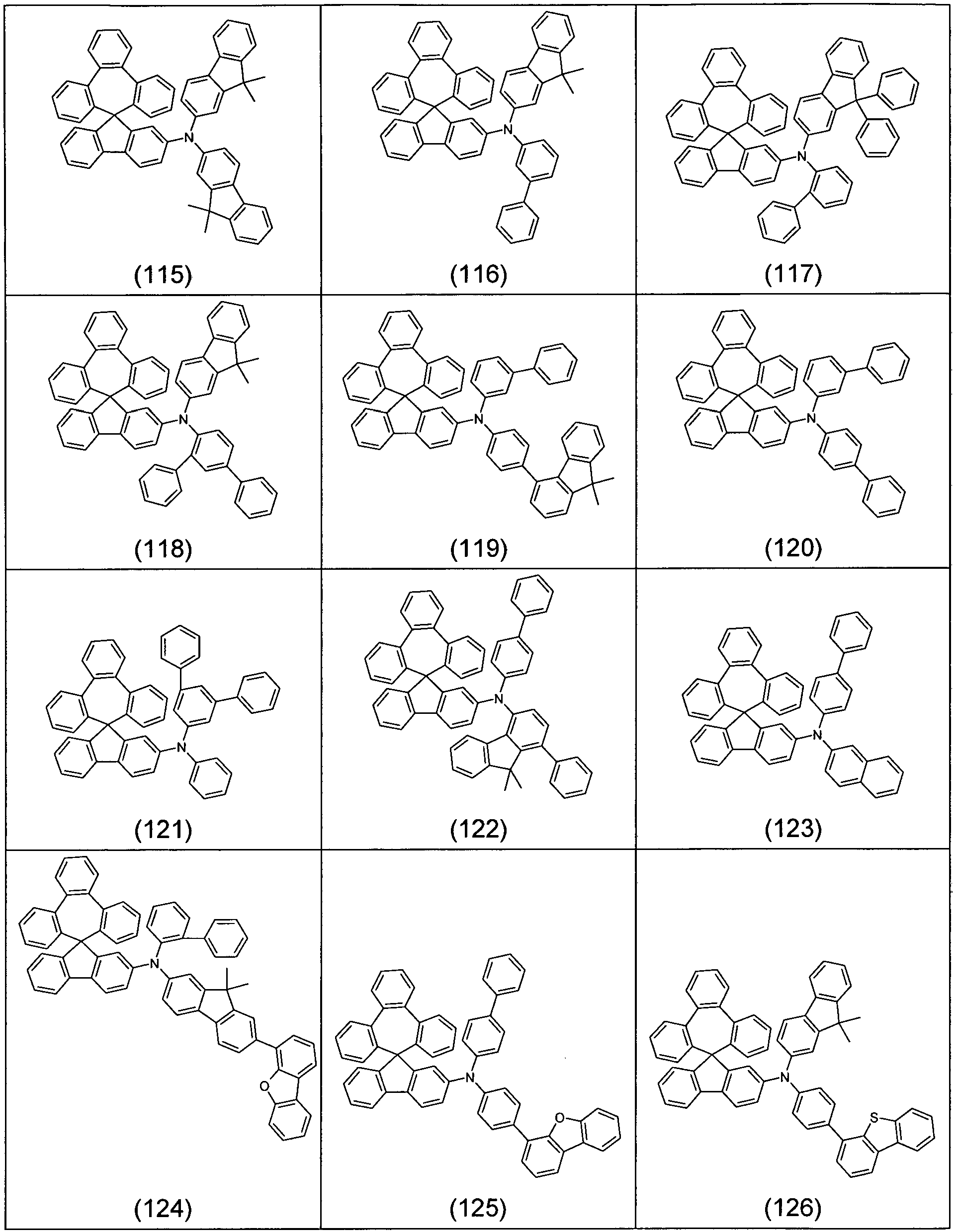

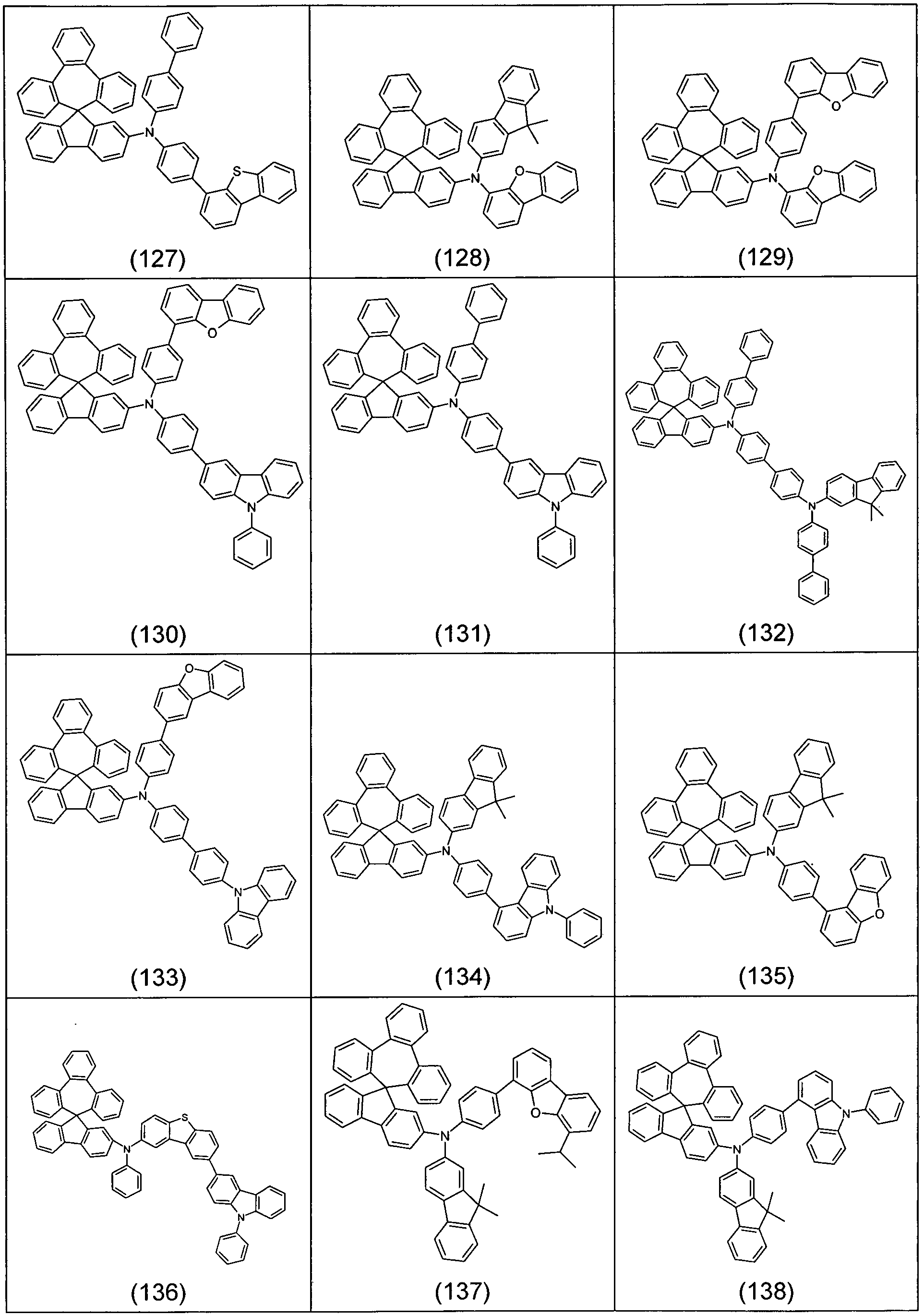

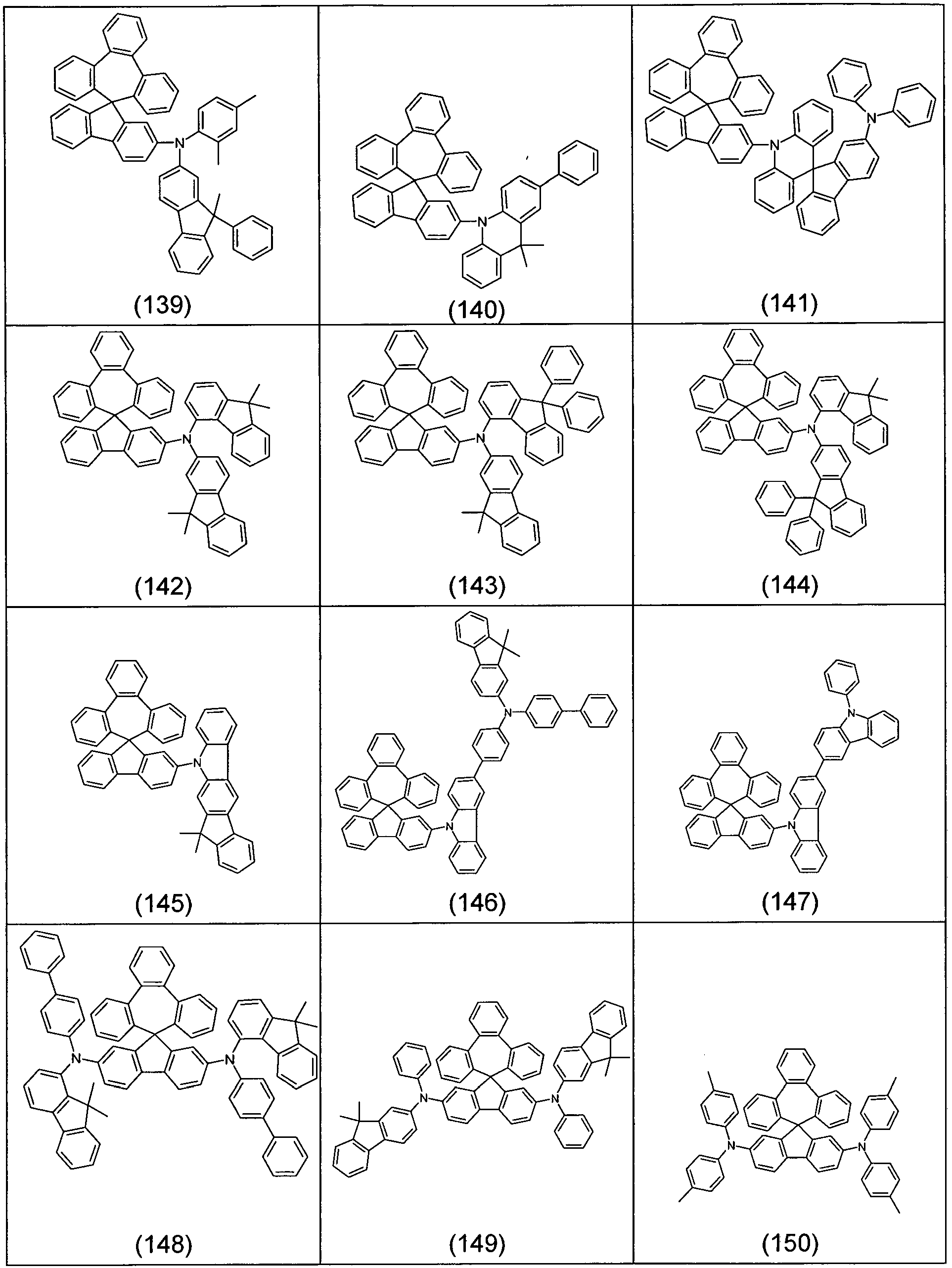

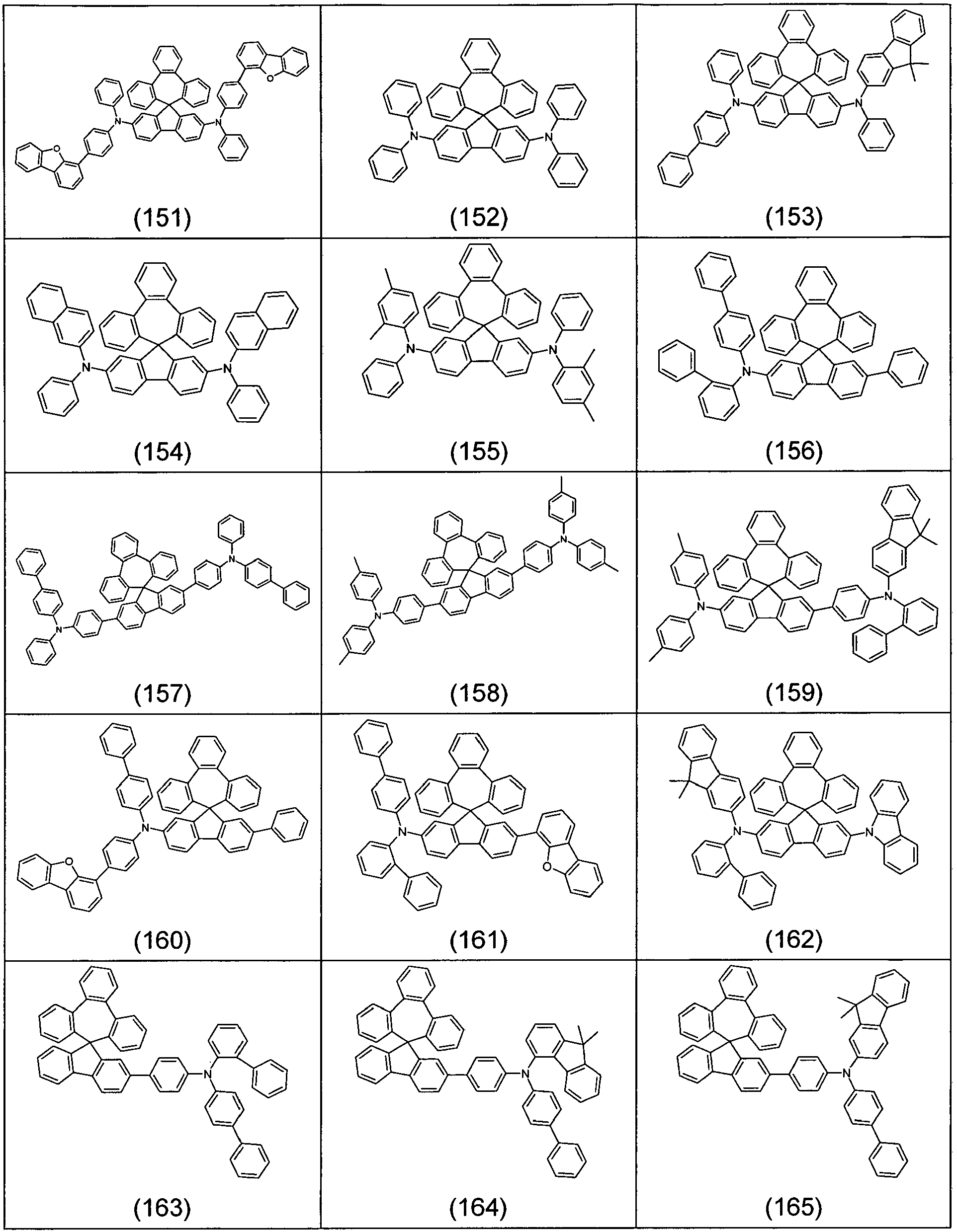

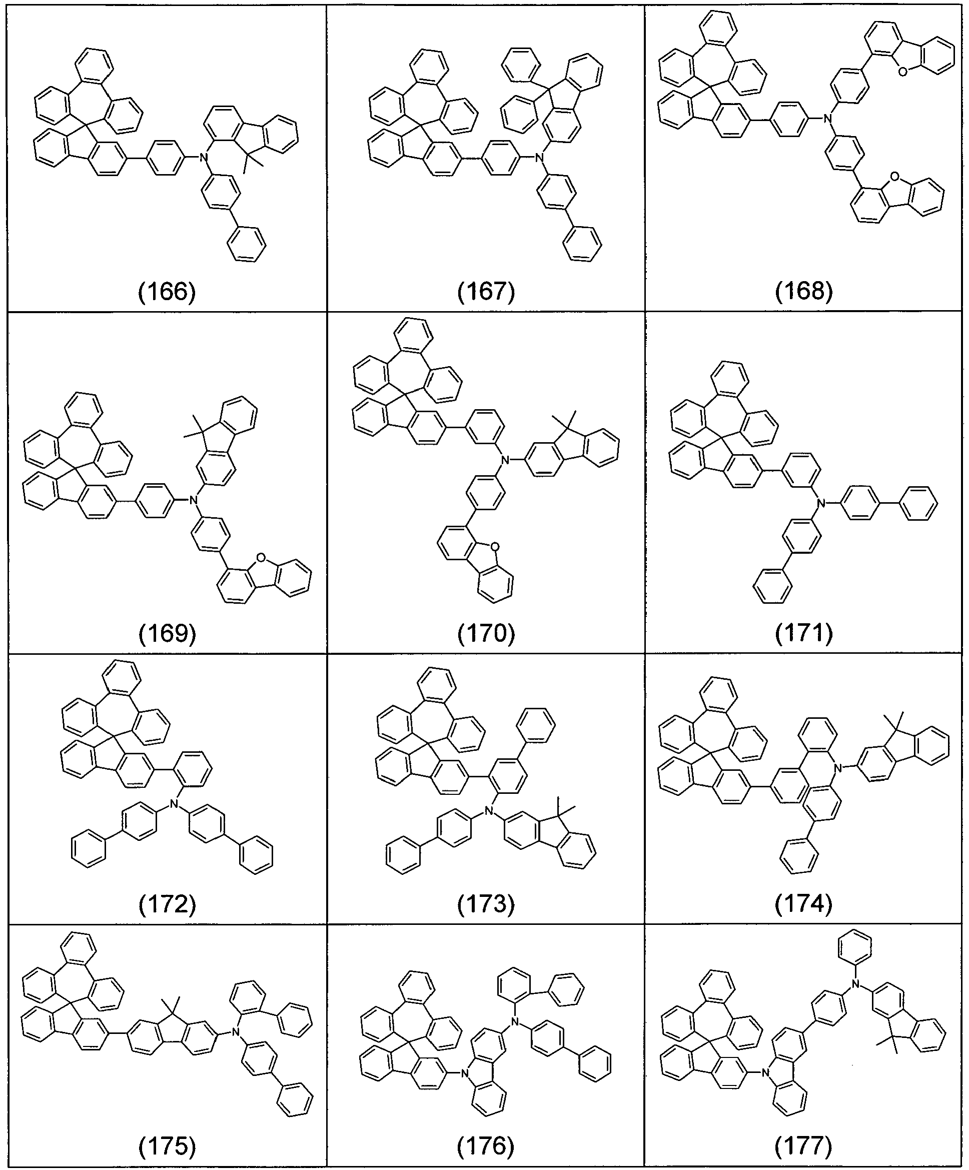

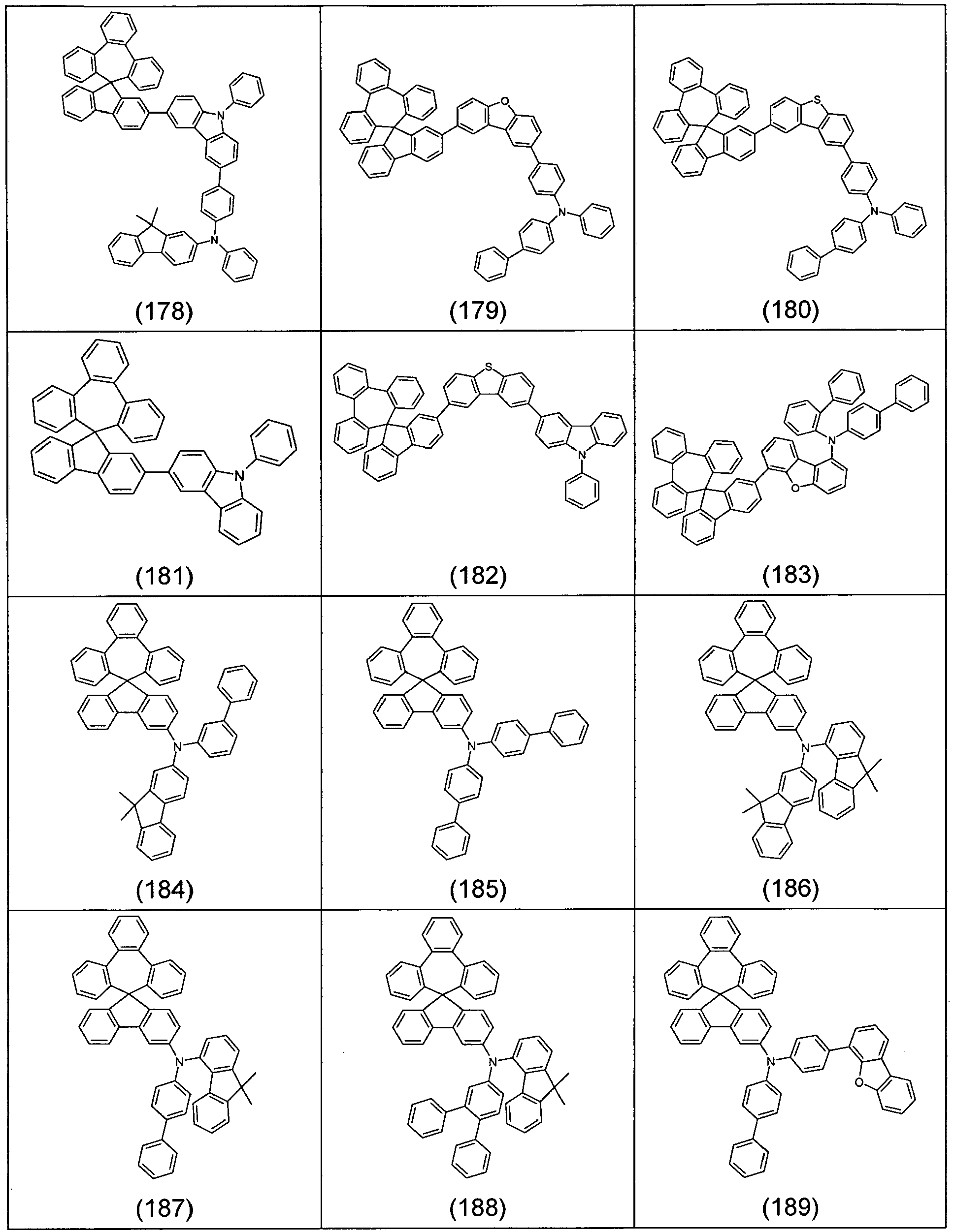

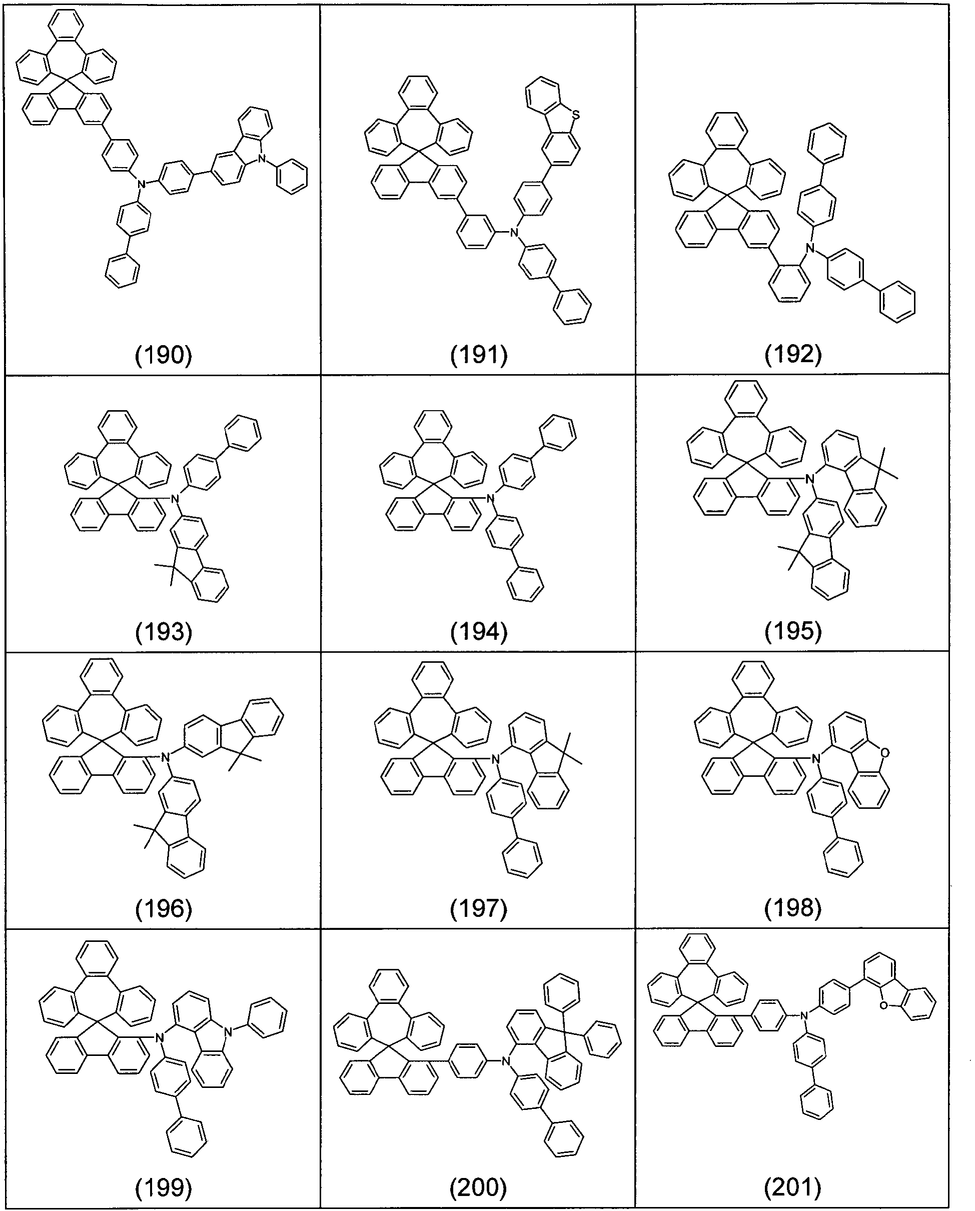

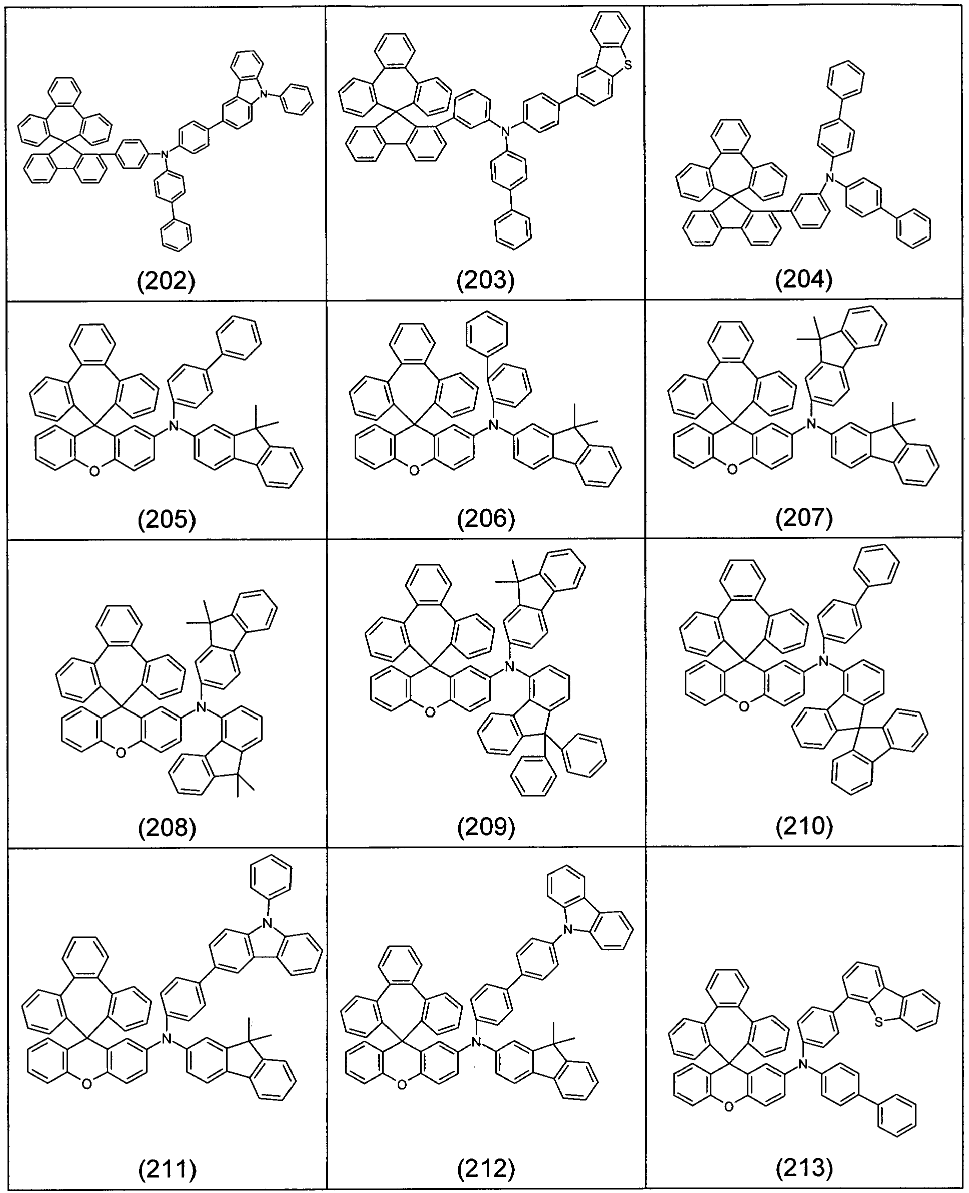

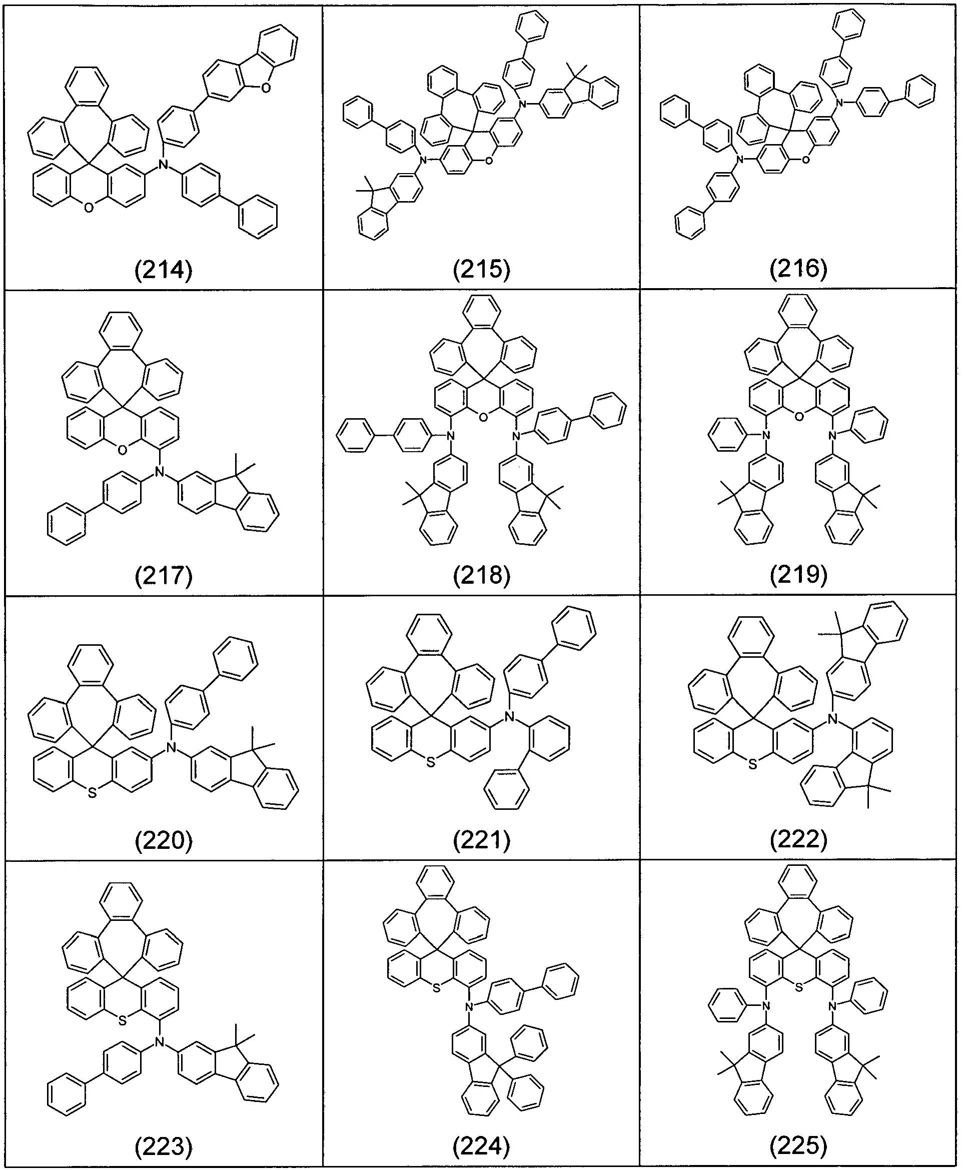

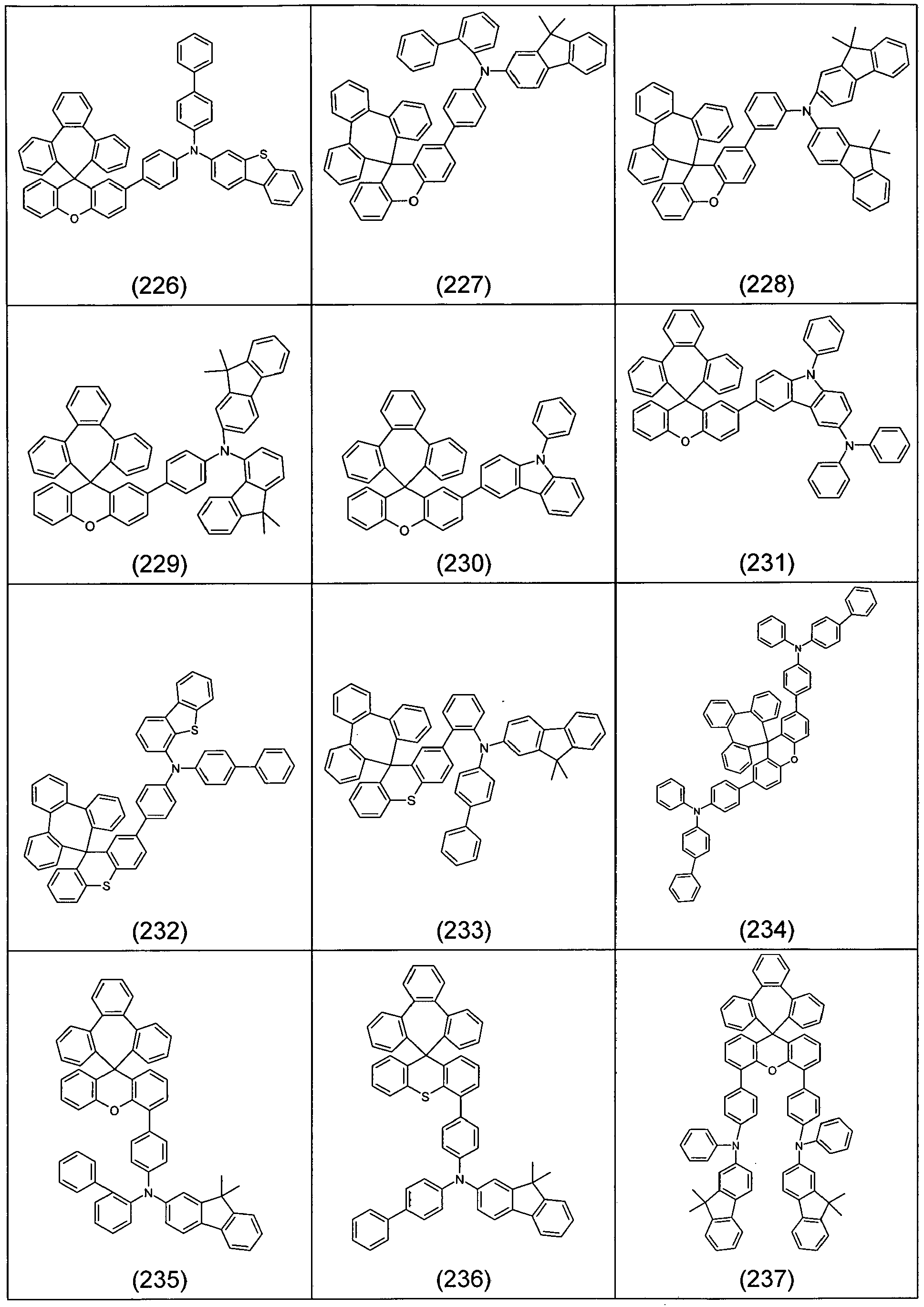

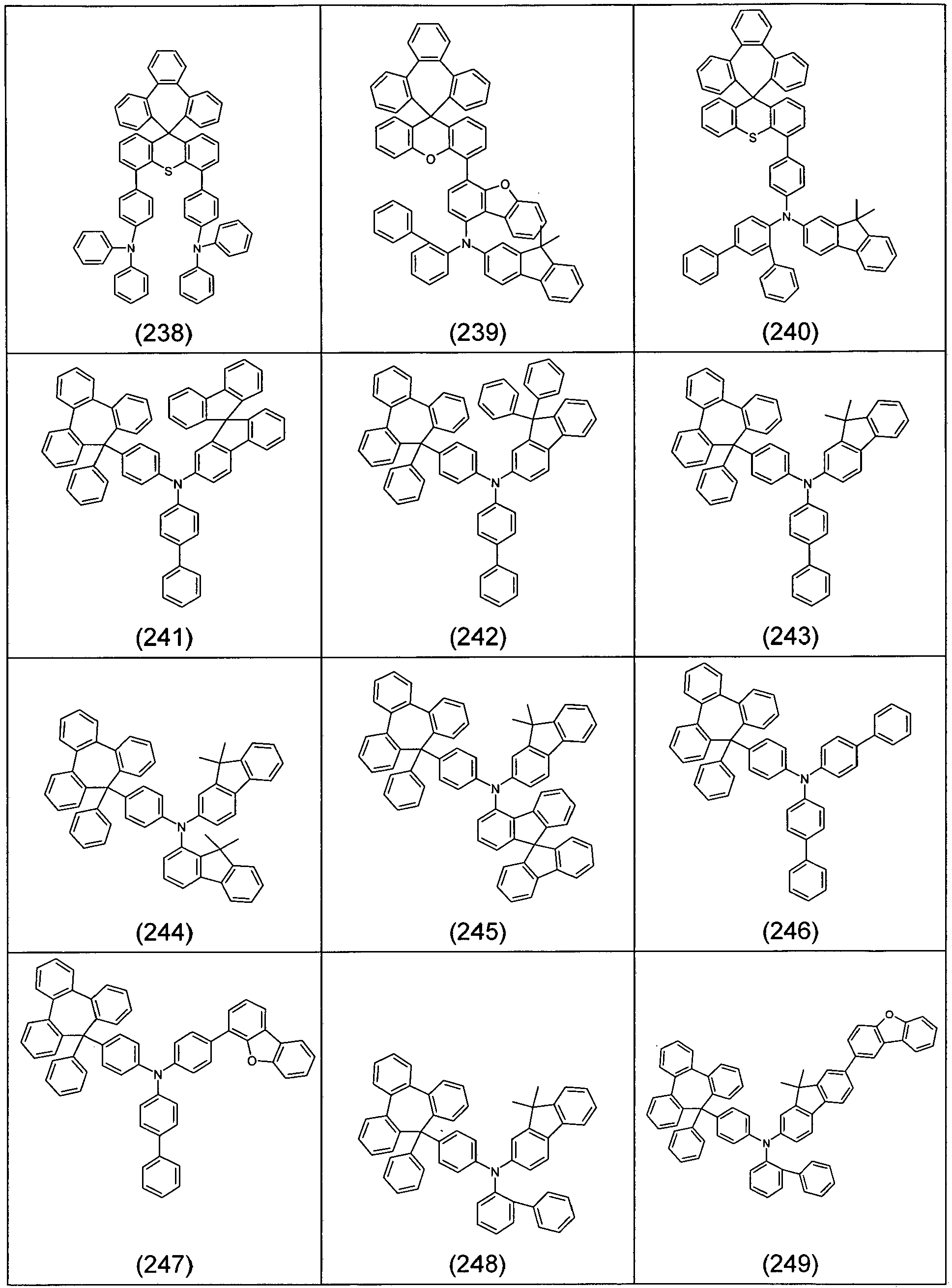

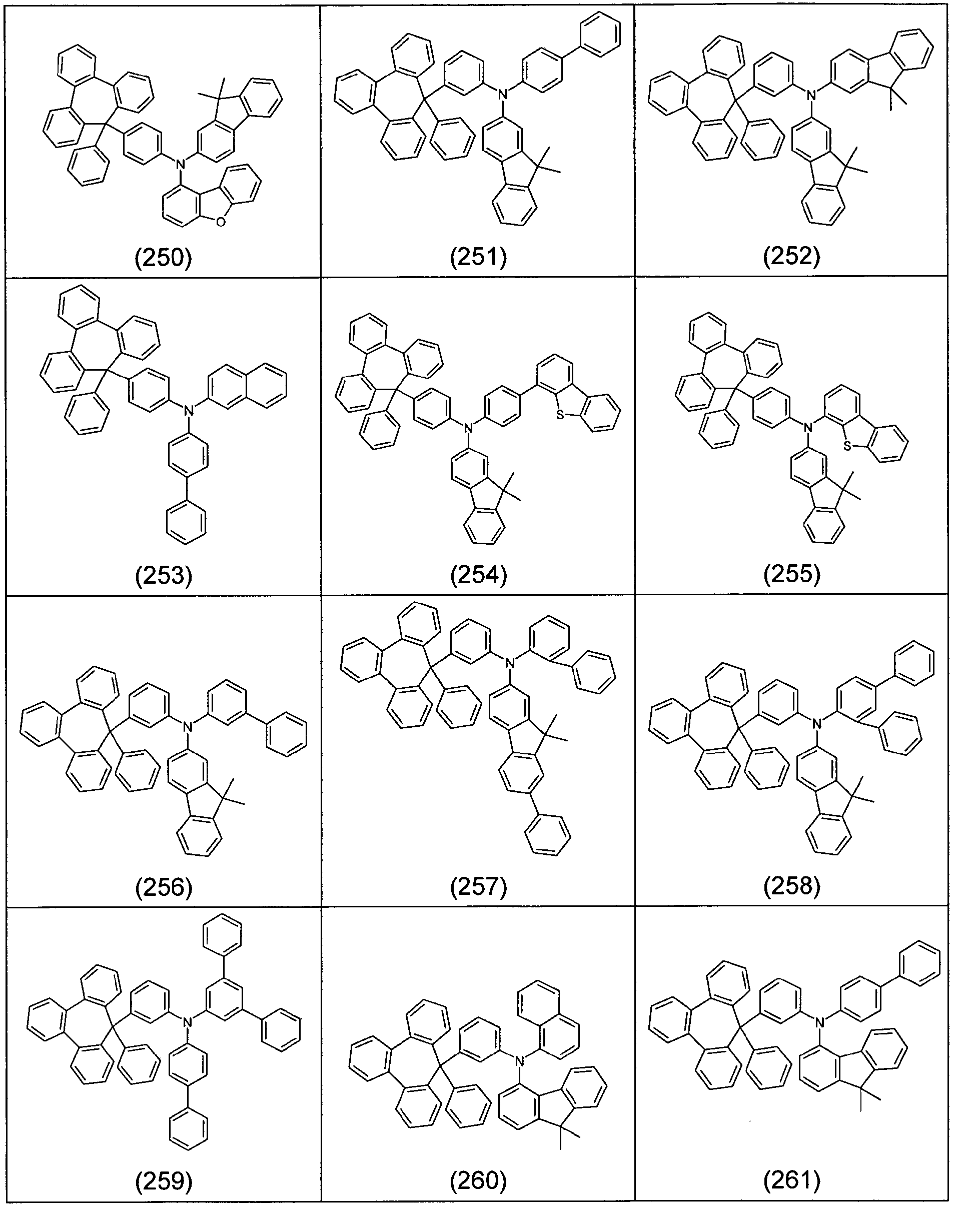

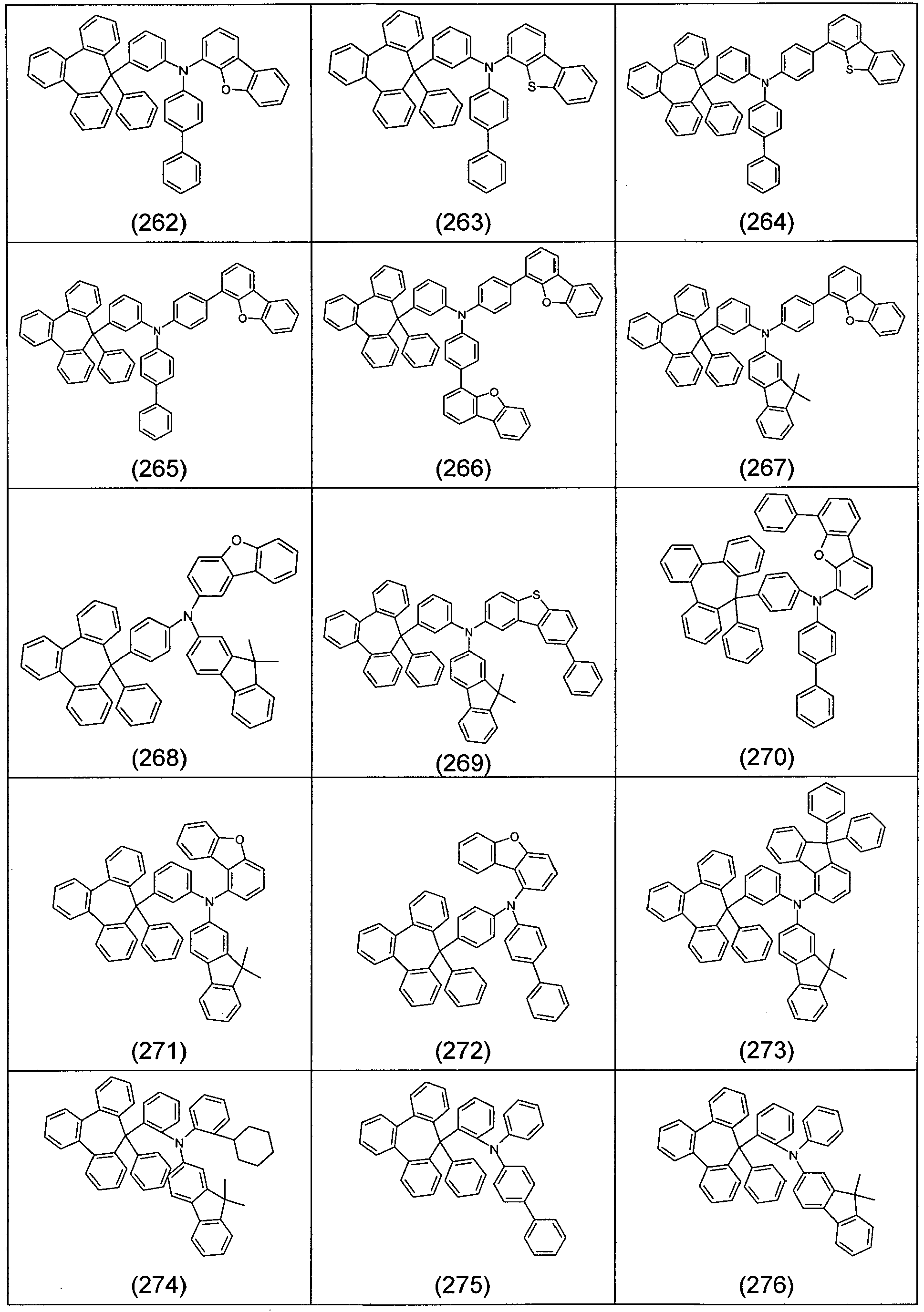

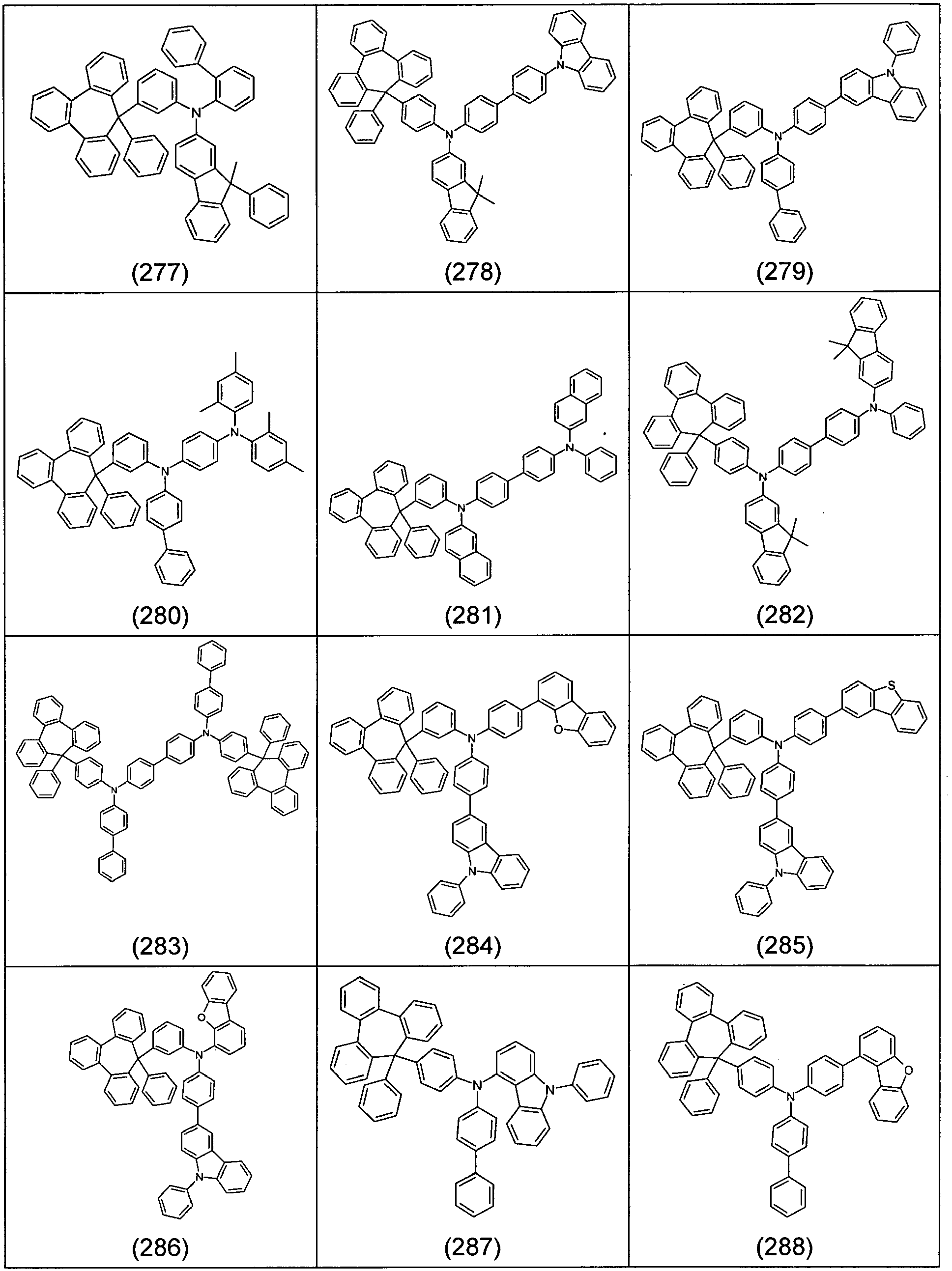

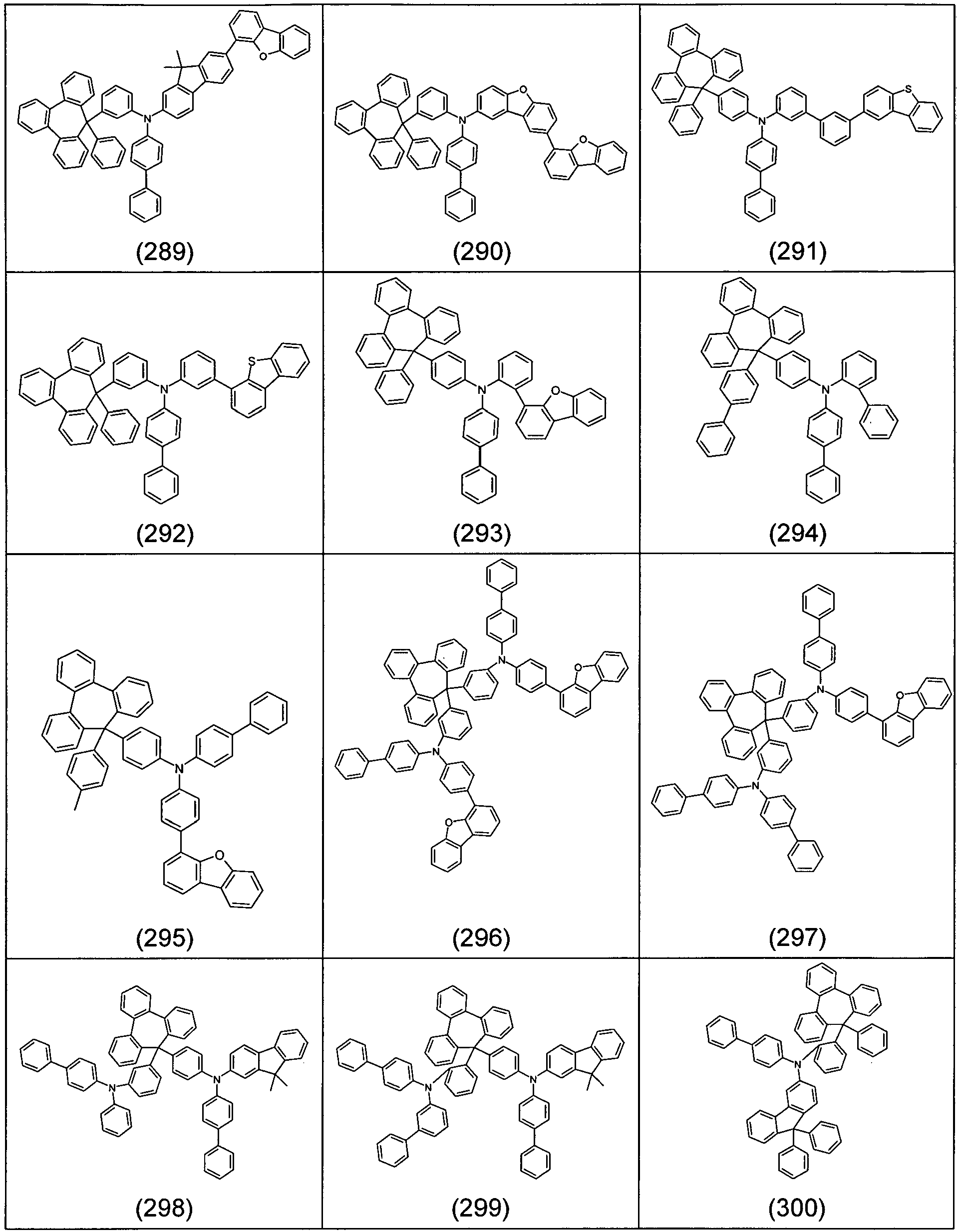

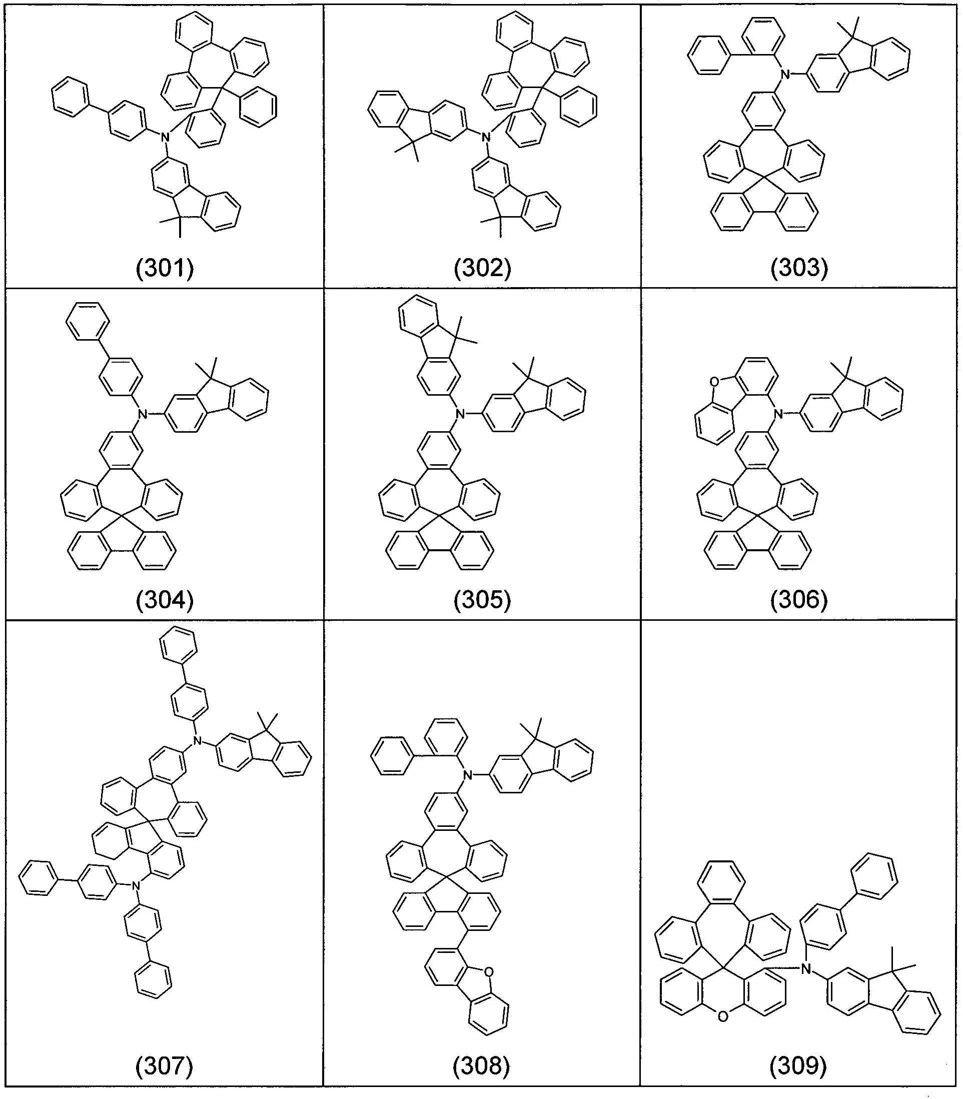

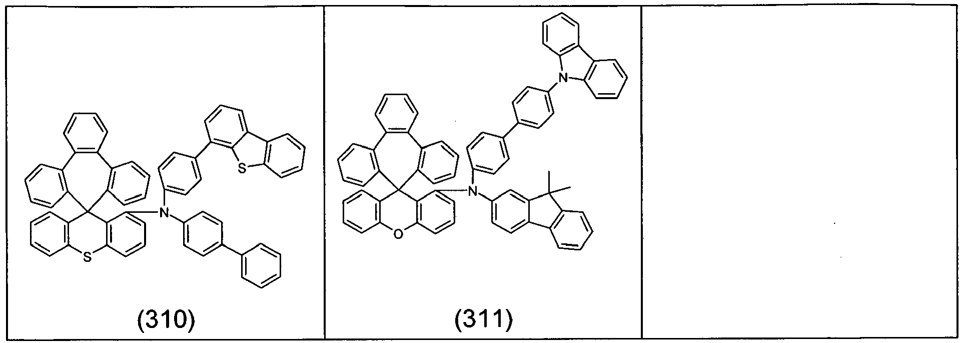

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden.

Die Synthese der erfindungsgemäßen Verbindungen geht beispielsweise von Tribenzotropon-Derivaten (9H-tribenzo[a,c,e]cyclohepten-9-on (CAS-Nr.: 68089-73-6)) aus. Durch die Reaktion mit der Ketogruppe mit entsprechend substituierten aromatischen oder heteroaromatischen Systemen kann das Grundgerüst aufgebaut werden. Bevorzugt ist dabei der nukleophile Angriff durch lithiierte Aromaten auf die Ketogruppe. Dies kann auch zum Aufbau des Spirozentrums bei m gleich 1 verwendet werden. Durch die Reaktion wird an Stelle der Ketogruppe das quartäre Kohlenstoffatom des Grundgerüsts aufgebaut.

Das Grundgerüst lässt sich durch weitere Kupplungsreaktionen, beispielsweise durch reaktive Abgangsgruppen, zu Verbindungen der Formel (1) funktionalisieren. Dafür eignen sich insbesondere übergangsmetallkatalysierte Kupplungsreaktionen (z.B. Suzuki-Kupplung, Hartwig-Buchwald-Kupplung oder Stille-Kupplung). Hierfür muss die zu kuppelnde Gruppe auch eine entsprechend geeignete Abgangsgruppe aufweisen, insbesondere Chlor, Brom, Iod, Triflat oder ein Boronsäurederivat, insbesondere Boronsäure bzw. einen Boronsäureester.

Ein weiterer Gegenstand der vorliegenden Erfindung ist daher ein Verfahren zur Herstellung einer Verbindung gemäß Formel (1) wobei die Verbindung der Formel (1) durch eine oder mehrere Kupplungsreaktionen und/oder Zyklisierungen aufgebaut wird.

Ein weiterer Gegenstand der vorliegenden Erfindung ist daher ein Verfahren zur Herstellung einer Verbindung gemäß Formel (1) durch Kupplung des Grundgerüsts, welches mit einer reaktiven Abgangsgruppe substituiert ist, mit
a) einem primären Amin, gefolgt von der Kupplung mit einer weiteren aromatischen Gruppe, die mit einer reaktiven Abgangsgruppe substituiert ist, oder
b) mit einem sekundären Amin, oder
c) mit einem Triarylaminderivat.

Dabei ist die reaktive Abgangsgruppe bevorzugt ausgewählt aus Cl, Br, I, Triflat oder Tosylat oder für eine Suzuki-Kupplung auch Boronsäure, bzw. ein Boronsäurederivat, insbesondere ein Boronsäureester.

Die oben gezeigten Syntheseverfahren haben exemplarischen Charakter und können vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. CC-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weitere Gegenstände der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen freien Positionen in Formel (1) lokalisiert sein können. Je nach Verknüpfung der erfindungsgemäßen Verbindung ist die Verbindung Bestandteil einer Seitenkette des Oligomers oder Polymers oder Bestandteil der Hauptkette.

Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist.

Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein.

In den linear verknüpften Strukturen können die Einheiten gemäß Formel (1) oder ihrer Ausführungsformen direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein.

In verzweigten und dendritischen Strukturen können beispielsweise 3, 5 oder mehrere Einheiten gemäß Formel (1) oder ihrer Ausführungsformen über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für die erfindungsgemäßen Verbindungen beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation
(B) YAMAMOTO-Polymerisation
(C) STILLE-Polymerisation und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können durch dem Fachmann bekannte Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1 995), 26(1-3), 127-36; Janssen, H. M.; Meijer, E. W., "The synthesis and characterization of dendritic molecules", Materials Science and Technology (1 999), 20 (Synthesis of Polymers), 403-458; Tomalia, Donald A., "Dendrimer molecules", Scientific American (1995), 272(5), 62-6; WO 2002/067343 A1 und WO 2005/026144 A1 .

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion enthaltend mindestens eine Verbindung gemäß Formel (1) oder ihrer Ausführungsformen oder mindestens ein Polymer, Oligomer oder Dendrimer enthaltend mindestens eine Einheit gemäß Formel (1) oder ihrer Ausführungsformen sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, sodass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder in einer elektronenblockierenden bzw. exzitonenblockierenden Schicht und/oder in einer Lochtransportschicht und/oder in einer Lochinjektionsschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Lochtransportmaterial und/oder Lochinjektionsmaterial eingesetzt.

Wird die Verbindung gemäß Formel (1), bzw. gemäß der bevorzugten Ausführungsformen, als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (1) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712,
WO 2009/003455, WO 2010/094378, WO 2011/120709,
US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃. Bevorzugt sind weiterhin Bismut-Komplexe mit elektronenarmen Carboxylat-Liganden, bevorzugt fluorierten Carboxylat-Liganden.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden inbesondere die folgenden Verbindungen:

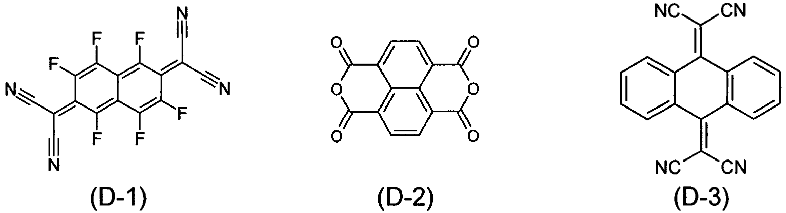

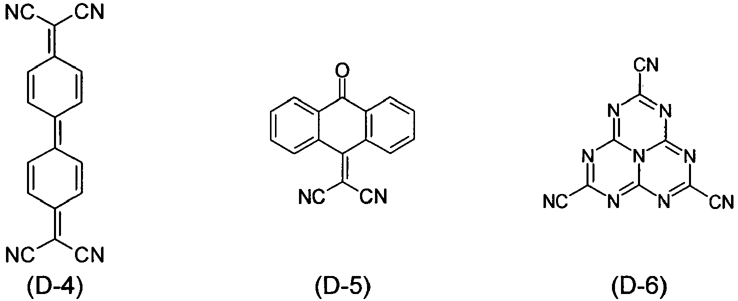

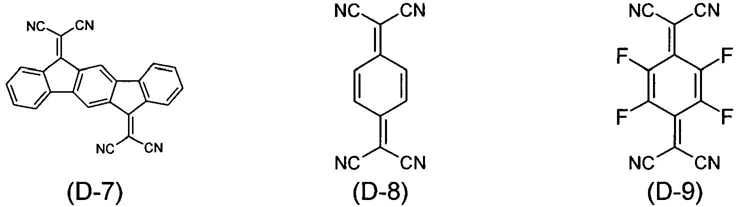

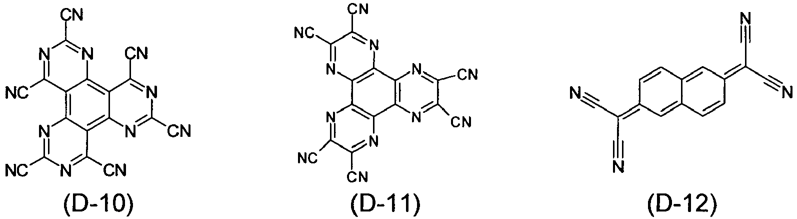

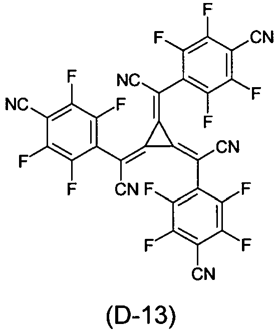

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710,
EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Vom Begriff phosphoreszierende Dotanden (Emitter) sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

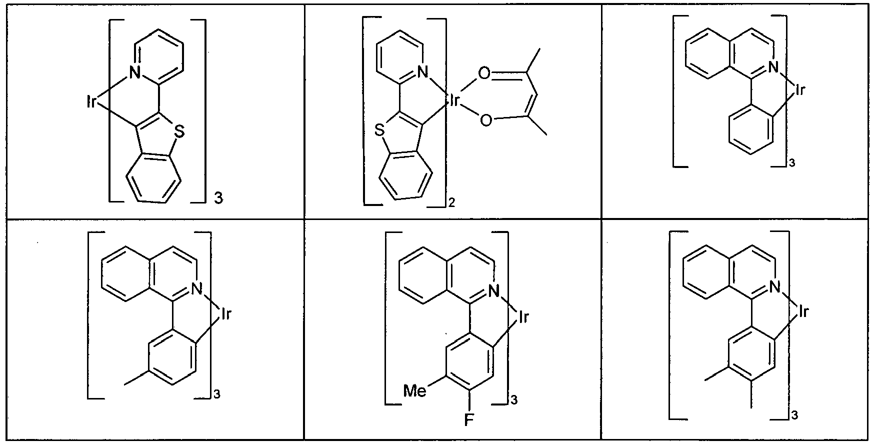

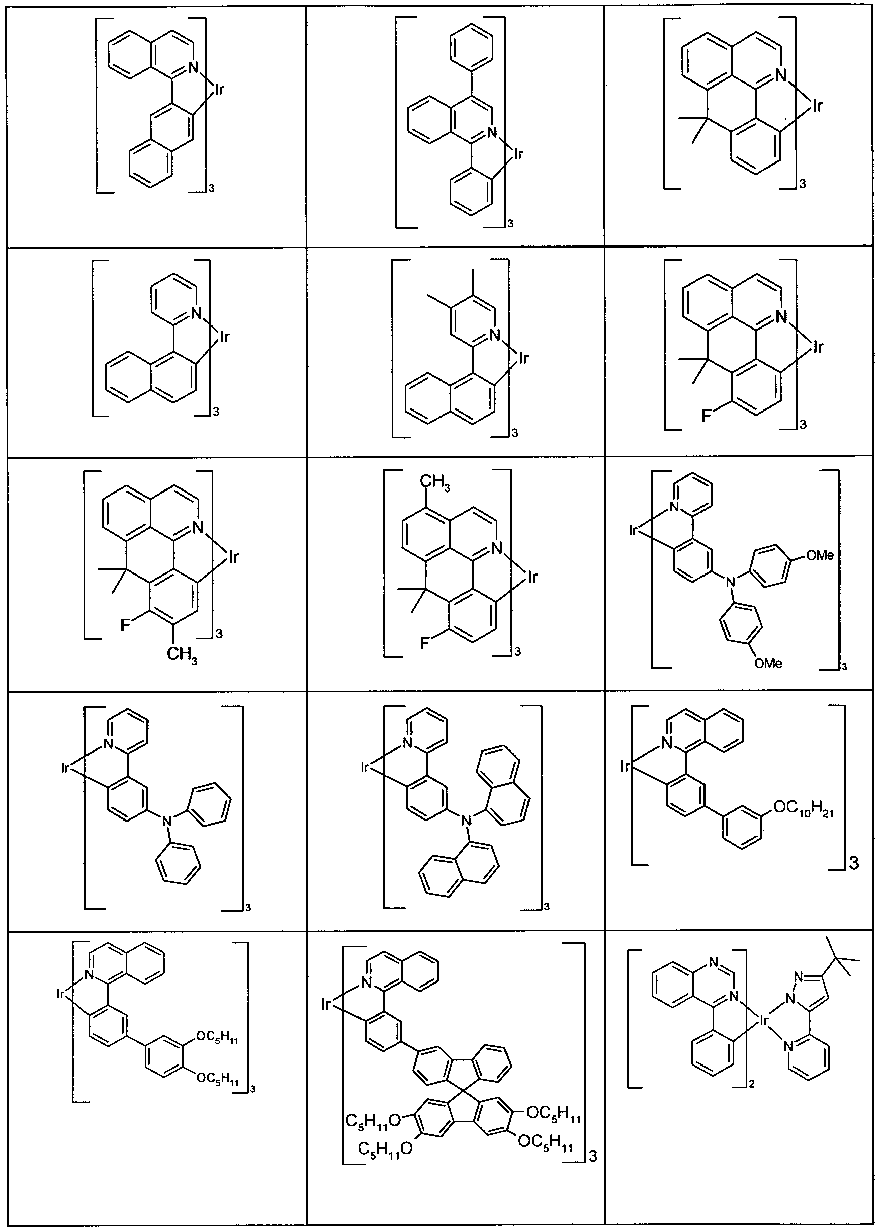

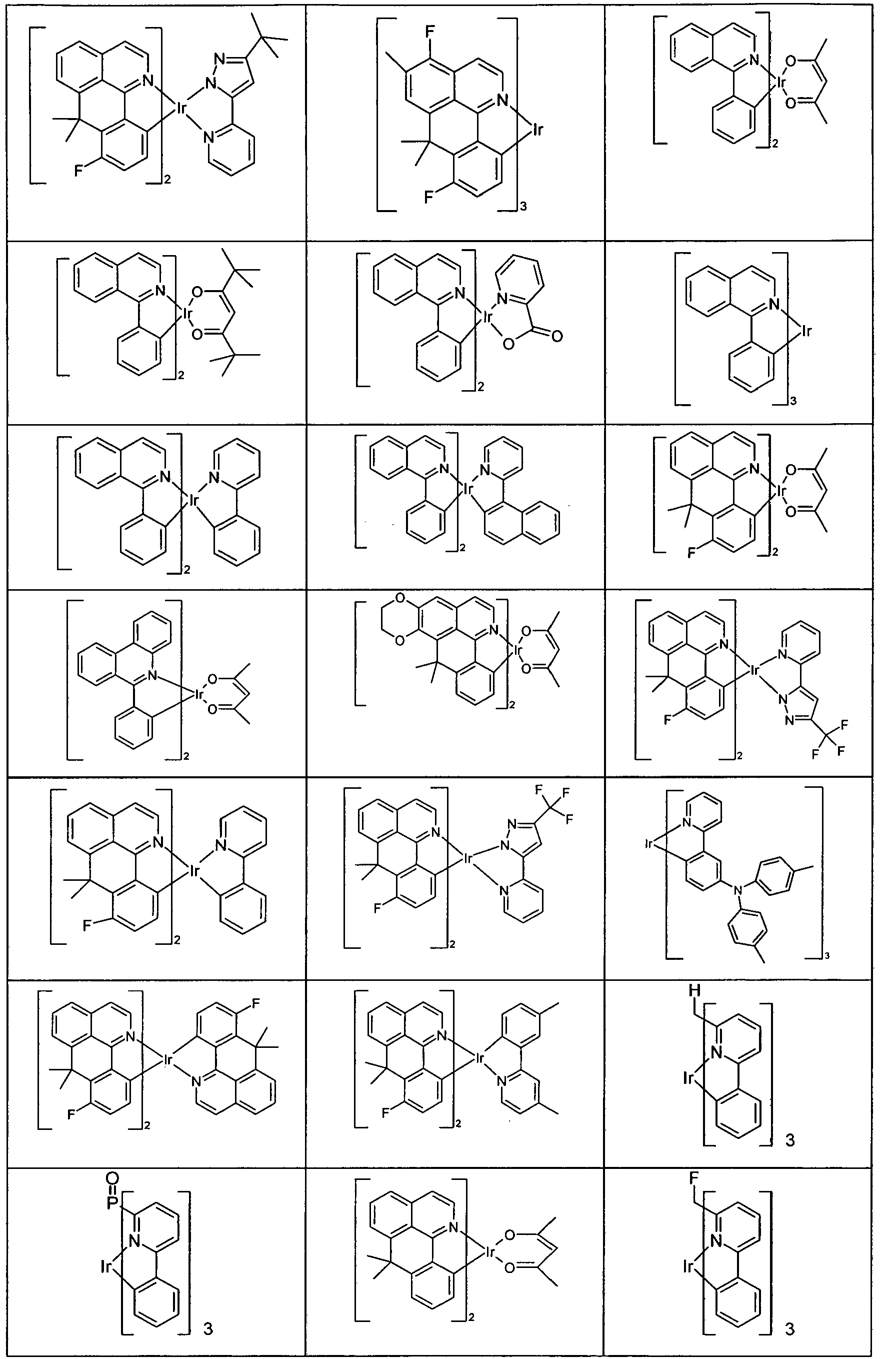

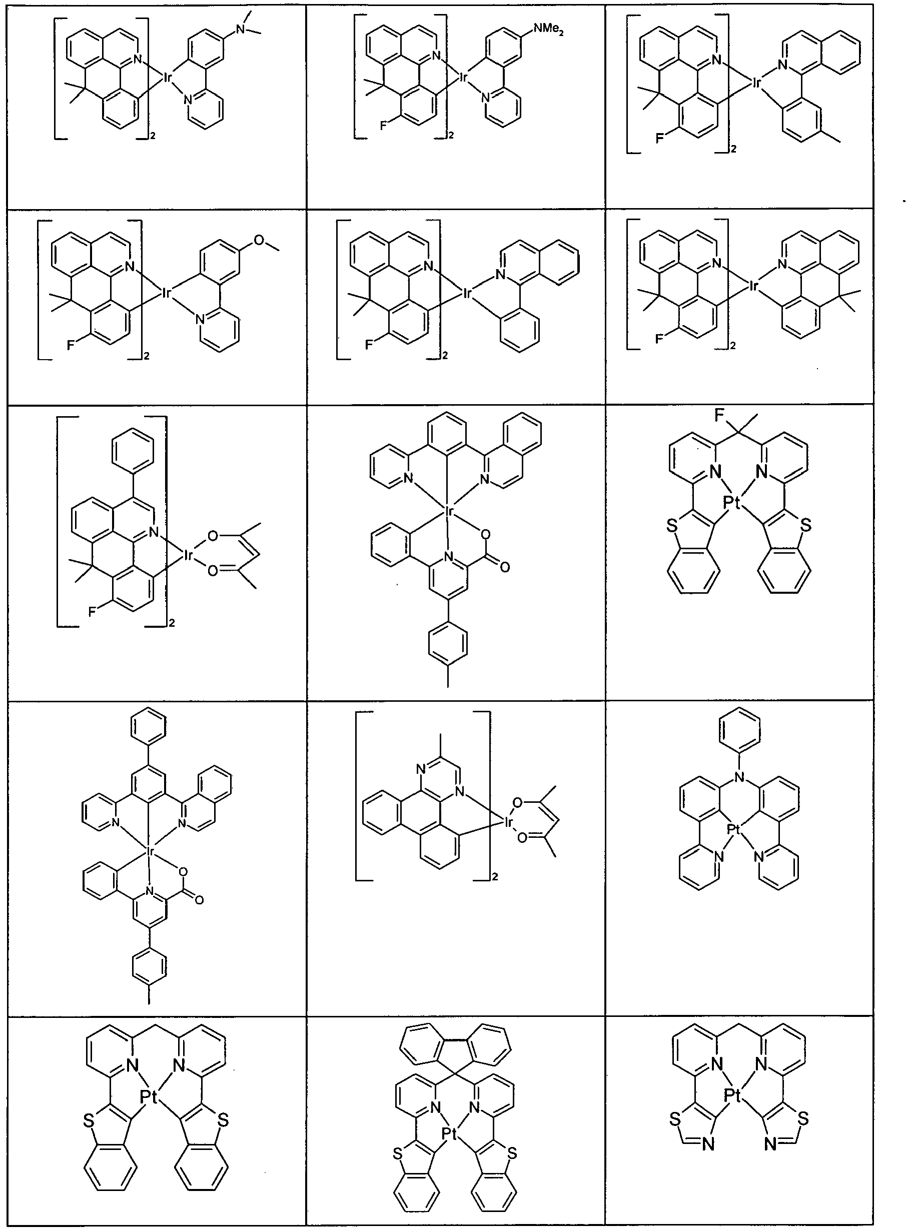

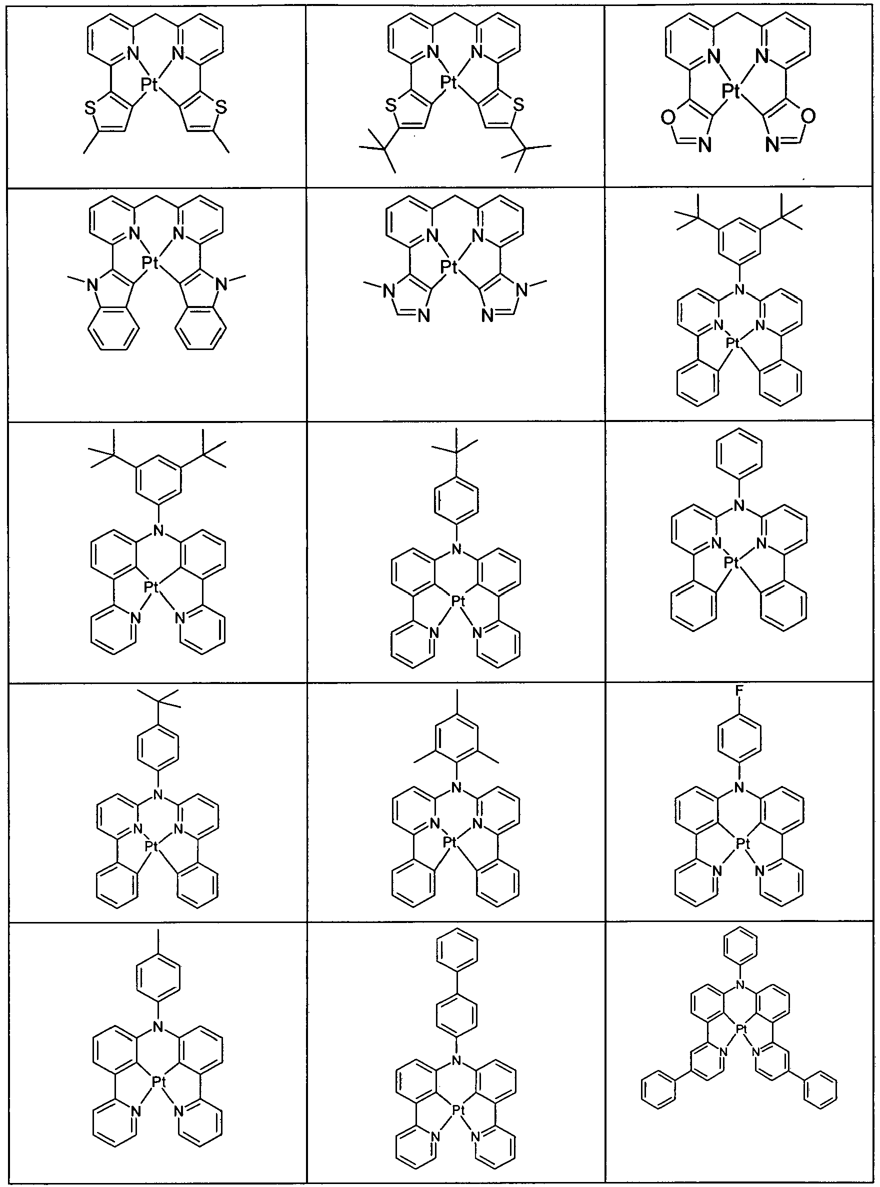

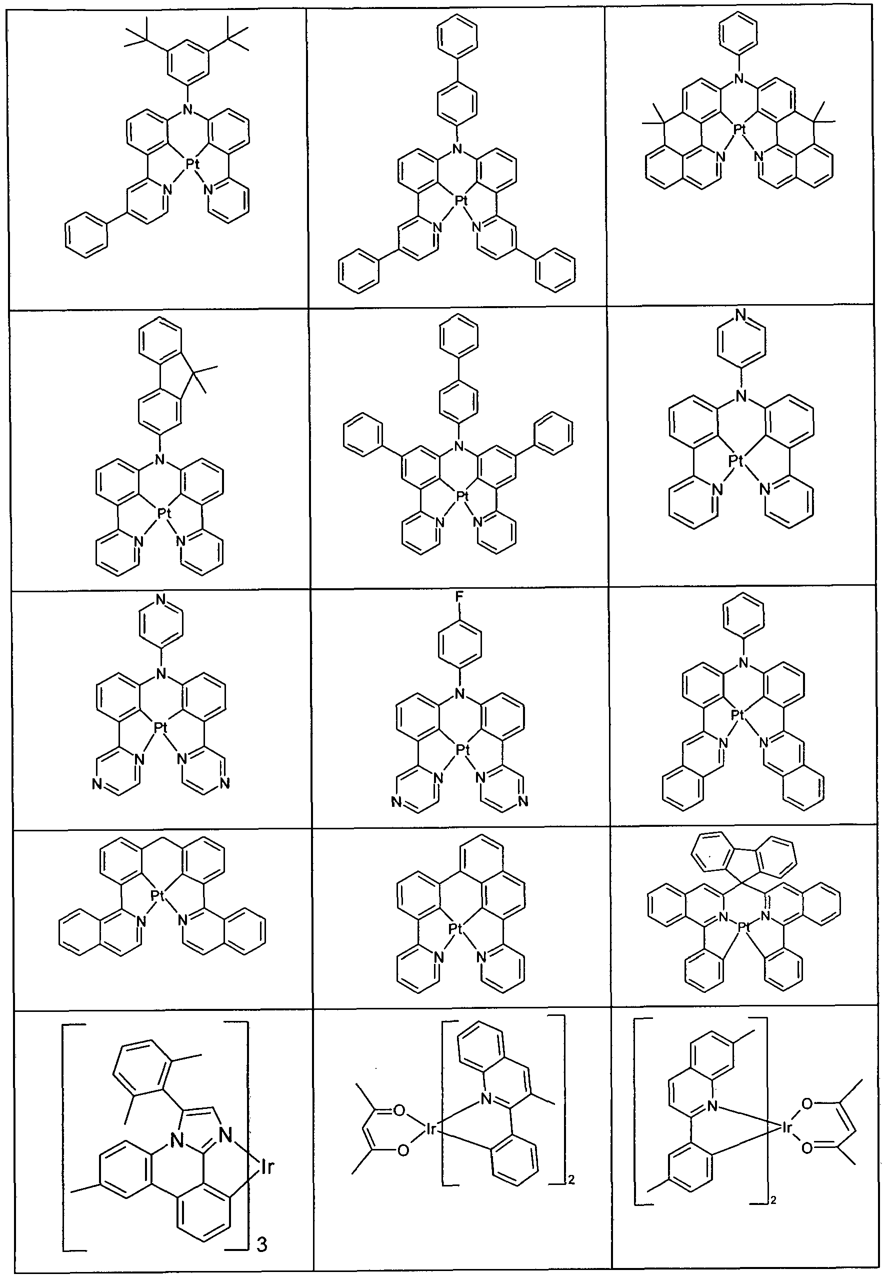

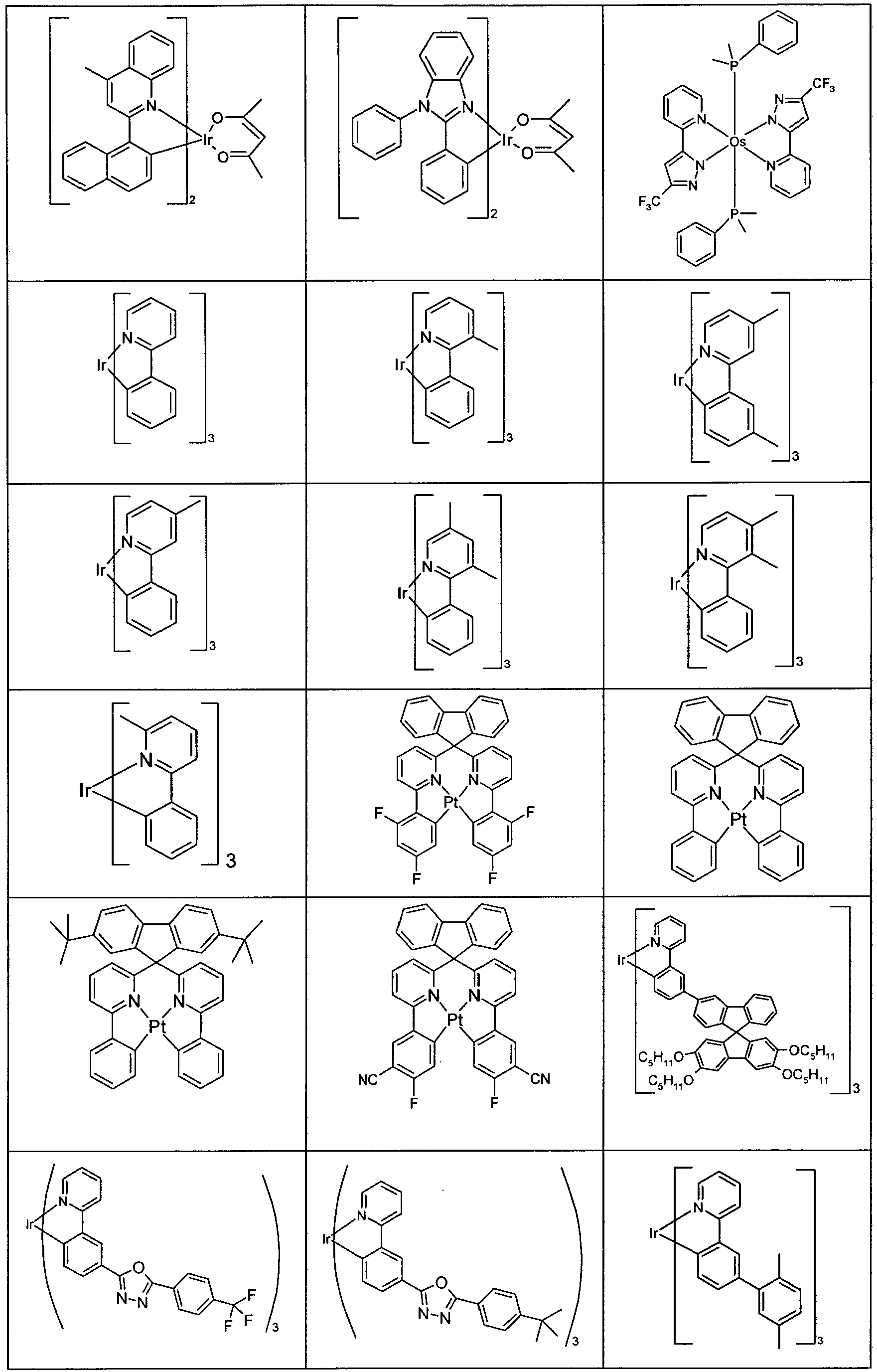

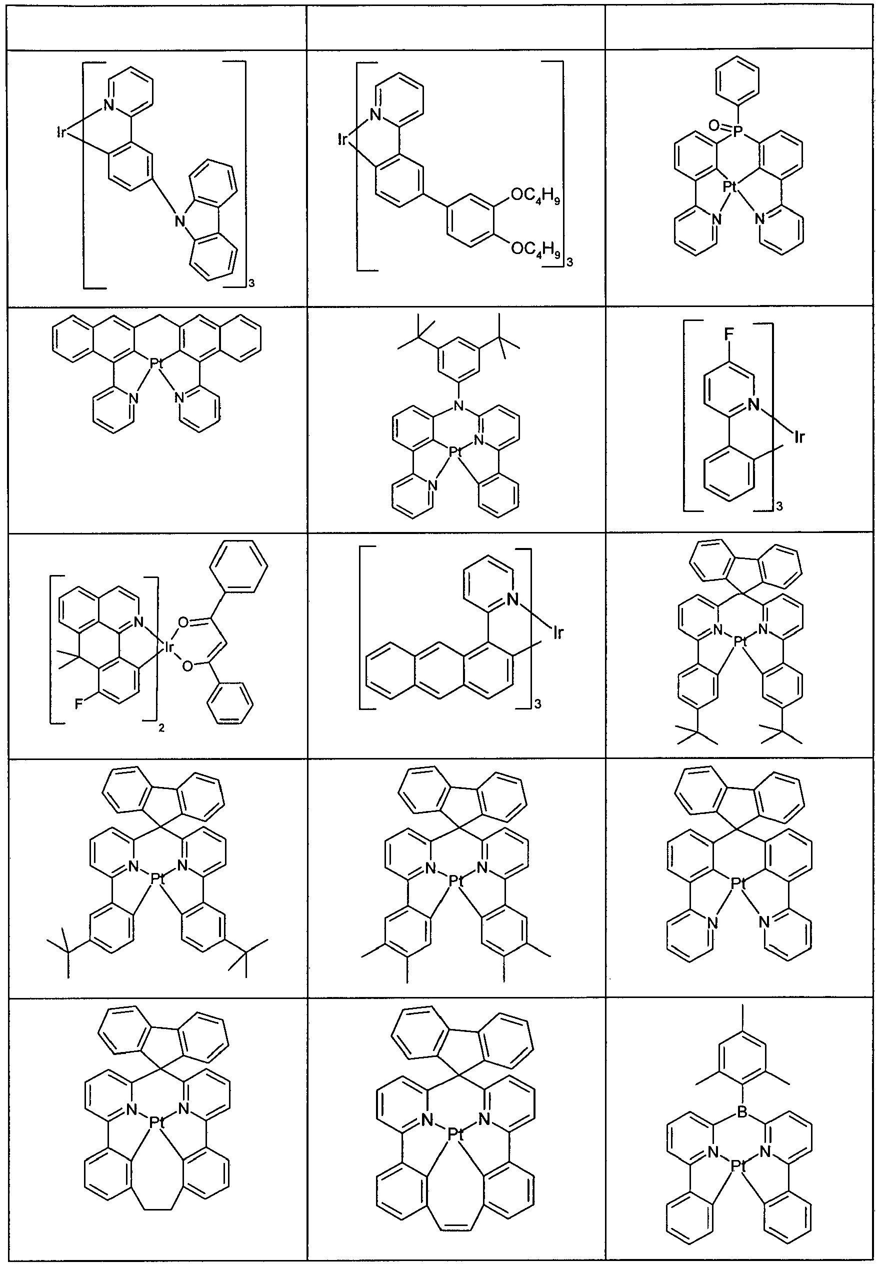

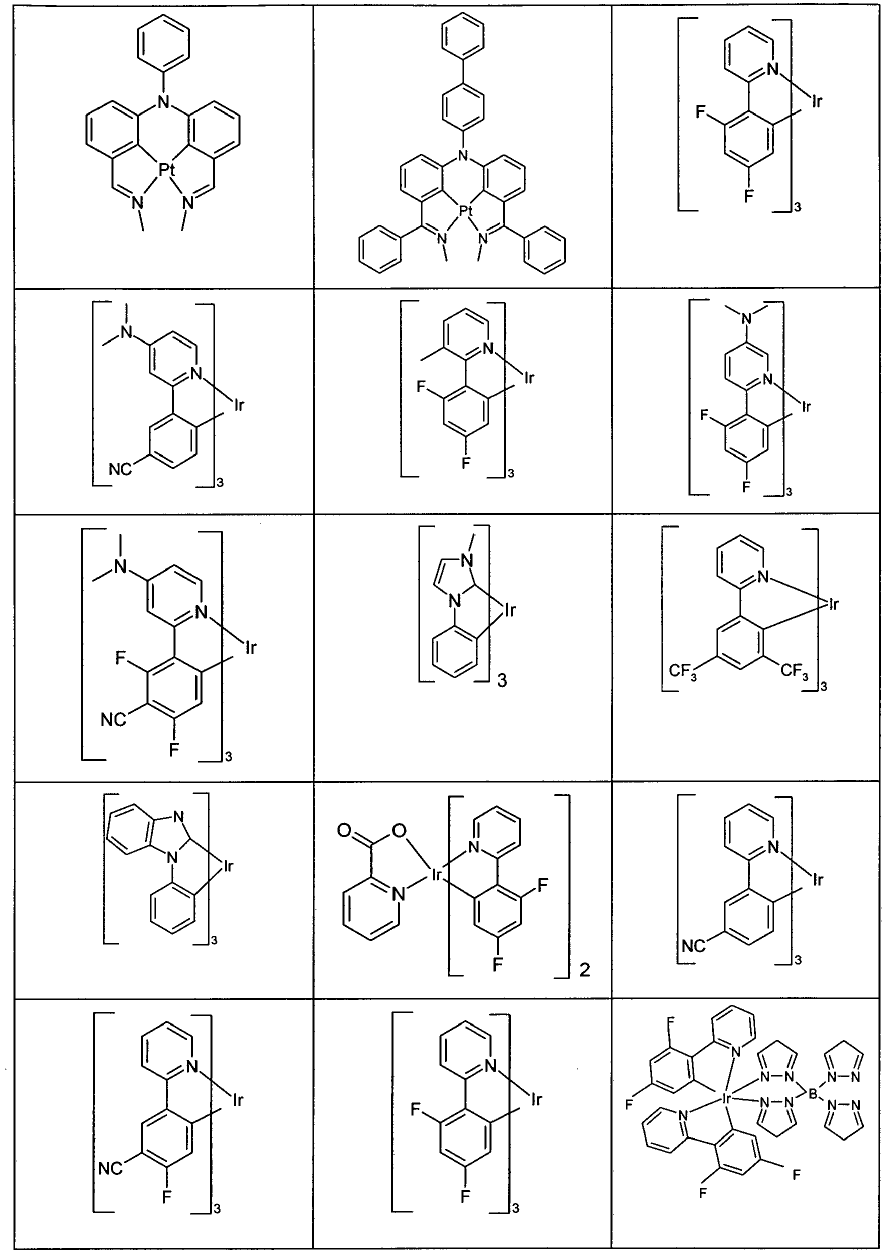

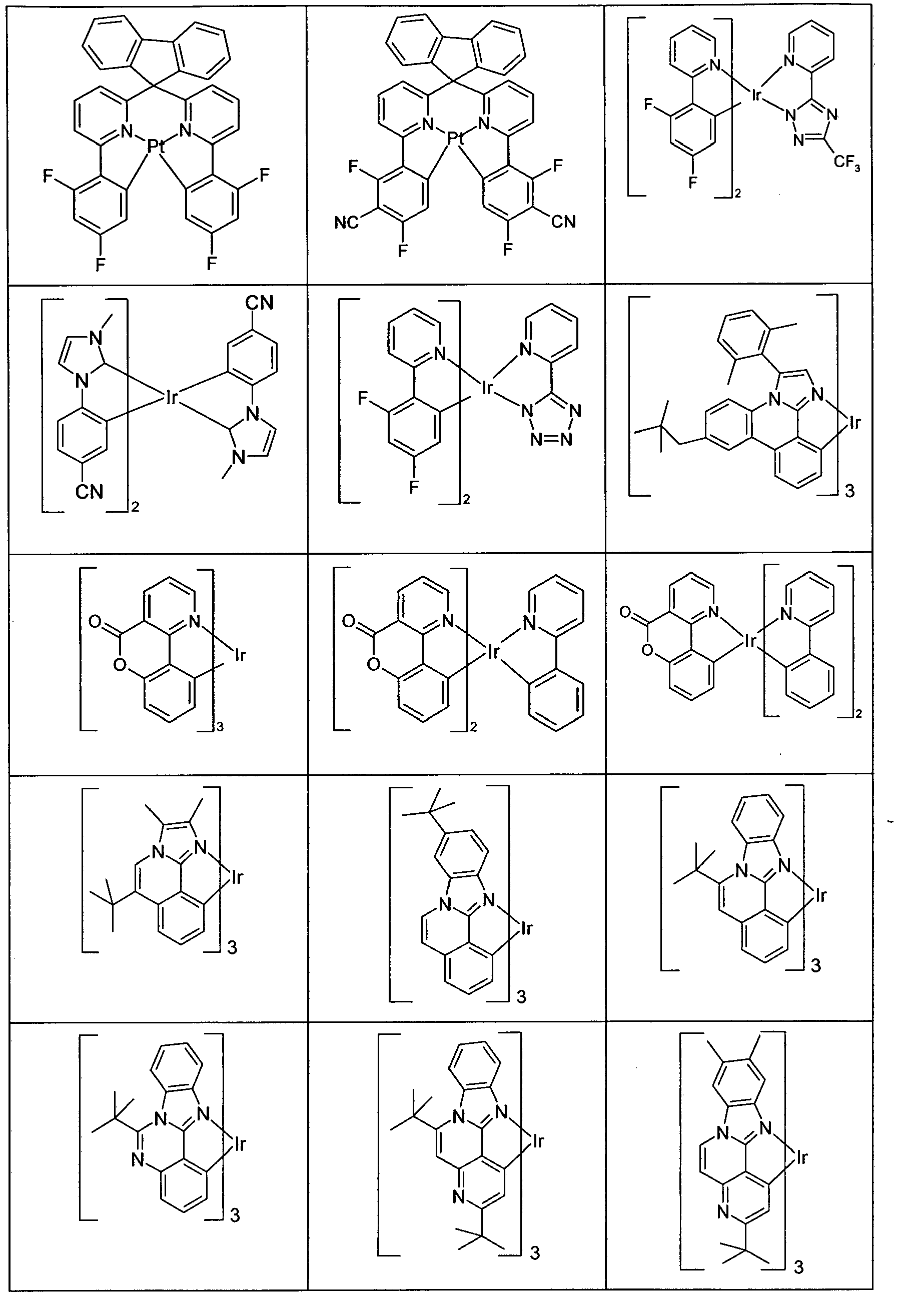

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransportschicht oder in einer Lochinjektionsschicht oder in einer Exzitonen- bzw. Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen einen oder mehrere der folgenden überraschenden Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die erfindungsgemäßen organische Elektroluminszenzvorrichtungen weisen eine verringerte Betriebsspannung auf.
4. Wenn die erfindungsgemäßen Verbindungen als Matrixmaterial für phosphoreszierende Emitter verwendet werden, ist es möglich, mit nur einer geringen Emitterkonzentration im Bereich von weniger als 10 Vol.% bereits sehr gute Ergebnisse zu erzielen.
5. Die erfindungsgemäßen Verbindungen weisen eine sehr gute thermische Stabilität auf.
6. Die erfindungsgemäßen Verbindungen weisen, eine niedrige Sublimationstemperatur auf.

Die Erfindung wird nun durch die nachfolgenden Beispiele näher erläutert, ohne sie dadurch einschränken zu wollen.

### Ausführungsbeispiele

Der Aufbau des Tribenzocyclohepten-Fluoren-Spiro Grundgerüsts erfolgt bevorzugt analog zur klassischen Spirosynthese. Als Ausgangmaterialien dienen das 9H-tribenzo[a,c,e]cyclohepten-9-on (CAS-Nr.: 68089-73-6) und 2-Brom-biphenyl Derivate. Dabei können durch die Verwendung von entsprechend substituierten Biphenylenen unterschiedliche Substitutionsmuster am Grundgerüst erhalten werden (Schema 1). Die Synthese von 9H-tribenzo[a,c,e]cyclohepten-9-on ist in Chem. Sci., 2011, 2, 2029 beschrieben. 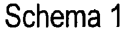 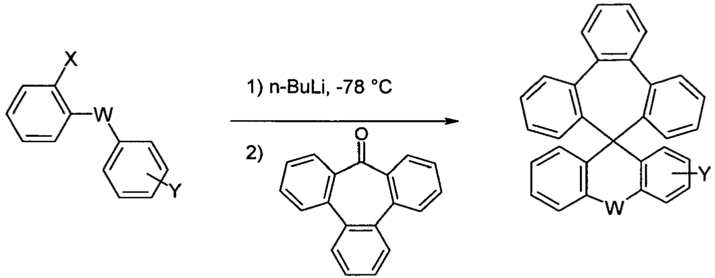 Dabei steht W für die Brücke zwischen den Phenylgruppen und X für ein Halogenid wie Chlor, Brom oder Iod und Y für eine reaktive Abgangsgruppe oder ein Substituent. Über Y können dann weitere Gruppen eingeführt werden.

Die Synthese der erfindungsgemäßen Verbindungen kann gemäß im Stand der Technik bekannten Verfahren und Reaktionstypen durchgeführt werden. Insbesondere lassen sich die Verbindungen aus einem entsprechend Halogen-substituierten Grundgerüst durch Einführung der Aminogruppe synthetisieren, wie in Schema 2 dargestellt. Dabei ist es entweder möglich, zunächst ein primäres Amin mit einem Substituenten Ar¹ einzuführen und die weitere Gruppe Ar¹ in einer weiteren Kupplungsreaktion einzuführen, wie in Schema 2 a) gezeigt. Ebenso ist es möglich, direkt in einem Schritt das sekundäre Amin Ar¹Ar¹NH einzuführen, wie in Schema 2 b) gezeigt. Als Gruppe Y am Grundgerüst eignen sich reaktive Abgangsgruppen, wie beispielsweise Cl, Br, I, Triflat oder Tosylat. Als Kupplungsreaktionen eignen sich beispielsweise Kupplungsreaktionen nach Hartwig-Buchwald oder nach Ullmann. Die Reaktionsbedingungen, die für diese Kupplungsrektionen verwendet werden können, sind dem Fachmann der organischen Synthese bekannt. 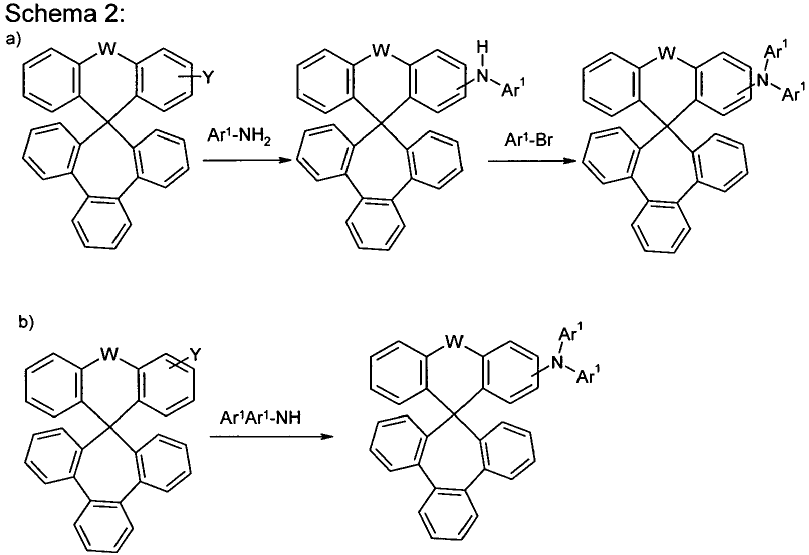 Für Verbindungen mit einem Linker lässt sich die Gruppe Ar²-NAr¹Ar¹ ebenfalls über eine metallkatalysierte Kupplungsreaktion einführen, beispielsweise über eine Suzuki-Kupplung oder eine Stille-Kupplung (Schema 3) 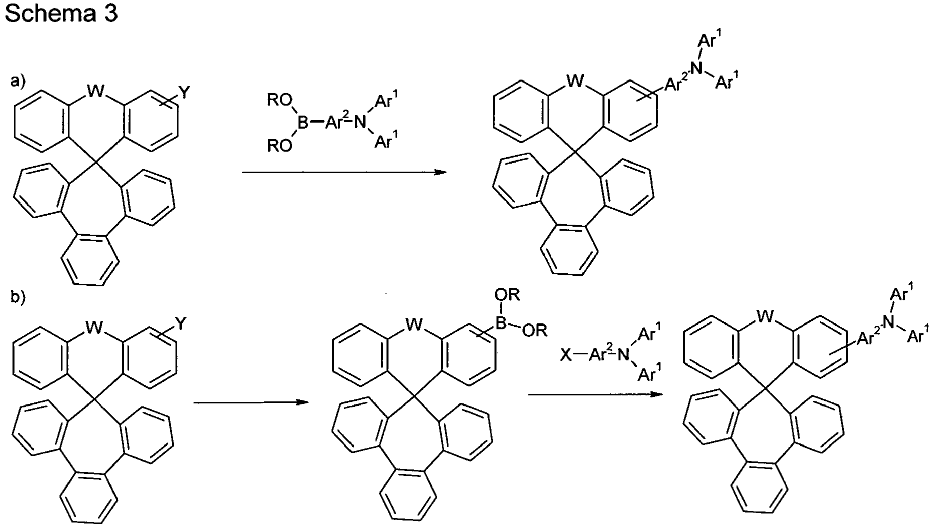 Dabei stehen Y und X für reaktive Abgangsgruppen. Schema 3 a) und Schema 3 b) zeigen zwei unterschiedliche Wege zur Kupplung eines Triarylamins an das Grundgerüst.

### Synthese der Verbindung (1-1)

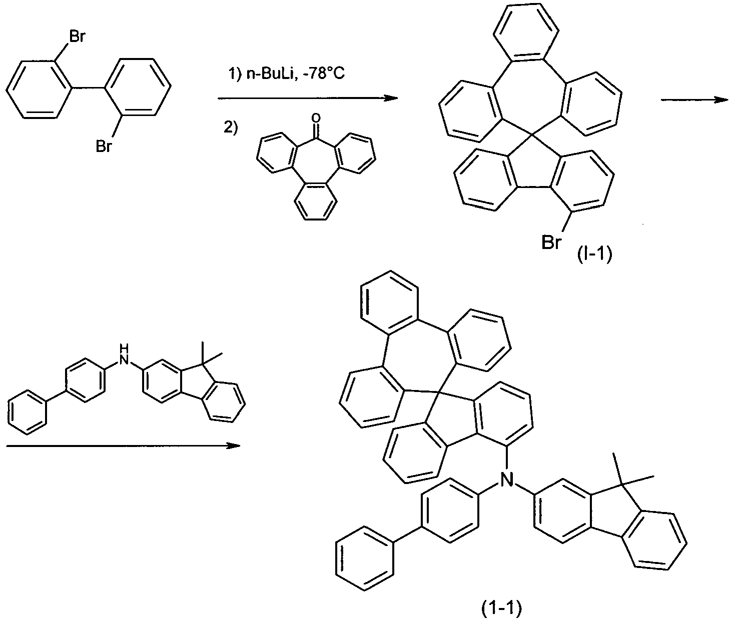

### Synthese der Verbindung (I-1)

26,7 g (85,8 mmol) 2,2'-Dibromo-biphenyl werden in einem ausgeheizten Kolben in 300 mL getrocknetem THF gelöst. Die Reaktionsmischung wird auf -78°C gekühlt. Bei dieser Temperatur werden 34,3 mL einer 2,5M Lösung n-BuLi in Hexan (85,8 mmol) langsam zugetropft. Der Ansatz wird 1 h bei -70°C nachgerührt. Anschließend werden 20,0 g Tribenzocyclohepten-9-one (CAS-Nr.: 68089-73-6) (78 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 500 ml Essigsäure versetzt und anschließend werden 90 ml rauchende HCl zugegeben. Der Ansatz wird auf 75°C erhitzt und 5 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Die Ausbeute beträgt 29 g (62 mmol), 79% der Theorie.

Analog hierzu werden die folgenden Verbindungen (I-2) bis (I-12) hergestellt.

| **Bsp** | **Edukt 1** | **Edukt 2** | **Produkt** | **Ausbeute** |
|---|---|---|---|---|
| I-2 | 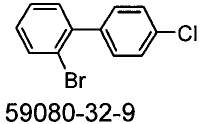 | 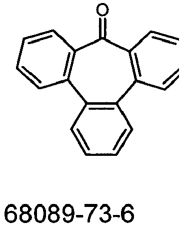 | 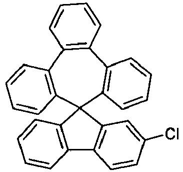 | 78% |
| I-3 | 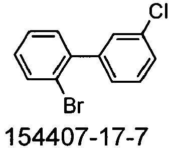 | 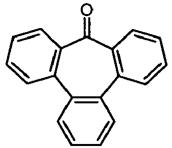 | 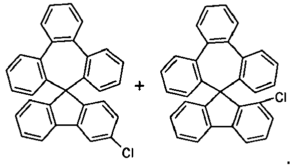 | 63% |
| I-4 | 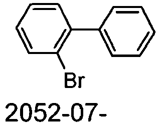 | 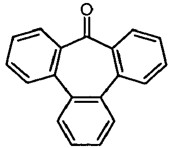 | 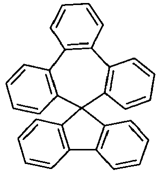 | 87% |
| I-5 | 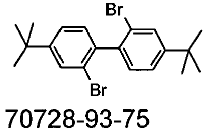 | 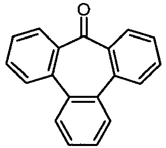 | 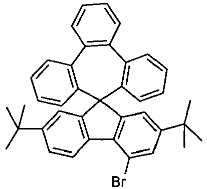 | 75% |
| I-6 | 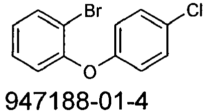 | 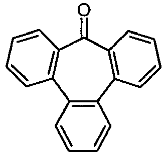 | 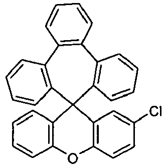 | 60% |
| I-7 | 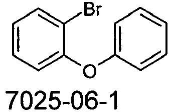 | 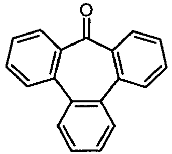 | 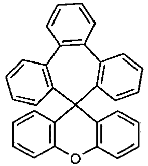 | 63% |
| I-8 | 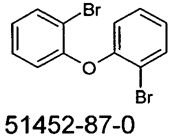 | 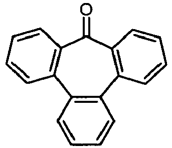 | 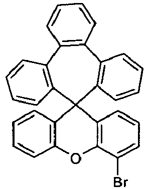 | 45% |
| I-9 | 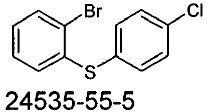 | 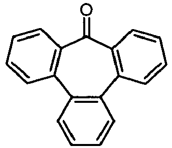 | 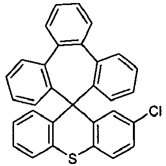 | 64% |
| I-10 | 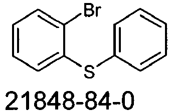 | 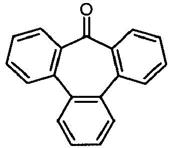 | 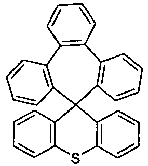 | 67% |
| I-11 | 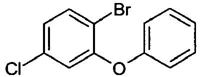 | 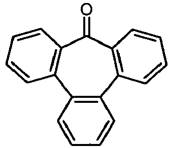 | 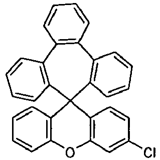 | 71% |
| I-12 | 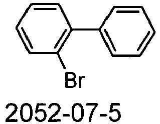 | 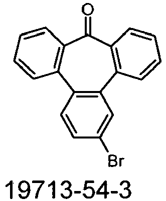 | 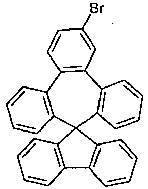 | 77% |

### Synthese der Verbindung (1-1)

7,9 g Biphenyl-4-yl-(9,9-dimethyl-9H-fluoren-2-yl)-amin (22 mmol) und 10,6 g des Bromderivates (I-1) (22 mmol) werden in 200 mL THF gelöst: Die Lösung wird entgast und mit N₂ gesättigt. Danach wird sie mit 1,1 mL (1,1 mmol) einer 1 M Tri-tert-Butylphosphin-Lösung und 0,12 g (55 mmol) Palladium(II)acetat versetzt. Anschließend werden 5,3 g Natrium-tert-butylat (55 mmol) zugegeben. Die Reaktionsmischung wird 3h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99,9%. Die Ausbeute beträgt 13,2 g (80% der Theorie).

Analog hierzu werden die folgenden Verbindungen (1-2) bis (1-24) hergestellt.

| **Bsp** | **Edukt 1** | **Edukt 2** | **Produkt** | **Ausbeute** |
|---|---|---|---|---|
| 1-2 | 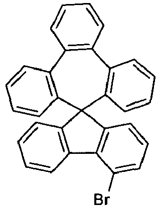 | 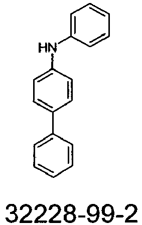 | 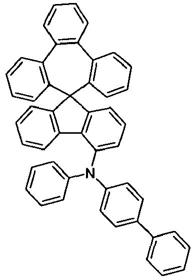 | 79 % |
| 1-3 | 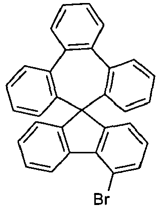 | 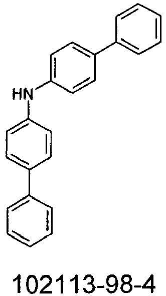 | 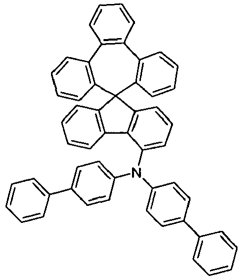 | 84% |
| 1-4 | 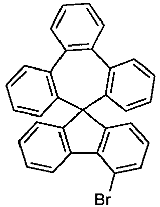 | 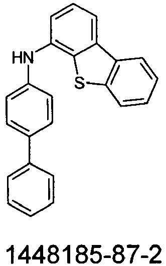 | 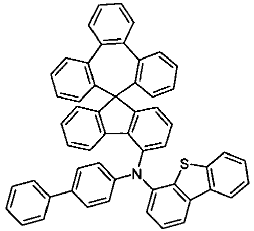 | 92% |
| 1-5 | 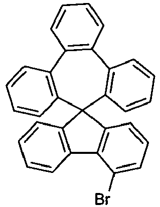 | 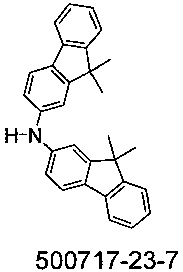 | 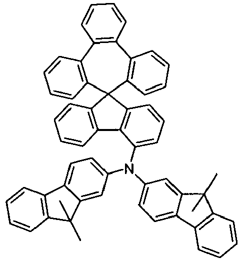 | 77% |
| 1-6 | 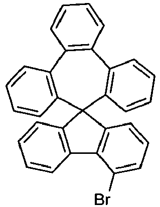 | 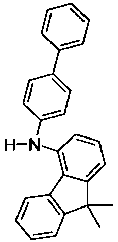 | 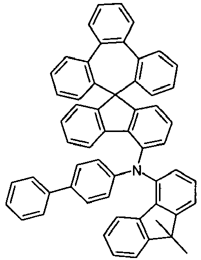 | 81% |
| 1-7 | 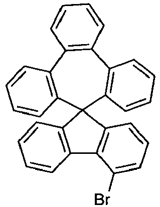 | 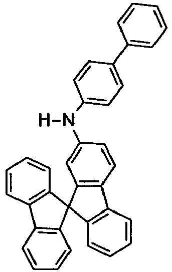 | 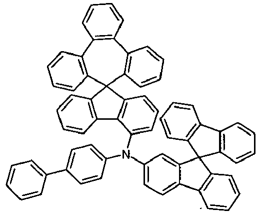 | 85% |
| 1-8 | 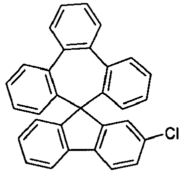 | 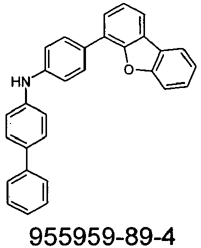 | 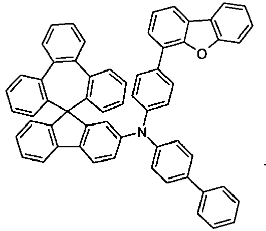 | 81% |
| 1-9 | 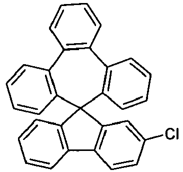 | 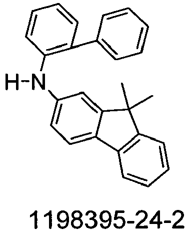 | 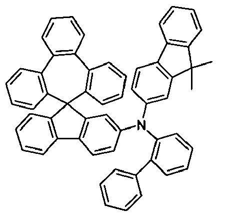 | 77% |
| 1-10 | 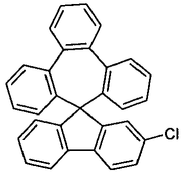 | 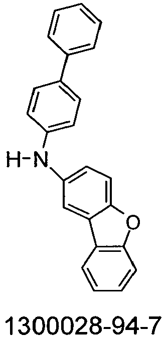 | 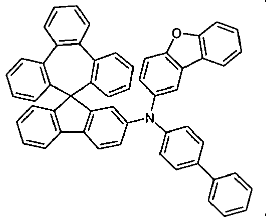 | 76% |
| 1-11 | 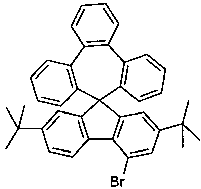 | 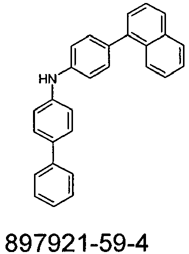 | 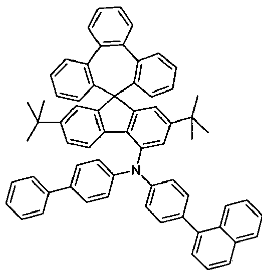 | 69% |
| 1-12 | 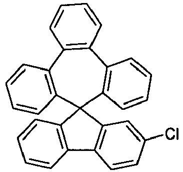 | 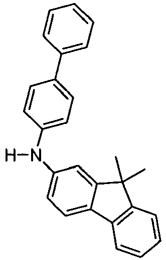 | 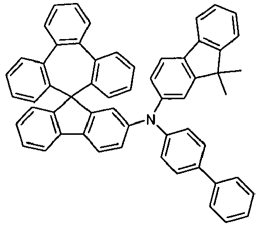 | 74% |
| 1-13 | 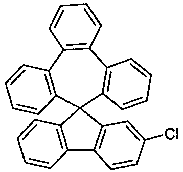 | 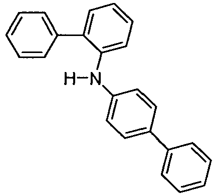 | 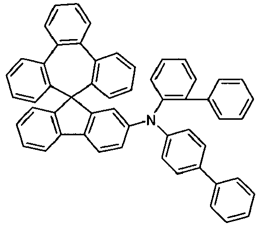 | 80% |
| 1-14 | 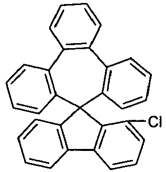 | 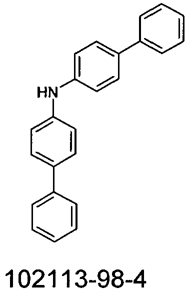 | 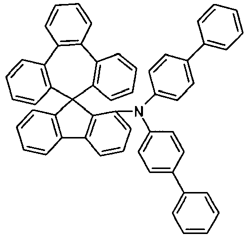 | 61% |
| 1-15 | 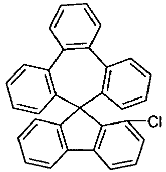 | 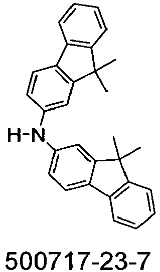 | 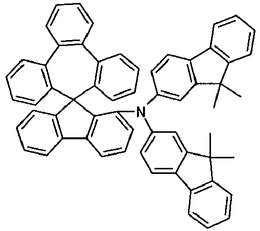 | 60% |
| 1-16 | 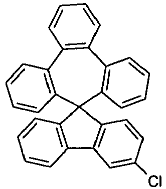 | 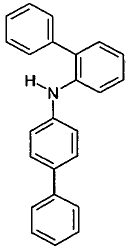 | 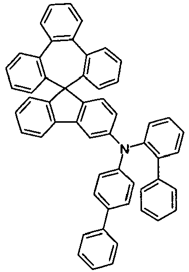 | 71% |
| 1-17 | 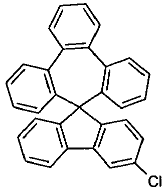 | 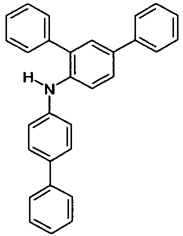 | 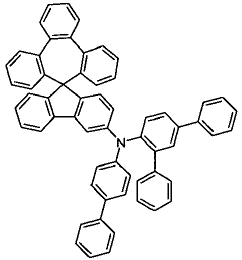 | 69% |
| 1-18 | 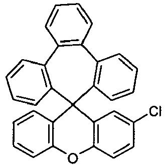 | 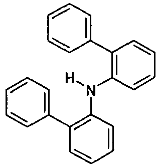 | 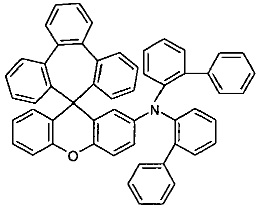 | 40% |
| 1-19 | 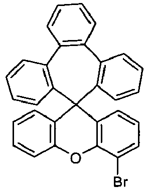 | 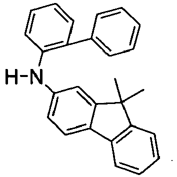 | 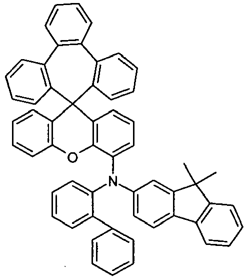 | 70% |
| 1-20 | 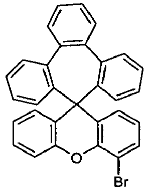 | 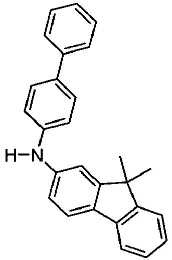 | 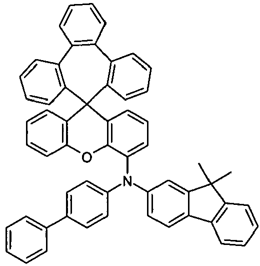 | 78% |
| 1-21 | 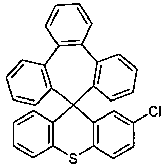 | 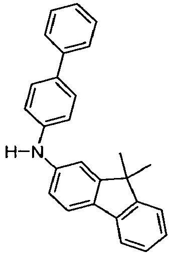 | 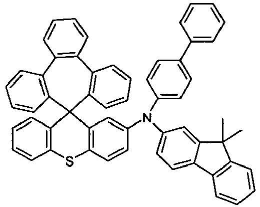 | 81% |
| 1-22 | 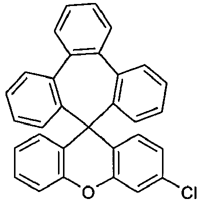 | 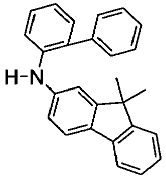 | 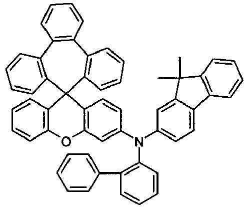 | 77% |
| 1-23 | 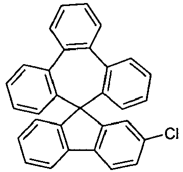 | 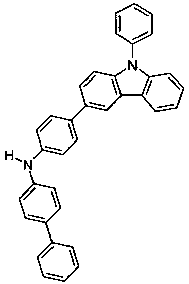 | 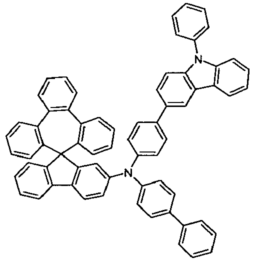 | 67% |
| 1-24 | 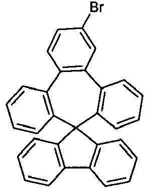 | 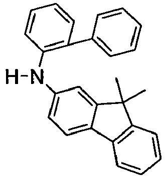 | 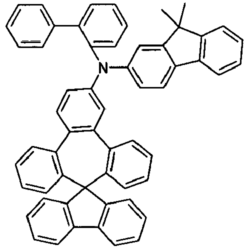 | 81% |

### Beispiel 2

### Synthese der Verbindung (2-1)

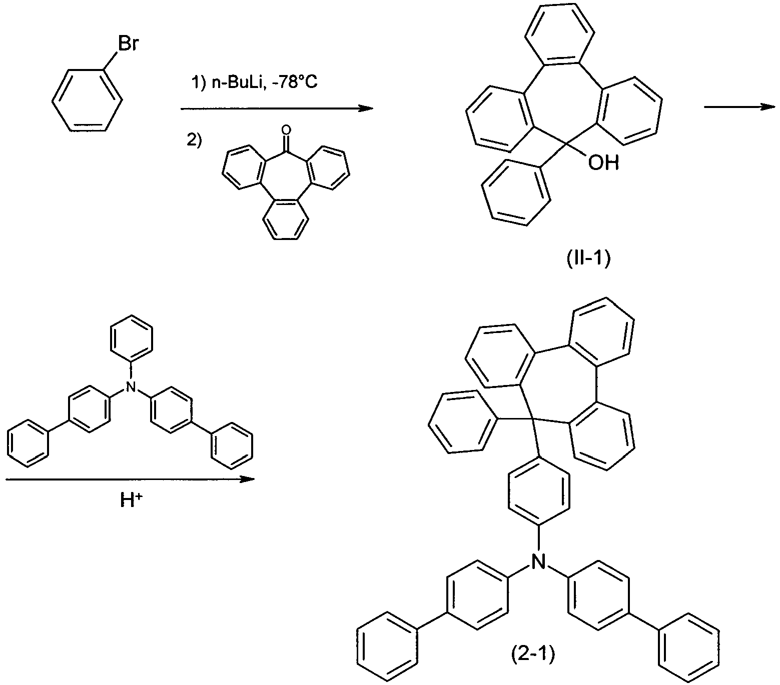

### Synthese der Verbindung (II-1)

40 g (255 mmol) Bromphenol werden in einem ausgeheizten Kolben in 600 mL getrocknetem THF gelöst. Die Reaktionsmischung wird auf -78°C gekühlt. Bei dieser Temperatur werden 102 mL einer 2,5M Lösung n-BuLi in Hexan (255 mmol) langsam zugetropft. Der Ansatz wird 0,5 h bei -70°C nachgerührt. Anschließend werden 65,3 g Tribenzo-cyclohepten-9-one (CAS-Nr.: 68089-73-6) (255 mmol) in 200 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Das Rohprodukt wird 2 h bei 80 °C mit 500 mL Heptan nachgerührt. Nach Erkalten saugt man vom ausgefallenen Feststoff ab, wäscht diesen einmal mit 100 ml Heptan und zweimal mit je 100 ml Ethanol. Ausbeute: 66,5 g, 78%.

Analog werden folgende Verbindungen erhalten:

| **Bsp** | **Edukt 1** | **Edukt 2** | **Produkt** | **Ausbeute** |
|---|---|---|---|---|
| II-2 | 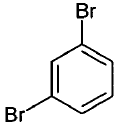 | 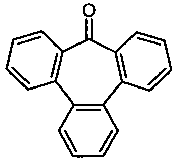 | 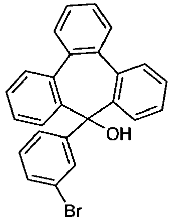 | 85 % |
| II-3 | 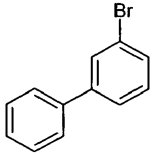 | 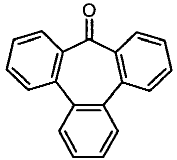 | 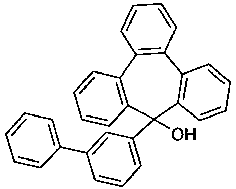 | 70% |
| II-4 | 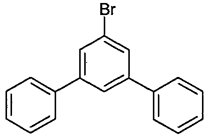 | 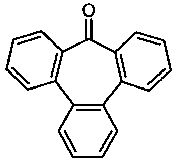 | 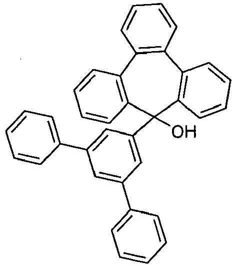 | 67% |
| II-5 | 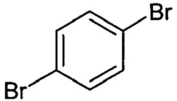 | 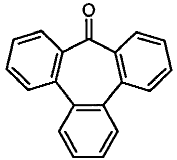 | 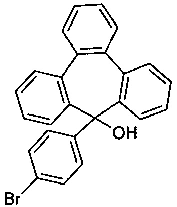 | 83% |
| II-6 | 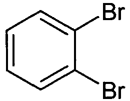 | 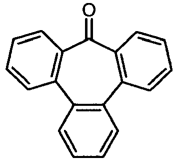 | 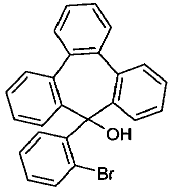 | 81 % |

### Synthese der Verbindung (2-1)

Ein Gemisch aus 20 g (60 mmol) Verbindung II-1 und 26,17 g (60 mmol) Bis-biphenyl-4-yl-phenyl-amin [122215-84-3], Trifluormethansulfonsäure [1493-13-6] 18 g (120 mmol, 10,5 mL) und 400 ml Dioxan wird 24 h unter Rückfluss erhitzt. Nach Erkalten versetzt man mit 200 ml Wasser, rührt 30 min. nach, trennt die org. Phase ab, filtriert diese über ein kurzes Celite-Bett und entfernt dann das Lösungsmittel im Vakuum. Der Rückstand wird aus Toluol/Heptan umkristallisiert und abschließend im Hochvakuum sublimiert. Die Reinheit beträgt 99,9%. Die Ausbeute beträgt 32,9 g (73% der Theorie).

Analog hierzu werden die folgenden Verbindungen (2-2) bis (2-11) hergestellt

| **Bsp.** | **Edukt 1** | **Edukt 2** | **Produkt:** | **Ausbeute** |
|---|---|---|---|---|
| 2-2 | 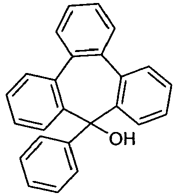 | 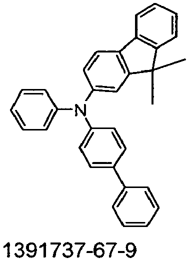 | 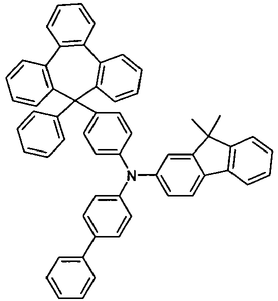 | 85% |
| 2-3 | 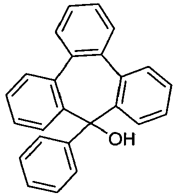 | 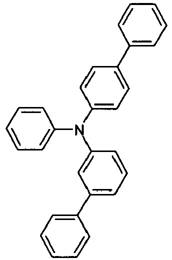 | 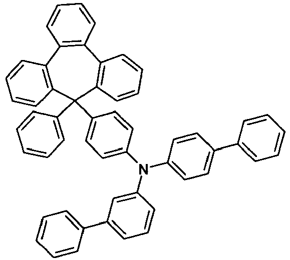 | 74 % |
| 2-4 | 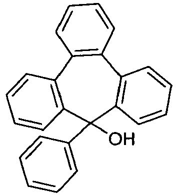 | 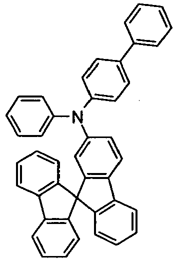 | 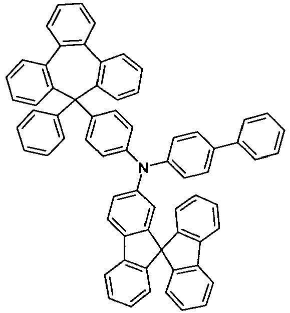 | 70 % |
| 2-6 | 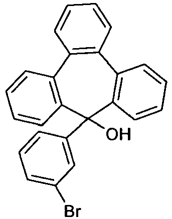 | 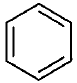 | 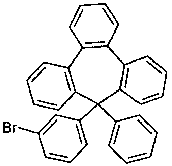 | 58 % |
| 2-7 | 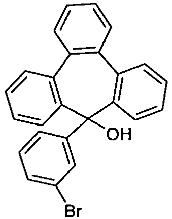 | 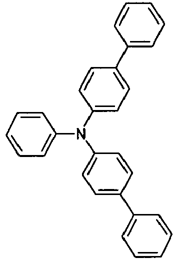 | 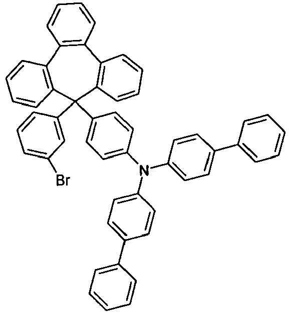 | 64% |
| 2-8 | 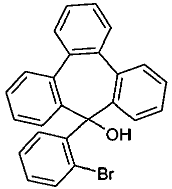 | 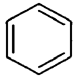 | 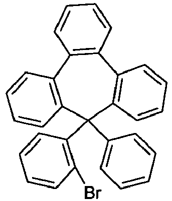 | 46% |
| 2-9 | 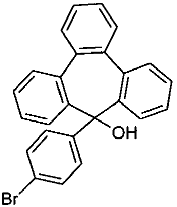 | 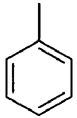 | 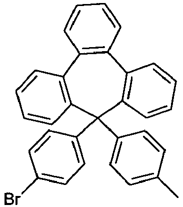 | 79 % |
| 2-10 | 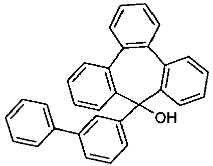 | 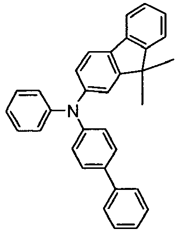 | 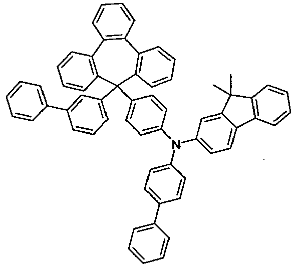 | 72 % |
| 2-11 | 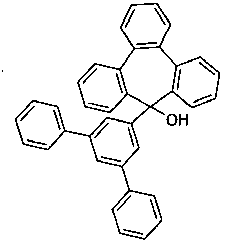 | 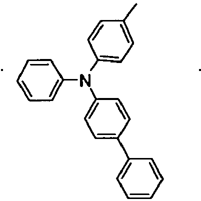 | 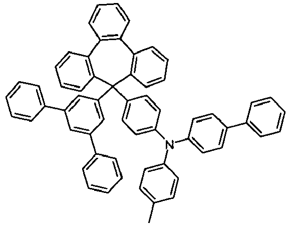 | 65% |

### Beispiel 3

### Synthese der Verbindung (3-1)

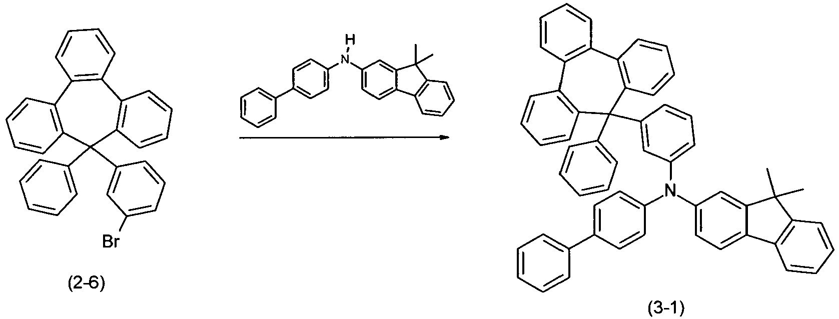

9,16 g Biphenyl-4-yl-(9,9-dimethyl-9H-fluoren-2-yl)-amin (25 mmol) und 12 g des Bromderivates (2-6) (25 mmol) werden in 200 mL Toluol gelöst. Die Lösung wird entgast und mit N₂ gesättigt. Danach wird sie mit 1,27 mL (1,27 mmol) einer 1 M Tri-tert-Butylphosphin-Lösung und 0,14 g (0.63 mmol) Palladium(II)acetat versetzt. Anschließend werden 6,1g Natrium-tert-butylat (63,4 mmol) zugegeben. Die Reaktionsmischung wird 8h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99,9%. Die Ausbeute beträgt 15 g (78 % der Theorie).

### Synthese der Verbindung (3-2) bis (3-8)

Analog hierzu werden die folgenden Verbindungen (3-2) bis (3-8) hergestellt.

| **Bsp** | **Edukt 1** | **Edukt 2** | **Produkt** | **Ausbeute** |
|---|---|---|---|---|
| 3-2 | 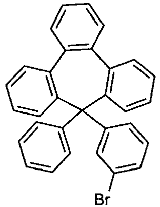 | 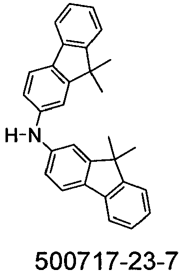 | 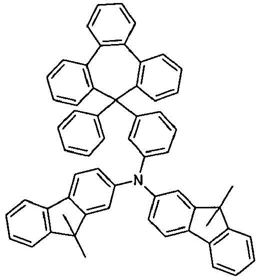 | 79 % |
| 3-3 | 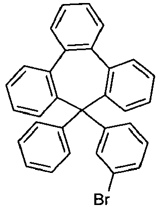 | 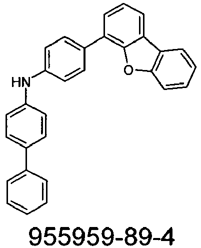 | 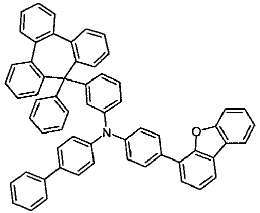 | 84% |
| 3-4 | 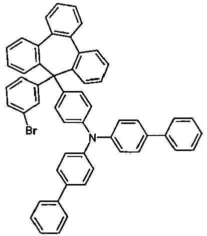 | 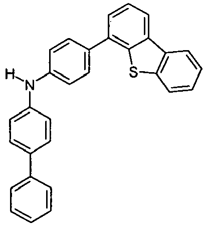 | 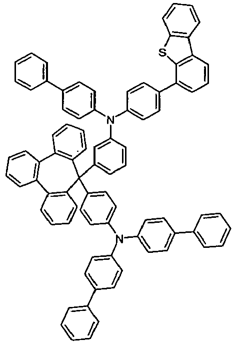 | 85% |
| 3-5 | 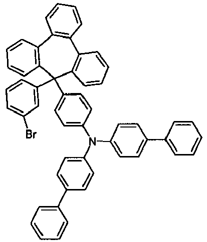 | 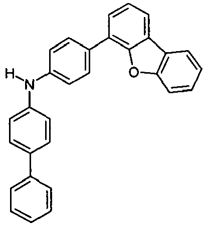 | 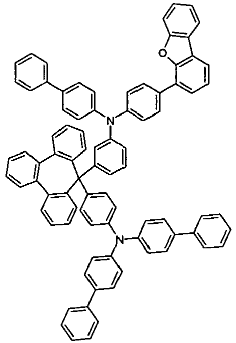 | 80% |
| 3-6 | 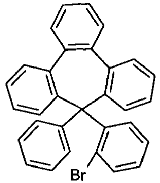 | 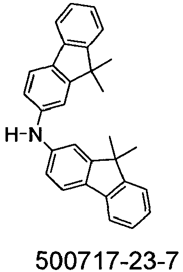 | 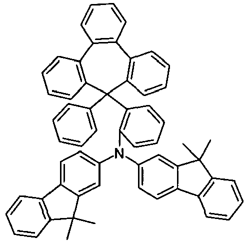 | 71% |
| 3-7 | 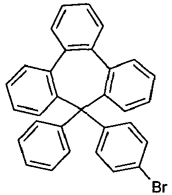 | 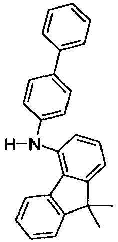 | 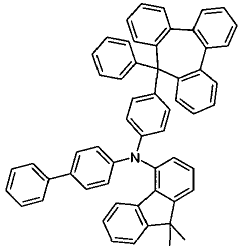 | 81% |
| 3-8 | 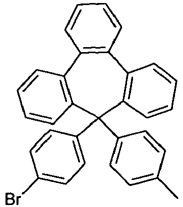 | 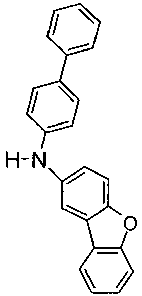 | 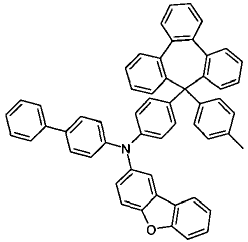 | 87% |

### Beispiel 4

### Synthese der Verbindung 4-1

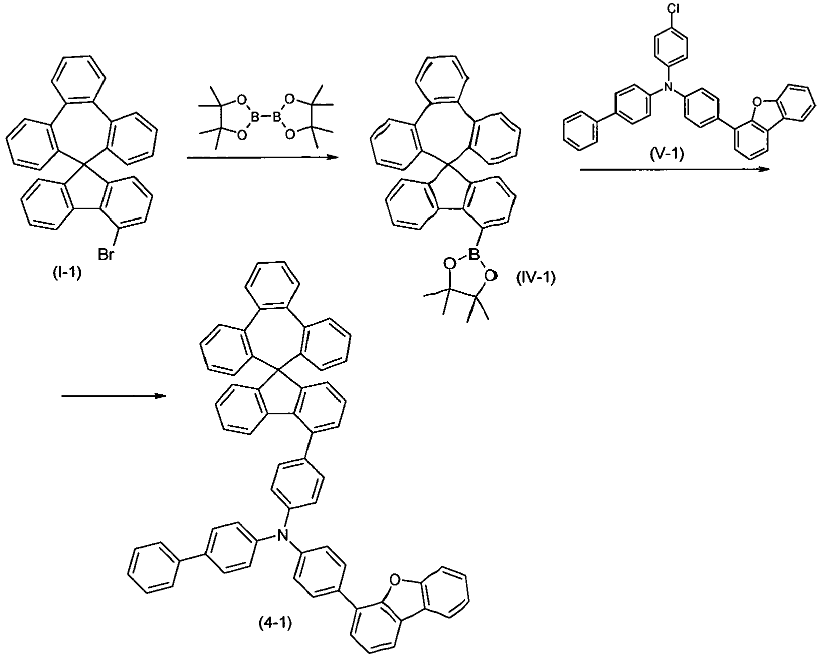

### Zwischenstufe: Boronester-Derivat (IV-1)

10 g (21,2 mmol) des Bromderivates 6,6 g (25,4 mmol) Bis(pinacolato)diboran und 6,3 g (63,6 mmol) Kalliumacetat werden in 200 ml DMF suspendiert. Zu dieser Suspension werden 0,52 g (0,64 mmol) 1,1-Bis(diphenylphosphino)ferrocen-dichloropalladium(II) Komplex mit DCM gegeben. Die Reaktionsmischung wird 6 h unter Rückfluss erhitzt. Nach Erkalten wird die organische Phase abgetrennt, dreimal mit 300 mL Wasser gewaschen und anschließend zur Trockene eingeengt. Der Rückstand wird aus Toluol umkristallisiert (10,3 g, 94% Ausbeute).

Analog dazu werden folgende Verbindungen (IV-2) bis (IV-13) hergestellt:

| **Bsp.** | **Edukt 1** | **Produkt** | **Ausbeute** |
|---|---|---|---|
| IV-2 | 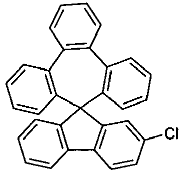 | 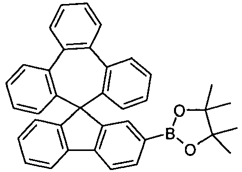 | 90% |
| IV-3 | 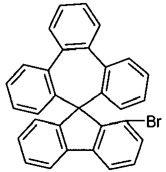 | 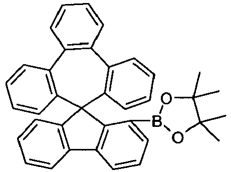 | 67% |
| IV-4 | 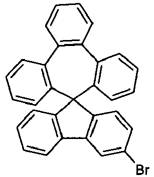 | 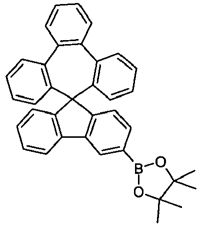 | 88% |
| IV-5 | 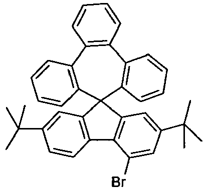 | 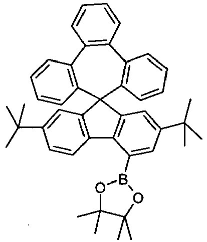 | 88% |
| IV-6 | 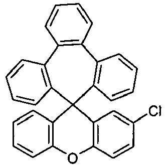 | 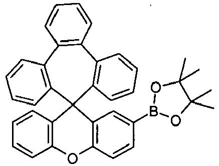 | 92% |
| IV-7 | 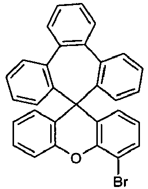 | 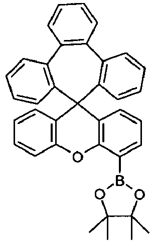 | 84% |
| IV-8 | 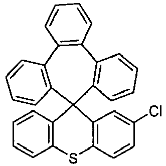 | 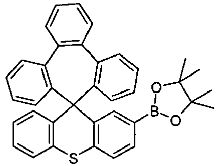 | 87% |
| IV-9 | 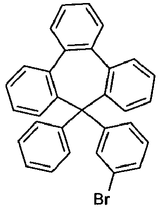 | 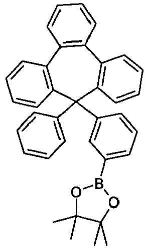 | 80% |
| IV-10 | 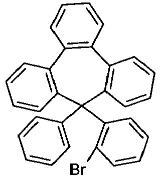 | 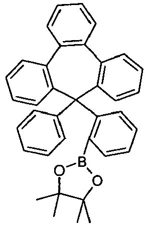 | 72% |
| IV-11 | 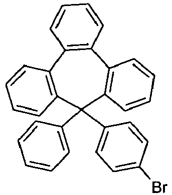 | 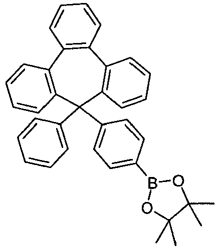 | 92% |
| IV-12 | 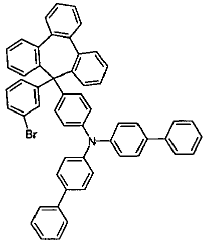 | 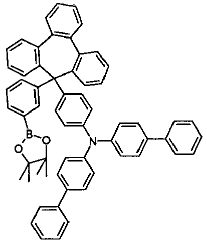 | 88% |
| IV-13 | 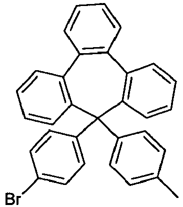 | 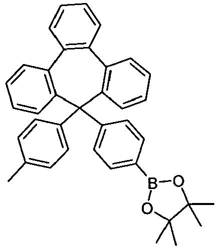 | 90% |

### Vorstufe: Biphenyl-4-yl-(4-chloro-phenyl)-(4-dibenzofuran-4-yl-phenyl)-amin (V-1)

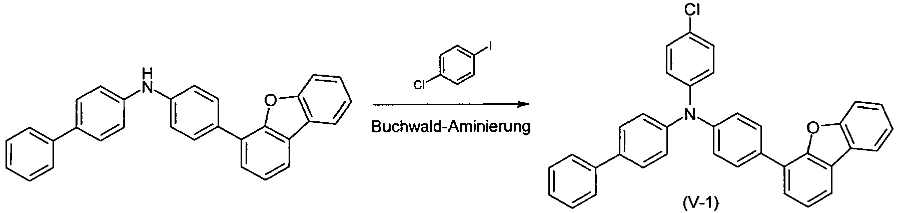

17 g Biphenyl-4-yl-(4-dibenzofuran-4-yl-phenyl)-amin (41mmol) und 14,8 g 4-Chlor-iodbenzol (62mmol) werden in 260 mL Toluol gelöst: Die Lösung wird entgast und mit N₂ gesättigt. Danach wird sie mit 1,6 mL (1,6 mmol) einer Tri-tert-Butylphosphin 1M-Lösung und 0,19 g (0,83 mmol) Palladium(II)acetat versetzt und anschließend werden 6,0 g Natrium-tert-butylat (62 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 16 g (75% d. Th).

Analog dazu werden folgende Verbindungen (V-2) bis (V-8) hergestellt:

| **Bsp** | **Edukt 1** | **Edukt 2** | **Produkt** | **Ausbeute** |
|---|---|---|---|---|
| V-2 | 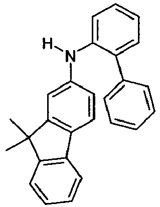 | 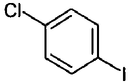 | 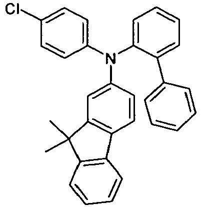 | 78 % |
| V-3 | 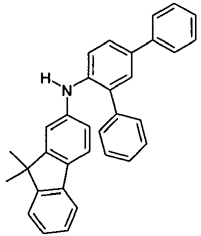 | 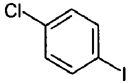 | 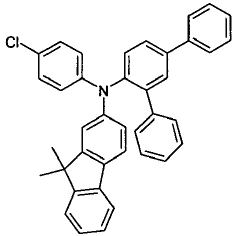 | 83% |
| V-4 | 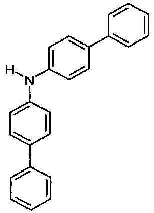 | 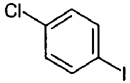 | 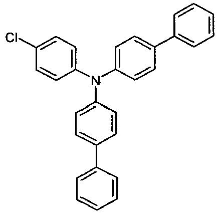 | 81% |
| V-5 | 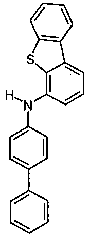 | 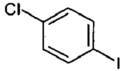 | 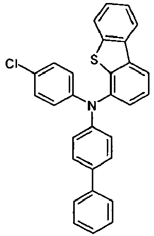 | 91% |
| V-6 | 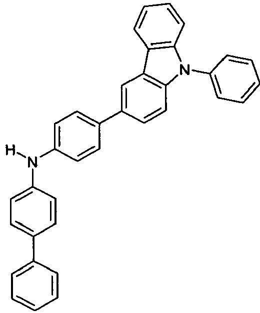 | 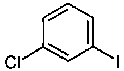 | 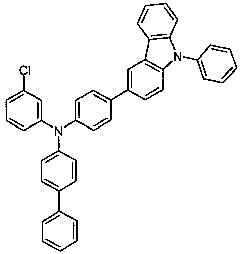 | 85% |
| V-7 | 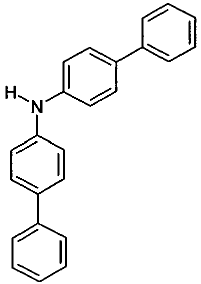 | 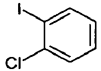 | 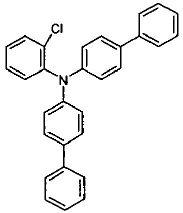 | 75% |
| V-8 | 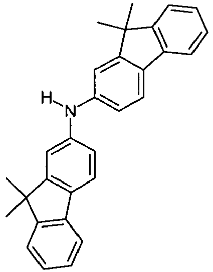 | 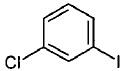 | 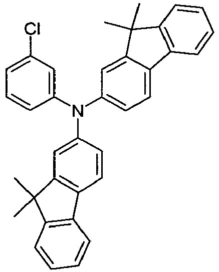 | 88% |

### Synthese der Verbindung 4-1

12 g (23,1 mmol) Pinacolboronester Derivat (IV-1) und 12,1 g (23,1 mmol) Chlor-Derivat (V-1) werden in 1750 mL Dioxan und 7,0 g Caesiumfluorid (46,3 mmol) suspendiert. Zu dieser Suspension werden 2,05 g (2,8 mmol) Palladium dichlorid-bis(tricyclohexylphosphin) gegeben, und die Reaktionsmischung wird 20 h unter Rückfluss erhitzt. Nach Erkalten wird die organische Phase abgetrennt, über Kieselgel filtriert, dreimal mit 100 mL Wasser gewaschen und anschließend zur Trockene eingeengt. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99,9%. Die Ausbeute beträgt 16,2 g (80% d. Th).

### Synthese der Verbindungen (4-2) bis (4-10)

Analog zu der in Beispiel 1 beschriebenen Synthese von Verbindung (2-1) werden auch die folgenden Verbindungen (4-2) bis (4-10) hergestellt.

| **Bsp** | **Edukt 1** | **Edukt 2** | **Produkt** | **Ausbeute** |
|---|---|---|---|---|
| 4-2 | 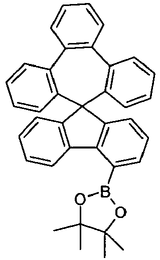 | 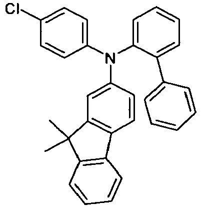 | 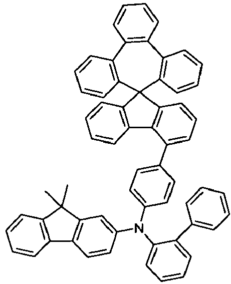 | 78 % |
| 4-3 | 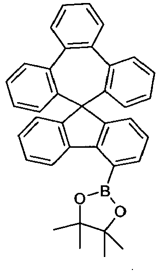 | 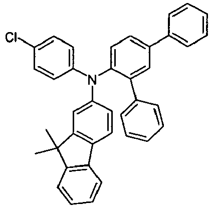 | 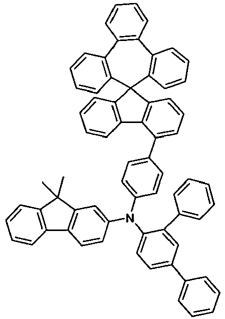 | 71% |
| 4-4 | 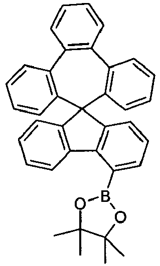 | 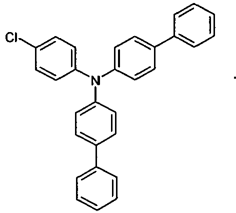 | 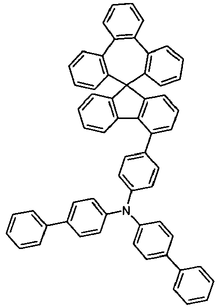 | 82% |
| 4-5 | 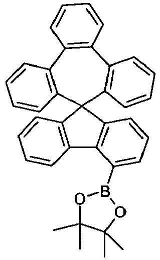 | 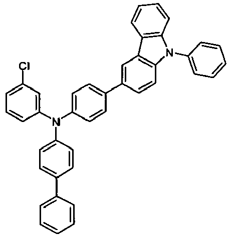 | 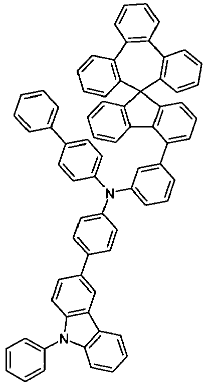 | 89% |
| 4-6 | 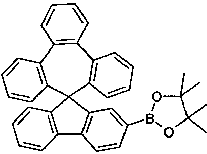 | 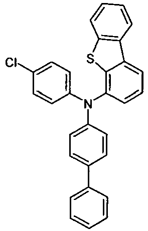 | 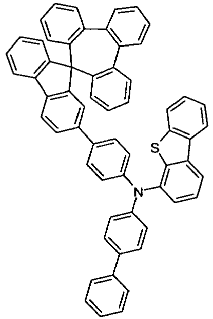 | 69% |
| 4-7 | 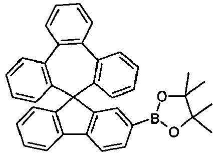 | 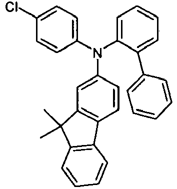 | 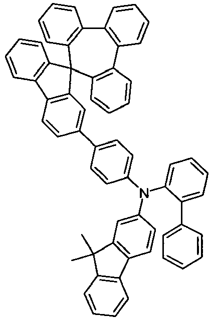 | 80% |
| 4-8 | 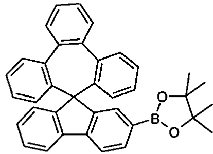 | 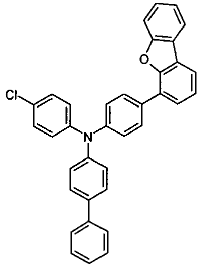 | 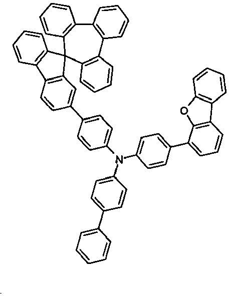 | 77% |
| 4-9 | 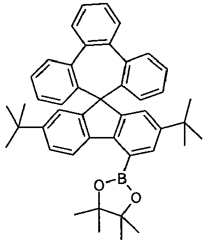 | 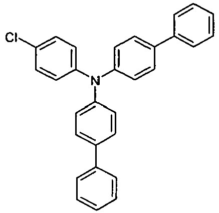 | 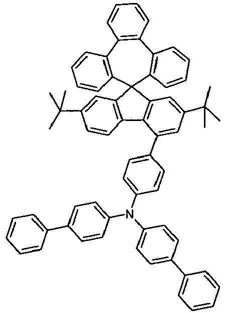 | 71% |
| 4-10 | 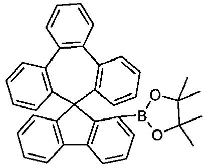 | 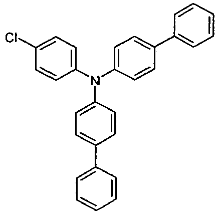 | 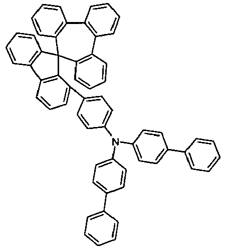 | 60% |
| | | | | |
| 4-11 | 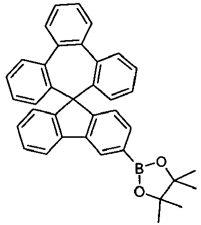 | 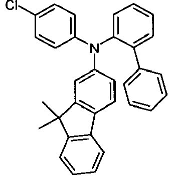 | 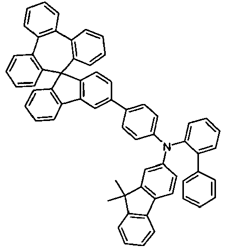 | 77% |
| 4-12 | 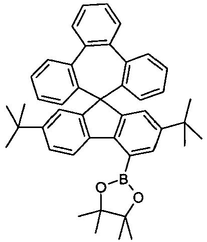 | 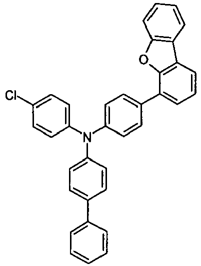 | 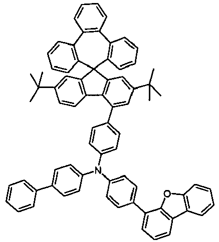 | 70% |
| 4-13 | 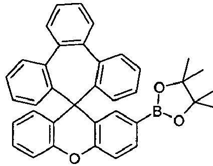 | 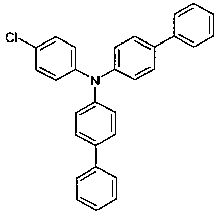 | 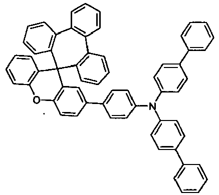 | 79% |
| 4-14 | 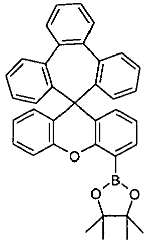 | 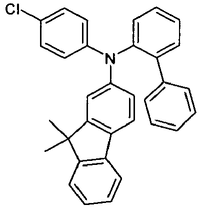 | 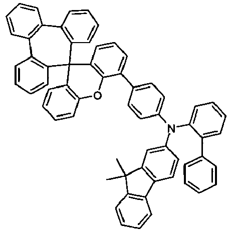 | 66% |
| 4-15 | 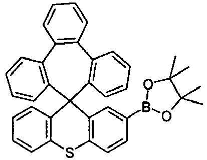 | 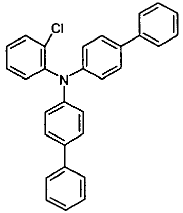 | 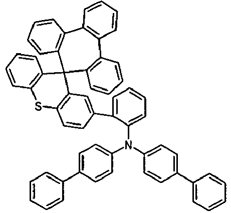 | 65% |
| 4-16 | 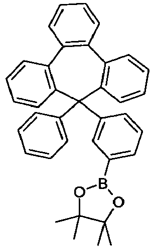 | 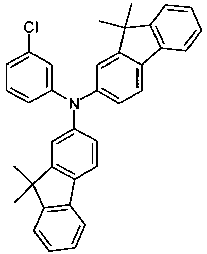 | 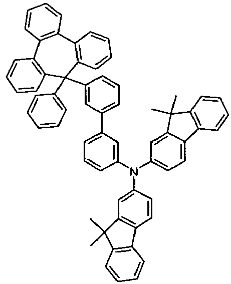 | 76% |
| 4-17 | 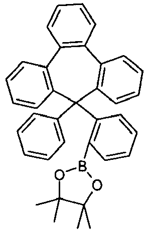 | 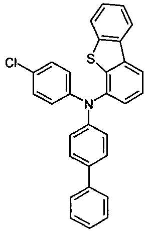 | 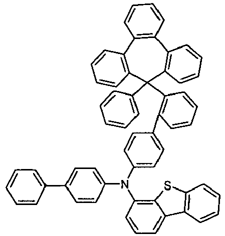 | 82% |
| 4-18 | 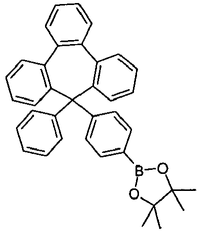 | 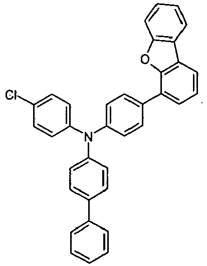 | 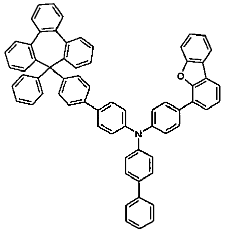 | 83% |
| 4-19 | 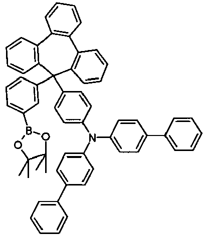 | 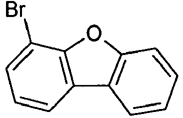 | 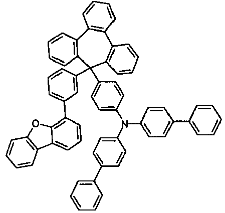 | 90% |
| 4-20 | 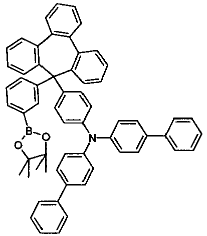 | 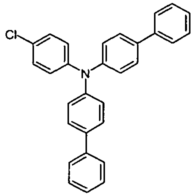 | 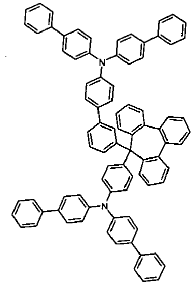 | 61% |
| 4-21 | 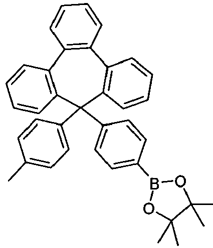 | 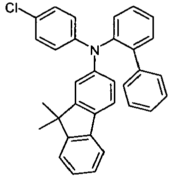 | 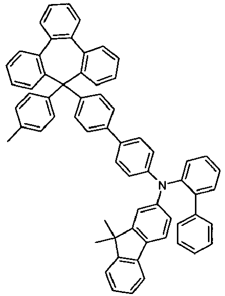 | 77% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden erfindungsgemäßen Beispielen werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben folgenden Schichtaufbau: Substrat / p-dotierte Lochtransportschicht (HIL1) / Lochtransportschicht (HTL) / p-dotierte Lochtransportschicht (HTL2) / Elektronenblockierschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1 :SEB(5%) bedeutet hierbei, dass das Material H1 in einem Volumenanteil von 95 % und SEB in einem Volumenanteil von 5 % in der Schicht vorliegt. Analog können auch die Elektronentransportschichten oder die Lochinjektionsschichten aus einer Mischung von zwei oder mehr Materialien bestehen.

Die OLEDs werden standardmäßig charakterisiert. Hierfür wird die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Angabe EQE @ 10 mA/cm² bezeichnet die externe Quanteneffizienz bei einer Stromdichte von 10mA/cm². LD80 @ 60 mA/cm² ist die Lebensdauer, bis zu der die OLED bei einer Starthelligkeit bei konstantem Strom von 60 mA/cm² auf 80 % der Anfangsintensität abgefallen ist.

| **Tabelle 1: Strukturen der verwendeten Materialien** | | |
|---|---|---|
| 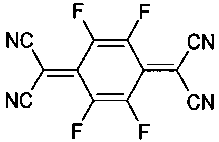 | 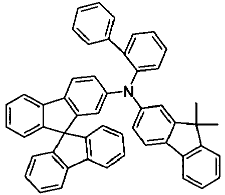 | 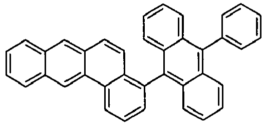 |
| F4TCNQ | HIM | H1 |
| 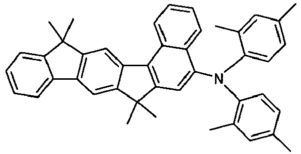 | 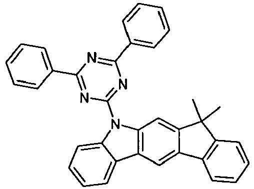 | 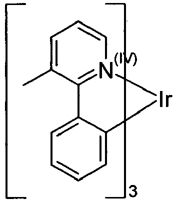 |
| SEB | H2 | TEG |
| 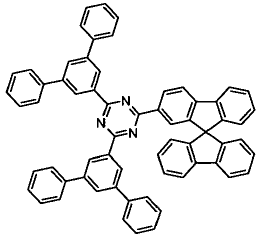 | 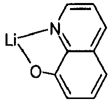 | 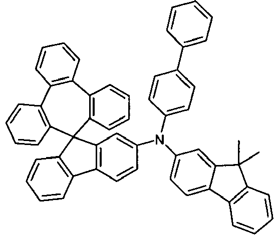 |
| ETM | LiQ | HTM1 |
| 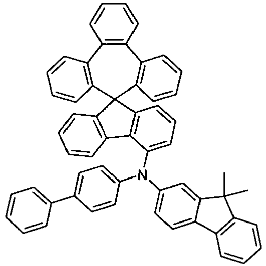 | 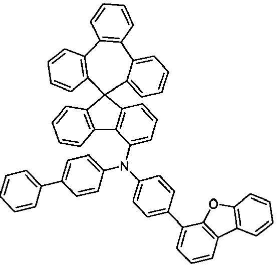 | 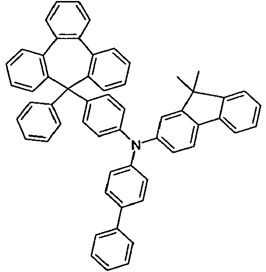 |
| HTM2 | HTM3 | HTM4 |
| 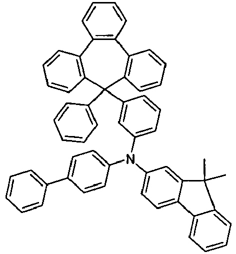 | | |
| HTM5 | | |

Es werden die Proben HTM1, HTM2, HTM3, HTM4 und HTM5 in einer blau fluoreszierenden OLED miteinander verglichen. Der Aufbau des Stacks gliedert sich wie folgt: HIM:F4TCNQ(5%)(20nm) / HIM(155nm) / HTM_x: F4TCNQ(5%)(20nm) / HTM_x(20nm) / H1:SEB(5%)(20nm) / ETM:LiQ(50%)(30nm) / LiQ(1nm). HTM_x ist jeweils das erfindungsgemäße Material HTM1, HTM2, HTM3, HTM4 oder HTM5. Wird in der dritten Schicht an der Stelle von HTM_x beispielsweise das dotierte HTM1 verwendet, so muss auch in der darauffolgenden Schicht HTM1 verwendet werden. Ein Kreuzkombination aus dotiertem HTM1 und undotiertem HTM2 wird hier nicht betrachtet.

Die Evaluation der externen Quanteneffizienzen bei 10 mA/cm² zeigt für die durchgeführten Experimente die folgenden Ergebnisse: HTM1 erzielt 8,6% EQE, wohingegen HTM2 8,2%, HTM3 8,1%, HTM4 7,8% und HTM5 8,0% erzielen. Die Lebensdauern der erzeugten Devices bis zum Abfall auf 80% der Anfangsintensität bei einem konstanten Strom von 60 mA/cm² belaufen sich auf 310 Stunden bei HTM1, 400 Stunden für HTM2, 420 Stunden bei HTM3, 350 Stunden für HTM4 und 410 Stunden für HTM5.

Mit den beiden gleichen Materialien wird ein Triplett-Grün-Bauteil hergestellt, dessen Aufbau sich wie folgt gliedert: HIM:F4TCNQ(5%)(20 nm) / HIM(210 nm) / HTM_x:F4TCNQ(5%)(20nm) / HTM_x(20nm) / H2:TEG(10%)(30 nm) / ETM:LiQ(50%)(40 nm) / LiQ(1 nm). HTM_x ist jeweils das erfindungsgemäße Material HTM1, HTM2, HTM3, HTM4 oder HTM5. Wird in der dritten Schicht an Stelle HTM_x beispielsweise das dotierte HTM1 verwendet, so muss auch in der darauffolgenden Schicht HTM1 verwendet werden. Ein Kreuzkombination aus beispielsweise dotiertem HTM1 und undotiertem HTM2 wird hier nicht betrachtet.

Die externen Quanteneffizienzen zeigt ein ähnlichen Trend wie bei der oben beschriebenen blau fluoreszierenden OLED. Die externe Quanteneffizienz für HTM1 liegt bei 2 mA/cm² in diesem Versuch bei 18,3%. Die Bauteile mit HTM2 erzielen 18,2%, HTM3 17,2%, HTM4 17,5% und HTM5 18,0%. Die Lebensdauern sind ähnlich hoch wie bei der blau fluoreszierenden OLED: HTM1 hat bei 20mA/cm² eine Lebensdauer bis zum Abfall auf 80% der Anfangsintensität von 155 Stunden. HTM2 besitzt eine LD80 von 210 Stunden, HTM3 von 220 Stunden, HTM4 von 190 Stunden und HTM5 von 210 Stunden.

## Patentansprüche

1. Verbindung gemäß Formel (1) 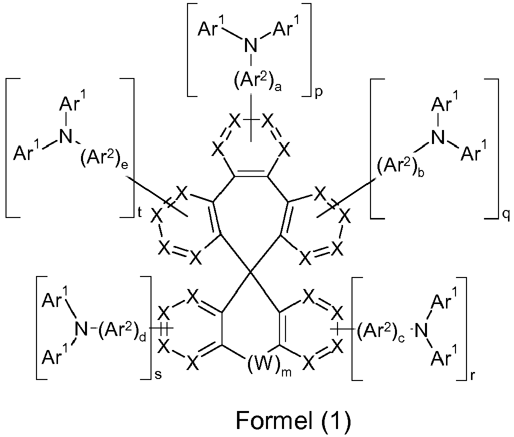 wobei für die verwendeten Symbole und Indizes gilt:
X ist bei jedem Auftreten gleich oder verschieden CR¹ oder N;
Ar² ist bei jedem Auftreten gleich oder verschieden ein bivalentes aromatisches oder heteroaromatisches Ringsystem mit 5 bis 60 aromatischen Ringatomen, welches mit einem oder mehreren Resten R² substituiert sein kann;
Ar¹ ist bei jedem Auftreten gleich oder verschieden ein aromatisches oder heteroaromatisches Ringsystem mit 5 bis 60 aromatischen Ringatomen, das jeweils auch mit einem oder mehreren Resten R² substituiert sein kann; wobei an das gleiche N-Atom gebundene Reste Ar¹ und/oder Ar² über mindestens eine Gruppe K verbunden sein können;
K ist bei jedem Auftreten gleich oder verschieden eine Einfachbindung oder eine bivalente Brücke, ausgewählt aus N(R²), B(R²), O,C=O, C(R²)₂, Si(R²)₂, C=C(R²)₂, S=O, P(R²), P(=O)R² und S;
W ist bei jedem Auftreten gleich oder verschieden eine Einfachbindung oder eine bivalente Brücke, ausgewählt aus B(R²), O, C=O, C(R²)₂, Si(R²)₂, S und R²C=CR²;
Ar ist bei jedem Auftreten gleich oder verschieden ein aromatisches oder heteroaromatisches Ringsystem, bevorzugt eine Aryl- oder Heteroarylgruppe mit 5 bis 40 aromatischen Ringatomen, die mit einem oder mehreren Resten R³ substituiert sein kann;
R¹ ist bei jedem Auftreten gleich oder verschieden H, D, F, Cl, Br, I, C(=O)Ar, P(=O)Ar₂, S(=O)Ar, S(=O)₂Ar, CR²=CR²Ar, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R² substituiert sein kann, oder eine Aryloxy- oder Heteroaryloxygruppe mit 5 bis 40 aromatischen Ringatomen, die mit einem oder mehreren Resten R² substituiert sein kann, oder eine Aralkyl- oder Heteroaralkylgruppe mit 5 bis 40 aromatischen Ringatomen, die durch einen oder mehrere Reste R² substituiert sein kann;
R² ist bei jedem Auftreten gleich oder verschieden H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, C=O, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils mit einem oder mehreren Resten R³ substituiert sein kann, oder eine Aryloxy- oder Heteroaryloxygruppe mit 5 bis 40 aromatischen Ringatomen, die mit einem oder mehreren Resten R³ substituiert sein kann, oder eine Aralkyl- oder Heteroaralkylgruppe mit 5 bis 40 aromatischen Ringatomen, die durch einen oder mehrere Reste R³ substituiert sein kann;
R³ ist bei jedem Auftreten gleich oder verschieden H, D, F, Cl, Br, I, C(=O)Ar, P(=O)Ar₂, S(=O)Ar, S(=O)₂Ar, CR⁴=CR⁴Ar, CN, NO₂, Si(R⁴)₃, B(OR⁴)₂, OSO₂R⁴, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R⁴ substituiert sein kann, wobei eine oder mehrere nicht benachbarte CH₂-Gruppen durch R⁴C=CR⁴, C=C , Si(R⁴)₂, C=O, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R⁴ substituiert sein kann, oder eine Aryloxy- oder Heteroaryloxygruppe mit 5 bis 40 aromatischen Ringatomen, die durch einen oder mehrere Reste R⁴ substituiert sein kann, oder eine Aralkyl- oder Heteroaralkylgruppe mit 5 bis 40 aromatischen Ringatomen, die durch einen oder mehrere Reste R⁴ substituiert sein kann;
R⁴ ist bei jedem Auftreten gleich oder verschieden H, D, F oder ein aliphatischer Kohlenwasserstoffrest mit 1 bis 20 C-Atomen oder eine Aryl- oder Heteroarylgruppe mit 5 bis 40 Ringatomen, die durch einen oder mehrere Reste R⁵ substituiert sein kann, oder eine Kombination dieser Gruppen;
R⁵ ist bei jedem Auftreten gleich oder verschieden H, D, F oder ein aliphatischer Kohlenwasserstoffrest mit 1 bis 20 C-Atomen;
m ist 0 oder 1, wobei m gleich 0 bedeutet, dass an dieser Position keine Gruppe W gebunden ist, und die betreffenden an W bindenden Kohlenstoffatome jeweils durch eine Gruppe X ersetzt sind; und
p, q, r, s, t sind bei jedem Auftreten gleich oder verschieden 0, 1 oder 2;
a, b, c, d, e sind bei jedem Auftreten gleich oder verschieden 0, 1 oder 2;
wobei
p+q+r+s+t größer als 1 oder gleich 1 ist; und
dass, falls r größer oder gleich 1 ist und s größer oder gleich 1 ist und m gleich 0 ist, und für je mindestens eine Gruppe (Ar²)_{c}N(Ar¹)₂ und (Ar²)_{d}N(Ar¹)2 c und d gleich 0 sind, diese beiden N(Ar¹)₂-Gruppen nicht jeweils in para-Position zum quartären Kohlenstoffatom des Grundgerüsts angeordnet sind.

2. Verbindung nach Anspruch 1, **dadurch gekennzeichnet, dass** W eine Einfachbindung ist.

3. Verbindung nach Anspruch 1 oder 2, **dadurch gekennzeichnet, dass** die Summe der Werte der Indices p, q, r, s, und t gleich 1 ist.

4. Verbindung nach einem oder mehreren der Ansprüche 1 bis 3, **dadurch gekennzeichnet, dass** der Index r gleich 1 ist, und die Indices p, q, s, und t gleich 0 sind, oder **dadurch gekennzeichnet, dass** der Index s gleich 1 ist, und die Indices p, q, r, und t gleich 0 sind.

5. Verbindung nach einem oder mehreren der Ansprüche 1 bis 4, **dadurch gekennzeichnet, dass** sie Formel (2) entspricht 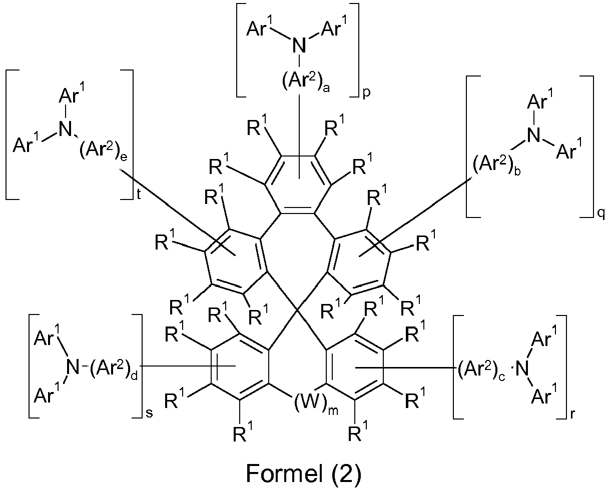 wobei die weiteren verwendeten Symbole und Indizes die in Anspruch 1 genannten Bedeutungen aufweisen.

6. Verbindung nach einem oder mehreren der Ansprüche 1 bis 5,
**dadurch gekennzeichnet, dass** die Verbindung eine Verbindung gemäß einer der Formeln (4) und (5) ist: 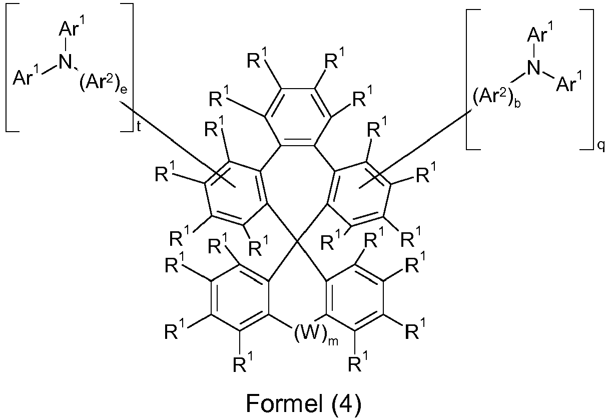 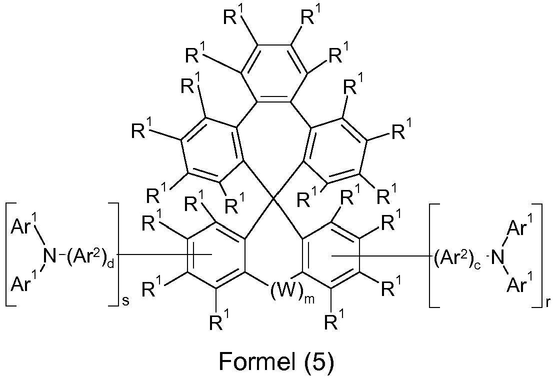 darstellt, wobei die verwendeten Symbole und Indizes die in Anspruch 1 genannten Bedeutungen aufweisen.

7. Verbindung nach einem oder mehreren der Ansprüche 1 bis 6,
**dadurch gekennzeichnet, dass** die Verbindung eine Verbindung gemäß einer der Formeln (6) bis (11): 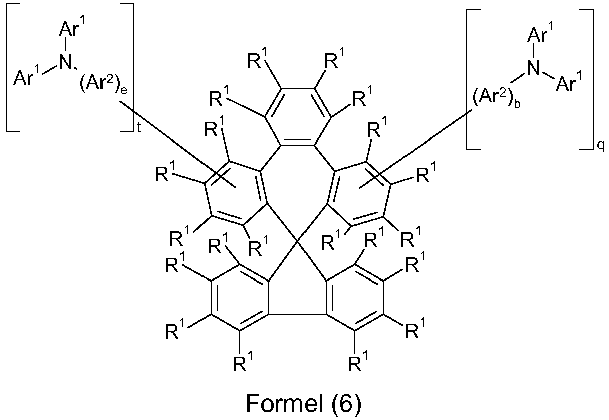 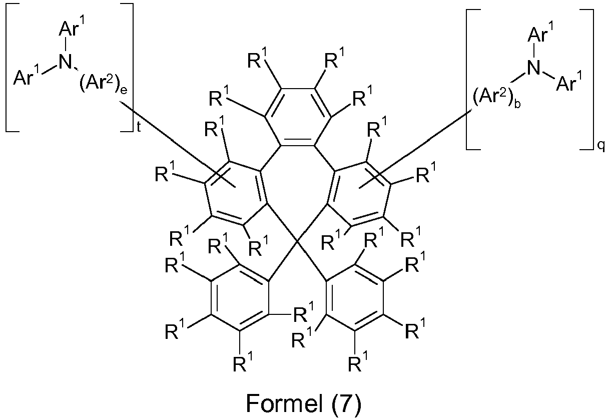 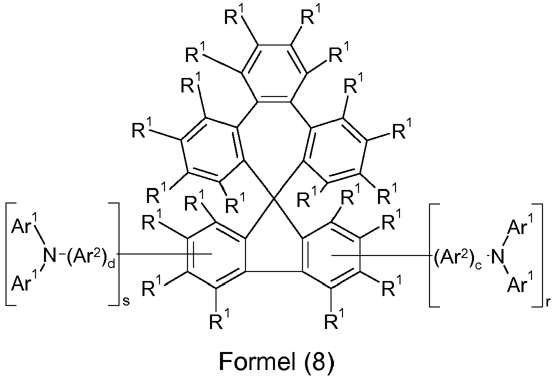 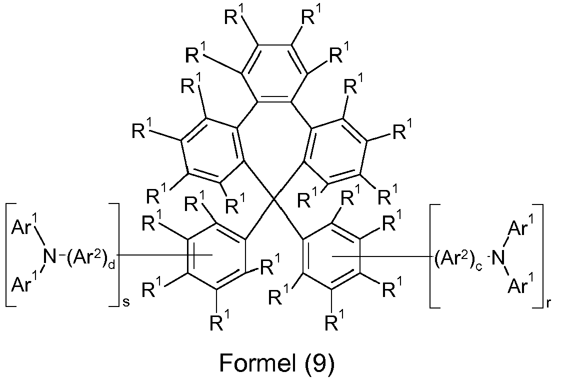 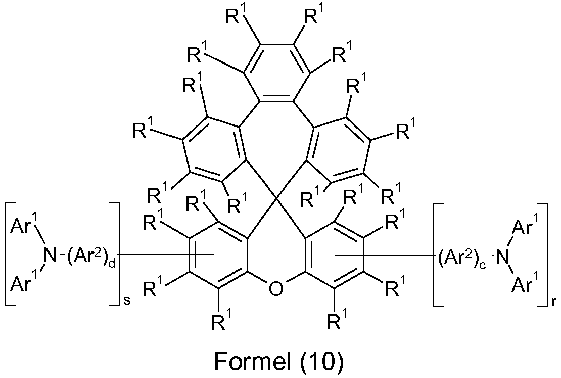 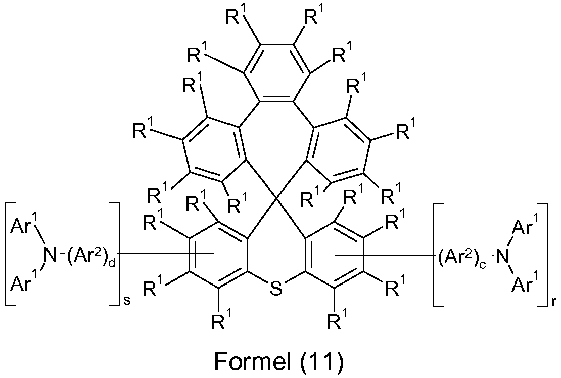 darstellt, wobei die verwendeten Symbole und Indizes die in Anspruch 1 genannten Bedeutungen aufweisen.

8. Verfahren zur Herstellung einer Verbindung nach einem oder mehreren der Ansprüche 1 bis 7, **dadurch gekennzeichnet, dass** die Verbindung der Formel (1) durch eine oder mehrere Kupplungsreaktionen und/oder Zyklisierungen aufgebaut wird.

9. Mischung enthaltend mindestens eine Verbindung nach einem oder mehreren der Ansprüche 1 bis 7 und mindestens einen fluoreszierenden oder phosphoreszierenden Dotanden.

10. Formulierung, insbesondere eine Lösung, eine Suspension oder eine Miniemulsion, enthaltend mindestens eine Verbindung nach einem oder mehreren der Ansprüche 1 bis 7 oder eine Mischung nach Anspruch 9, und ein oder mehrere Lösemittel.

11. Verwendung einer Verbindung nach einem oder mehreren der Ansprüche 1 bis 7 oder einer Mischung nach Anspruch 9 in einer elektronischen Vorrichtung.

12. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices", enthaltend mindestens eine Verbindung nach einem oder mehreren der Ansprüche 1 bis 7 oder eine Mischung nach Anspruch 9.

13. Elektronische Vorrichtung nach Anspruch 12, **dadurch gekennzeichnet, dass** es sich um eine organische Elektrolumineszenzvorrichtung handelt und die Verbindung nach einem oder mehreren der Ansprüche 1 bis 7 als Matrixmaterial für eine fluoreszierende oder phosphoreszierende Verbindung in einer emittierenden Schicht, und/oder in einer Lochtransportschicht und/oder in einer Lochinjektionsschicht und/oder in einer Elektronenblockierschicht eingesetzt.

## Claims

1. Compound of the formula (1) 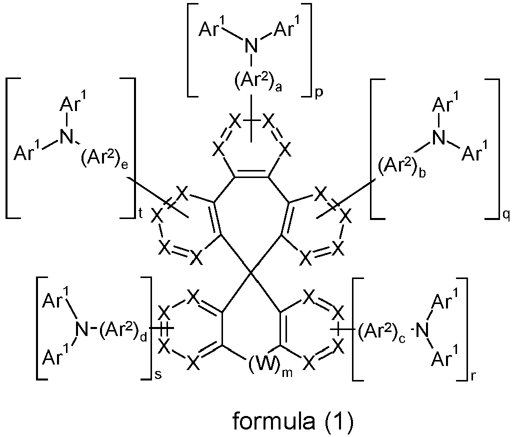 where the following applies to the symbols and indices used:
X is on each occurrence, identically or differently, CR¹ or N;
Ar² is on each occurrence, identically or differently, a divalent aromatic or heteroaromatic ring system having 5 to 60 aromatic ring atoms, which may be substituted by one or more radicals R²;
Ar¹ is on each occurrence, identically or differently, an aromatic or heteroaromatic ring system having 5 to 60 aromatic ring atoms, which may in each case also be substituted by one or more radicals R²; where radicals Ar¹ and/or Ar² which are bonded to the same N atom may be connected via at least one group K;
K is on each occurrence, identically or differently, a single bond or a divalent bridge selected from N(R²), B(R²), O, C=O, C(R²)₂, Si(R²)₂, C=C(R²)₂, S=O, P(R²), P(=O)R² and S;
W is on each occurrence, identically or differently, A single bond or a divalent bridge selected from B(R²), O, C=O, C(R²)₂, Si(R²)₂, S and R²C=CR²;
Ar is on each occurrence, identically or differently, an aromatic or heteroaromatic ring system, preferably an aryl or heteroaryl group having 5 to 40 aromatic ring atoms, which may be substituted by one or more radicals R³;
R¹ is on each occurrence, identically or differently, H, D, F, CI, Br, I, C(=O)Ar, P(=O)Ar₂, S(=O)Ar, S(=O)₂Ar, CR²=CR²Ar, CN, NO₂, Si(R²)₃, B(OR²)₂, OSO₂R², a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R², where one or more non-adjacent CH₂ groups may be replaced by R²C=CR², C=C, Si(R²)₂, C=O, C=NR², P(=O)(R²), SO, SO₂, NR², O, S or CONR² and where one or more H atoms may be replaced by D, F, CI, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 40 aromatic ring atoms, which may be substituted by one or more radicals R², or eine aralkyl or heteroaralkyl group having 5 to 40 aromatic ring atoms, which may be substituted by one or more radicals R²;
R² is on each occurrence, identically or differently, H, D, F, CI, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R³, where one or more non-adjacent CH₂ groups may be replaced by R³C=CR³, C=C , Si(R³)₂, C=O, C=NR³, P(=O)(R³), SO, SO₂, NR³, O, S or CONR³ and where one or more H atoms may be replaced by D, F, CI, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 40 aromatic ring atoms, which may be substituted by one or more radicals R³, or an aralkyl or heteroaralkyl group having 5 to 40 aromatic ring atoms, which may be substituted by one or more radicals R³;
R³ is on each occurrence, identically or differently, H, D, F, CI, Br, I, C(=O)Ar, P(=O)Ar₂, S(=O)Ar, S(=O)₂Ar, CR⁴=CR⁴Ar, CN, NO₂, Si(R⁴)₃, B(OR⁴)₂, OSO₂R⁴, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R⁴, where one or more non-adjacent CH₂ groups may be replaced by R⁴C=CR⁴, C=C , Si(R⁴)₂, C=O, C=NR⁴, P(=O)(R⁴), SO, SO₂, NR⁴, O, S or CONR⁴ and where one or more H atoms may be replaced by D, F, CI, Br, I, CN or NO₂, or an aromatic or heteroaromatic ring system having 5 to 60 aromatic ring atoms, which may in each case be substituted by one or more radicals R⁴, or an aryloxy or heteroaryloxy group having 5 to 40 aromatic ring atoms, which may be substituted by one or more radicals R⁴, or an aralkyl or heteroaralkyl group having 5 to 40 aromatic ring atoms, which may be substituted by one or more radicals R⁴;
R⁴ is on each occurrence, identically or differently, H, D, F or an aliphatic hydrocarbon radical having 1 to 20 C atoms or an aryl or heteroaryl group having 5 to 40 ring atoms, which may be substituted by one or more radicals R⁵, or a combination of these groups;
R⁵ is on each occurrence, identically or differently, H, D, F or an aliphatic hydrocarbon radical having 1 to 20 C atoms;
m is 0 or 1, where m equals 0 means that a group W is not bonded at this position, and the relevant carbon atoms bonded to W have in each case been replaced by a group X; and
p, q, r, s, t are on each occurrence, identically or differently, 0, 1 or 2;
a, b, c, d, e are on each occurrence, identically or differently, 0, 1 or 2;
where
p+q+r+s+t is greater than 1 or equal to 1; and
if r is greater than or equal to 1 and s is greater than or equal to 1 and m is equal to 0, and c and d are equal to 0 for at least one group (Ar²)_{c}N(Ar¹)₂ and (Ar²)_{d}N(Ar¹)2 in each case, neither of these two N(Ar¹)₂ groups is arranged in the para position to the quaternary carbon atom of the skeleton.

2. Compound according to Claim 1, **characterised in that** W is a single bond.

3. Compound according to Claim 1 or 2, **characterised in that** the sum of the values of the indices p, q, r, s and t is equal to 1.

4. Compound according to one or more of Claims 1 to 3, **characterised in that** the index r is equal to 1, and the indices p, q, s and t are equal to 0, or **characterised in that** the index s is equal to 1, and the indices p, q, r and t are equal to 0.

5. Compound according to one or more of Claims 1 to 4, **characterised in that** it conforms to formula (2) 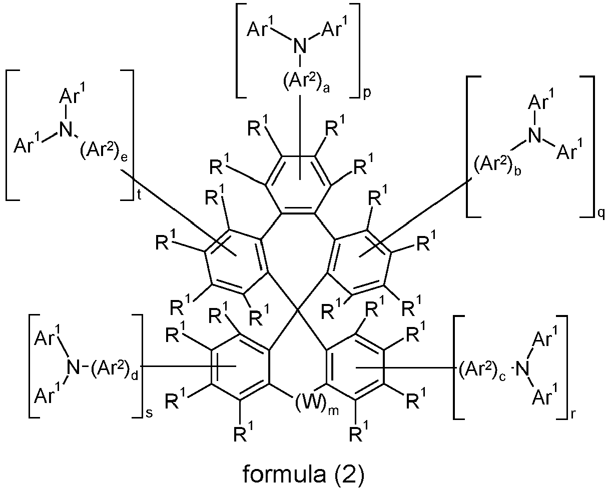 where the other symbols and indices used have the meanings given in Claim 1.

6. Compound according to one or more of Claims 1 to 5, **characterised in that** the compound is a compound of one of the formulae (4) and (5): 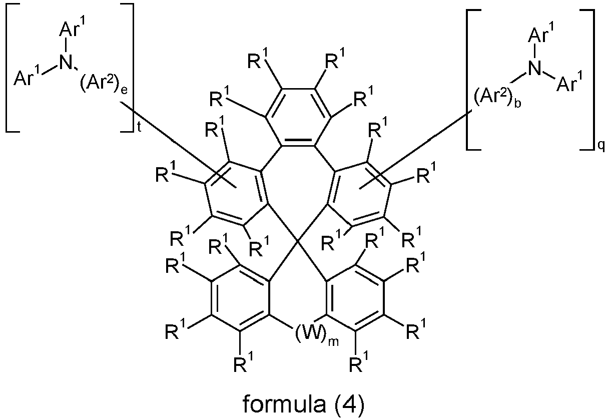 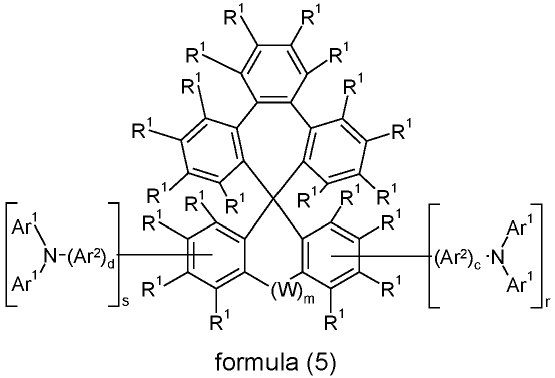 where the symbols and indices used have the meanings given in Claim 1.

7. Compound according to one or more of Claims 1 to 6, **characterised in that** the compound is a compound of one of the formulae (6) to (11): 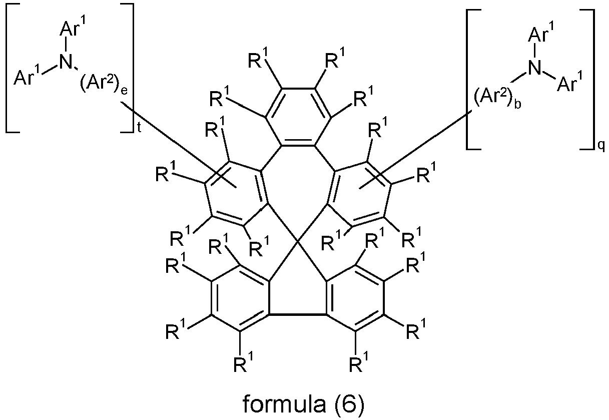 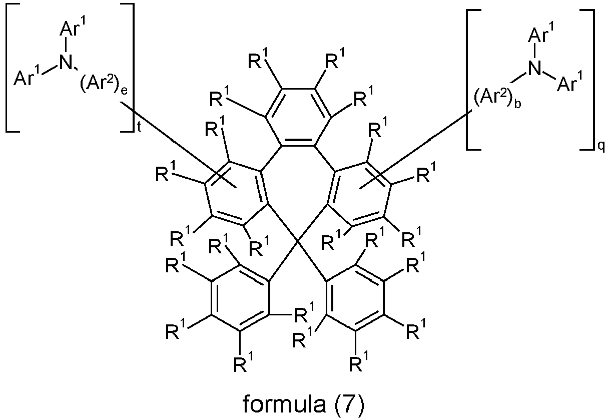 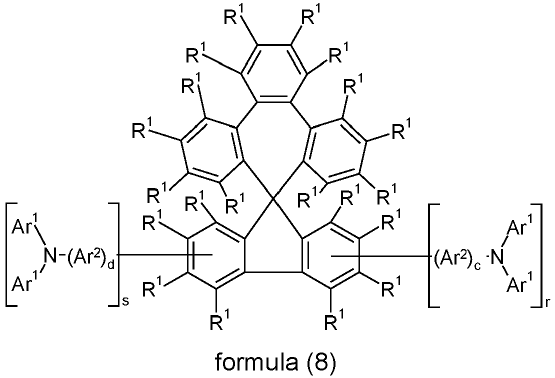 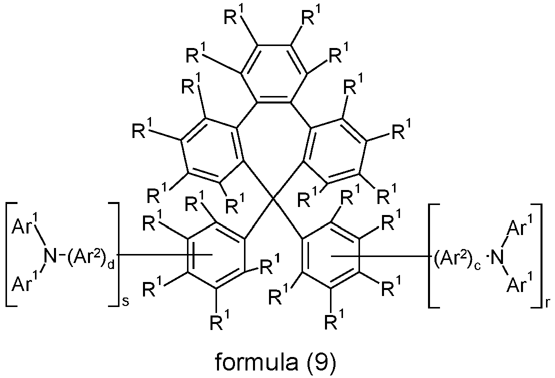 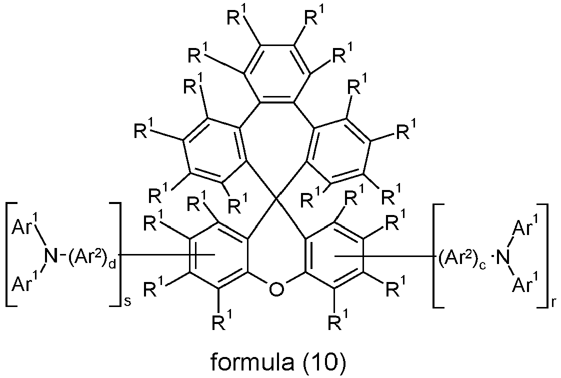 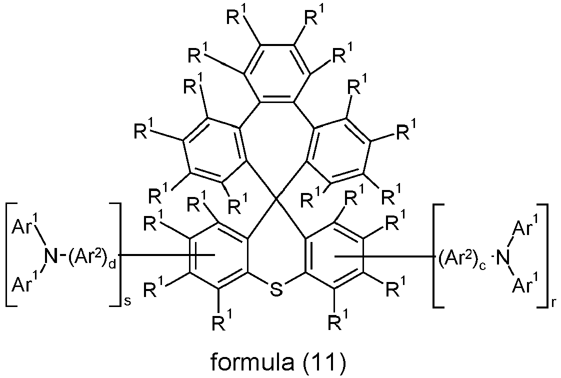 where the symbols and indices used have the meanings given in Claim 1.

8. Process for the preparation of a compound according to one or more of Claims 1 to 7, **characterised in that** the compound of the formula (1) is built up by one or more coupling reactions and/or cyclisations.

9. Mixture comprising at least one compound according to one or more of Claims 1 to 7 and at least one fluorescent or phosphorescent dopant.

10. Formulation, in particular a solution, a suspension or a miniemulsion, comprising at least one compound according to one or more of Claims 1 to 7 or a mixture according to Claim 9, and one or more solvents.

11. Use of a compound according to one or more of Claims 1 to 7 or a mixture according to Claim 9 in an electronic device.

12. Electronic device, in particular selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and "organic plasmon emitting devices", containing at least one compound according to one or more of Claims 1 to 7 or a mixture according to Claim 9.

13. Electronic device according to Claim 12, **characterised in that** it is an organic electroluminescent device and the compound according to one or more of Claims 1 to 7 is employed as matrix material for a fluorescent or phosphorescent compound in an emitting layer, and/or in a hole-transport layer and/or in a hole-injection layer and/or in an electron-blocking layer.

## Revendications

1. Composé de la formule (1) 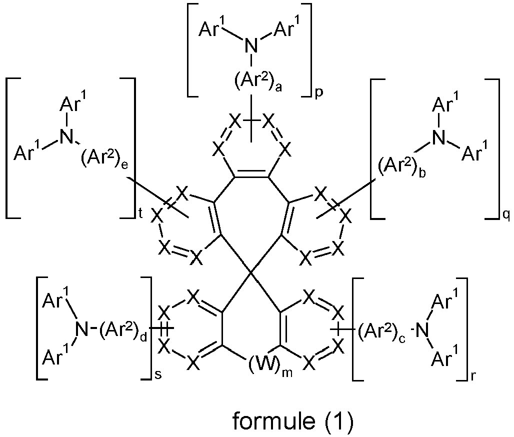 dans laquelle ce qui suit s'applique aux symboles et aux indices qui sont utilisés :
X est pour chaque occurrence, de manière identique ou différente, CR¹ ou N ;
Ar² est pour chaque occurrence, de manière identique ou différente, un système de cycle aromatique ou hétéroaromatique divalent qui comporte de 5 à 60 atomes de cycle aromatique, lequel peut être substitué par un radical ou par plusieurs radicaux R² ;
Ar¹ est pour chaque occurrence, de manière identique ou différente, un système de cycle aromatique ou hétéroaromatique qui comporte de 5 à 60 atomes de cycle aromatique, lequel peut dans chaque cas également être substitué par un radical ou par plusieurs radicaux R² ; où les radicaux Ar¹ et/ou Ar² qui sont liés au même atome de N peuvent être connectés via au moins un groupe K ;
K est pour chaque occurrence, de manière identique ou différente, une liaison simple ou un pont divalent qui est sélectionné parmi N(R²), B(R²), O, C=O, C(R²)₂, Si(R²)₂, C=C(R²)₂, S=O, P(R²), P(=O)R² et S ;
W est pour chaque occurrence, de manière identique ou différente, une liaison simple ou un pont divalent qui est sélectionné parmi B(R²), O, C=O, C(R²)₂, Si(R²)₂, S et R²C=CR²;
Ar est pour chaque occurrence, de manière identique ou différente, un système de cycle aromatique ou hétéroaromatique, de préférence un groupe aryle ou hétéroaryle qui comporte de 5 à 40 atomes de cycle aromatique, lequel peut être substitué par un radical ou par plusieurs radicaux R³ ;
R¹ est pour chaque occurrence, de manière identique ou différente, H, D, F, Cl, Br, I, C(=O)Ar, P(=O)Ar₂, S(=O)Ar, S(=O)₂Ar, CR²=CR²Ar, CN, NO₂, Si(R²)₃, B(OR²)₂, OSO₂R², un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C=C, Si(R²)₂, C=O, C=NR², P(=O)(R²), SO, SO₂, NR², O, S ou CONR² et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou un groupe aryloxy ou hétéroaryloxy qui comporte de 5 à 40 atomes de cycle aromatique, lequel peut être substitué par un radical ou par plusieurs radicaux R², ou un groupe aralkyle ou hétéroaralkyle qui comporte de 5 à 40 atomes de cycle aromatique, lequel peut être substitué par un radical ou par plusieurs radicaux R² ;
R² est pour chaque occurrence, de manière identique ou différente, H, D, F, Cl, Br, I, N(R³)₂, N(Ar)₂, C(=O)Ar, P(=O)Ar₂, S(=O)Ar, S(=O)₂Ar, CR³=CR³Ar, CN, NO₂, Si(R³)₃, B(OR³)₂, OSO₂R³, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R³, où un ou plusieurs groupe(s) CH₂ non adjacents peut/peuvent être remplacé(s) par R³C=CR³, C=C , Si(R³)₂, C=O, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R³, ou un groupe aryloxy ou hétéroaryloxy qui comporte de 5 à 40 atomes de cycle aromatique, lequel peut être substitué par un radical ou par plusieurs radicaux R³, ou un groupe aralkyle ou hétéroaralkyle qui comporte de 5 à 40 atomes de cycle aromatique, lequel peut être substitué par un radical ou par plusieurs radicaux R³ ;
R³ est pour chaque occurrence, de manière identique ou différente, H, D, F, Cl, Br, I, C(=O)Ar, P(=O)Ar₂, S(=O)Ar, S(=O)₂Ar, CR⁴=CR⁴Ar, CN, NO₂, Si(R⁴)₃, B(OR⁴)₂, OSO₂R⁴, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux R⁴, où un ou plusieurs groupe(s) CH₂ non adjacents peut/peuvent être remplacé(s) par R⁴C=CR⁴, C=C , Si(R⁴)₂, C=O, C=NR⁴, P(=O)(R⁴), SO, SO₂, NR⁴, O, S ou CONR⁴ et où un ou plusieurs atome(s) de H peut/peuvent être remplacé(s) par D, F, Cl, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R⁴, ou un groupe aryloxy ou hétéroaryloxy qui comporte de 5 à 40 atomes de cycle aromatique, lequel peut être substitué par un radical ou par plusieurs radicaux R⁴, ou un groupe aralkyle ou hétéroaralkyle qui comporte de 5 à 40 atomes de cycle aromatique, lequel peut être substitué par un radical ou par plusieurs radicaux R⁴ ;
R⁴ est pour chaque occurrence, de manière identique ou différente, H, D, F ou un radical hydrocarbone aliphatique qui comporte de 1 à 20 atome(s) de C ou un groupe aryle ou hétéroaryle qui comporte de 5 à 40 atomes de cycle, lequel peut être substitué par un radical ou par plusieurs radicaux R⁵, ou une combinaison de ces groupes ;
R⁵ est pour chaque occurrence, de manière identique ou différente, H, D, F ou un radical hydrocarbone aliphatique qui comporte de 1 à 20 atome(s) de C ;
m est 0 ou 1, où m égal à 0 signifie qu'un groupe W n'est pas lié au niveau de cette position, et les atomes de carbone pertinents qui sont liés à W ont été dans chaque cas remplacés par un groupe X ; et
p, q, r, s, t sont pour chaque occurrence, de manière identique ou différente, 0, 1 ou 2 ;
a, b, c, d, e sont pour chaque occurrence, de manière identique ou différente, 0, 1 ou 2 ;
où :
p+q+r+s+t est supérieur à 1 ou égal à 1 ; et
si r est supérieur ou égal à 1 et si s est supérieur ou égal à 1 et si m est égal à 0, et si c et d sont égaux à 0 pour au moins un groupe pris parmi (Ar²)_{c}N(Ar¹)₂ et (Ar²)_{d}N(Ar¹)₂ dans chaque cas, aucun de ces deux groupes N(Ar¹)₂ n'est agencé au niveau de la position para par rapport à l'atome de carbone quaternaire du squelette.

2. Composé selon la revendication 1, **caractérisé en ce que** W est une liaison simple.

3. Composé selon la revendication 1 ou 2, **caractérisé en ce que** la somme des valeurs des indices p, q, r, s et t est égale à 1.

4. Composé selon une ou plusieurs des revendications 1 à 3, **caractérisé en ce que** l'indice r est égal à 1, et les indices p, q, s et t sont égaux à 0, ou **caractérisé en ce que** l'indice s est égal à 1, et les indices p, q, r et t sont égaux à 0.

5. Composé selon une ou plusieurs des revendications 1 à 4, **caractérisé en ce qu'**il est conforme à la formule (2) 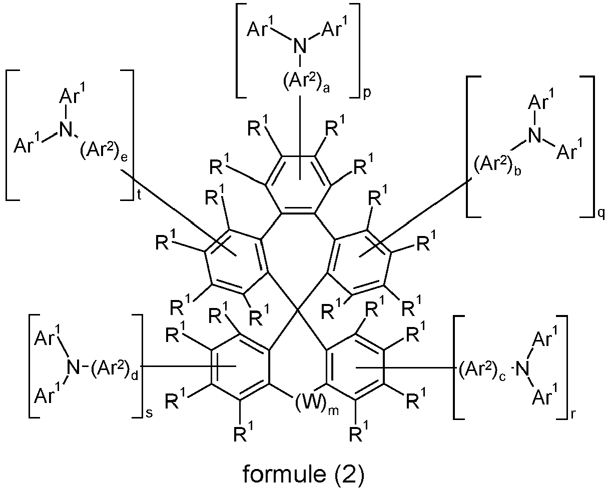 dans laquelle les autres symboles et les autres indices qui sont utilisés présentent les significations qui ont été données selon la revendication 1.

6. Composé selon une ou plusieurs des revendications 1 à 5, **caractérisé en ce que** le composé est un composé de l'une des formules (4) et (5) 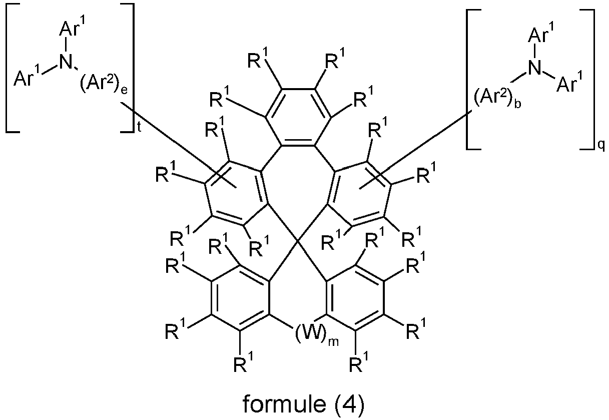 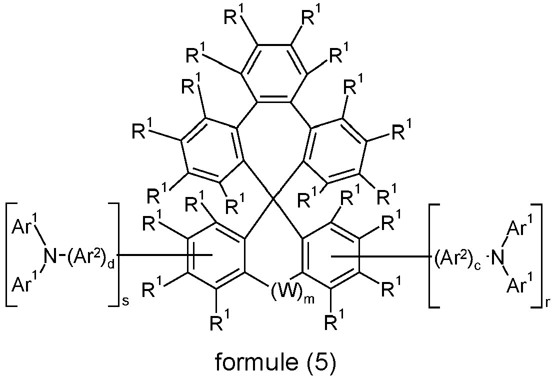 dans lesquelles les symboles et les indices qui sont utilisés présentent les significations qui ont été données selon la revendication 1.

7. Composé selon une ou plusieurs des revendications 1 à 6, **caractérisé en ce que** le composé est un composé de l'une des formules (6) à (11) : 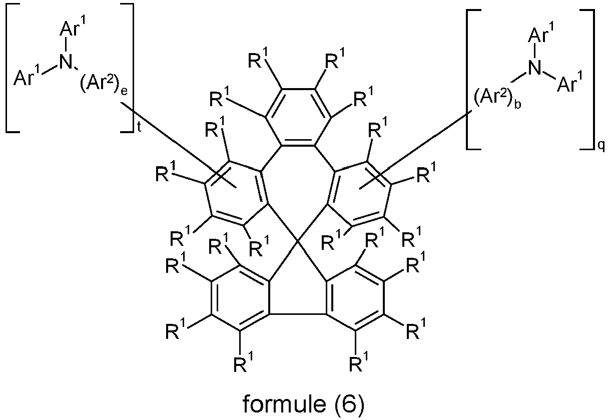 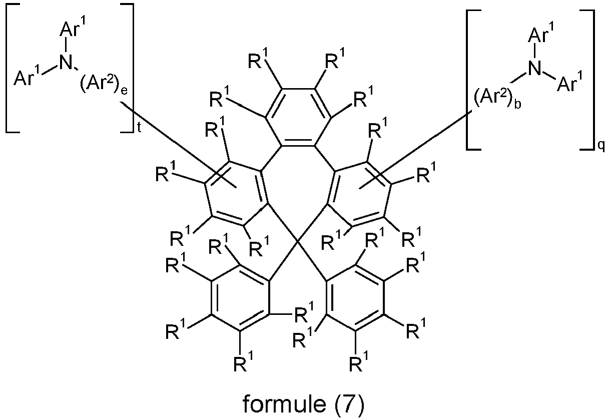 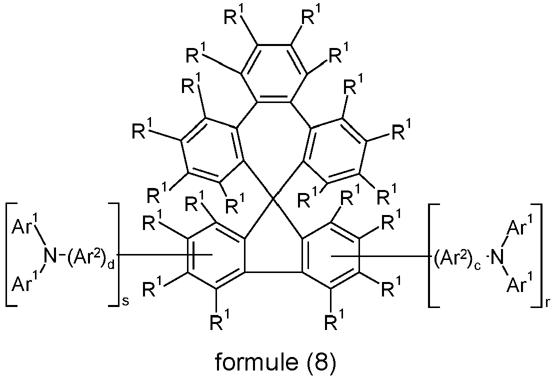 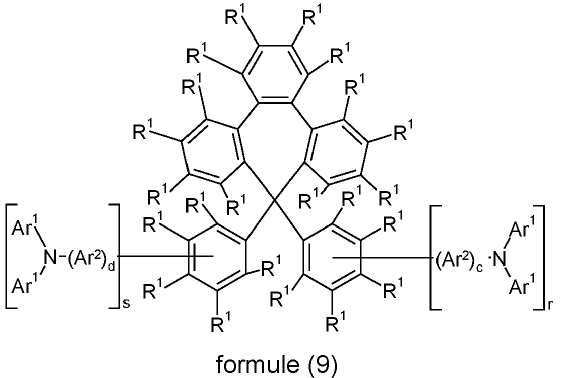 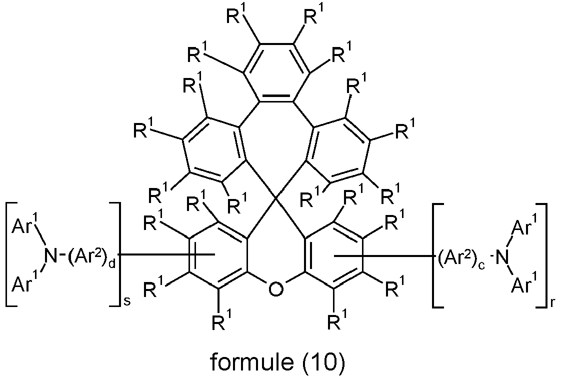 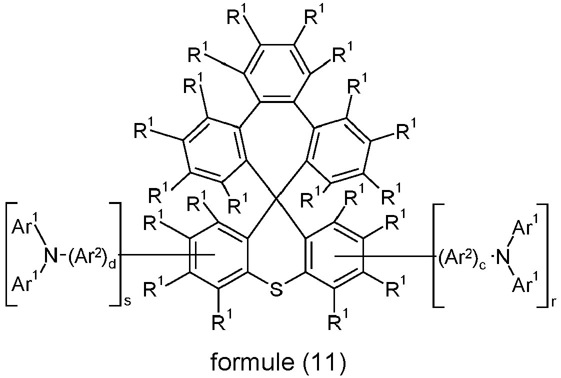 dans lesquelles les symboles et les indices qui sont utilisés présentent les significations qui ont été données selon la revendication 1.

8. Procédé pour la préparation d'un composé selon une ou plusieurs des revendications 1 à 7, **caractérisé en ce que** le composé de la formule (1) est élaboré au moyen d'une ou de plusieurs réaction(s) de couplage et/ou cyclisation(s).

9. Mélange comprenant au moins un composé selon une ou plusieurs des revendications 1 à 7 et au moins un dopant fluorescent ou phosphorescent.

10. Formulation, en particulier une solution, une suspension ou une mini-émulsion, comprenant au moins un composé selon une ou plusieurs des revendications 1 à 7 ou un mélange selon la revendication 9, et un ou plusieurs solvant(s).

11. Utilisation d'un composé selon une ou plusieurs des revendications 1 à 7 ou d'un mélange selon la revendication 9 dans un dispositif électronique.

12. Dispositif électronique, en particulier sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant(s) organiques, les détecteurs optiques organiques, les photorécepteurs organiques, les dispositifs à extinction de champ organiques, les cellules électrochimiques à émission de lumière, les diodes laser organiques et les "dispositifs à émission de plasmons organiques", qui contiennent au moins un composé selon une ou plusieurs des revendications 1 à 7 ou un mélange selon la revendication 9.

13. Dispositif électronique selon la revendication 12, **caractérisé en ce qu'**il s'agit d'un dispositif électroluminescent organique et le composé selon une ou plusieurs des revendications 1 à 7 est utilisé en tant que matériau de matrice pour un composé fluorescent ou phosphorescent dans une couche d'émission, et/ou dans une couche de transport de trous et/ou dans une couche d'injection de trous et/ou dans une couche de blocage d'électrons.
